# EUROPEAN PATENT SPECIFICATION

(11) **EP 1 251 827 B1**
(45) Date of publication and mention of the grant of the patent: **26.05.2004**
(21) Application number: 01909587.6
(22) Date of filing: 09.01.2001
(51) Int. Cl.: A61K 9/00, A61K 9/19, A61K 47/36, A61K 47/02, A61K 38/10, A61K 38/12, A61P 31/10

(54) **NASALLY ADMINISTRABLE ANTIFUNGAL CYCLIC PEPTIDE COMPOSITIONS**
NASAL VERABREICHBARE CYCLISCHE ANTIMYKOTISCHE PEPTIDZUSAMMENSETZUNGEN
COMPOSITIONS PEPTIDIQUES CYCLIQUES ANTIFUNGALES POUVANT ETRE ADMINISTREES PAR VOIE NASALE

(30) Priority: 20.01.2000 EP 00101057
(43) Date of publication of application: 30.10.2002
(73) Proprietor: Basilea Pharmaceutica AG, 4102 Binningen (CH)
(72) Inventor: HORII, Ikuo, Yokohama-shi Kanagawa-ken 247-0027 (JP); KOBAYASHI, Kazuko, Kamakura-shi Kanagawa-ken 247-0072 (JP); SHIMMA, Nobuo, Chigasaki-shi Kanagawa-ken 253-0054 (JP); YANAGAWA, Akira, Yokohama-shi, Kanagawa, 224-0051 (JP)
(74) Representative: Zimmermann, Hans, Dr.
(86) International application number: PCT/EP2001/000163
(87) International publication number: WO 2001/052894

(56) References cited:
- EP-A- 0 588 255
- EP-A- 0 681 833
- WO-A-00/05251
- US-A- 4 462 983
- US-A- 5 051 400
- US-A- 5 331 089
- MERKUS F W H M ET AL: "ABSORPTION ENHANCERS IN NASAL DRUG DELIVERY: EFFICACY AND SAFETY" JOURNAL OF CONTROLLED RELEASE,ELSEVIER SCIENCE PUBLISHERS B.V. AMSTERDAM,NL, vol. 24, no. 1/03, 1993, pages 201-208, XP000938562 ISSN: 0168-3659
- DATABASE BIOSIS [Online] BIOSCIENCES INFORMATION SERVICE, PHILADELPHIA, PA, US; April 1998 (1998-04) PFALLER M A ET AL: "In vitro activity of two echinocandin derivatives, LY303366 and MK-0991 (L-743,792), against clinical isolates of Aspergillus, Fusarium, Rhizopus, and other filamentous fungi." Database accession no. PREV199800268178 XP002171879 & DIAGNOSTIC MICROBIOLOGY AND INFECTIOUS DISEASE, vol. 30, no. 4, April 1998 (1998-04), pages 251-255, ISSN: 0732-8893

## Description

The present invention relates to a nasally administrable composition containing an antifungal cyclic peptide or a pharmaceutically acceptable salt thereof, which attains improved absorbability of said cyclic peptide into the body when administered nasally.

There exist many physiologically active cyclic peptides derived from natural origin (e.g. antifungal cyclic peptides such as aureobasidines, echinocandins, pneumocandins and aerothricins; immunosuppressants such as cyclosporin A; antibiotics such as vancomycin and daptomycin) as well as cyclic peptides that were designed and synthesized so as to mimic a part of the structure of physiologically active peptides in mammals (e.g. growth hormone release inhibiting factors/somatostatin analogs such as lanreotide and vapreotide; vasopressin antagonists (USP 5,095,003) and fibrinogen gpIIb/IIIa receptor antagonists such as eptifibatid). Although those physiologically active cyclic peptides have significant therapeutic potential, their clinical utility is often limited due to their poor oral bioavailability.

For example, antifungal cyclic peptides such as aerothricins (EP Application Nos. 98113744.1 and 99107637.3), echinocandin analogs (LY303366: EP 0 736 541; FK463 and its analogs: WO 98/723637, WO 99/740108) and pneumocandin analogs (MK0991: WO 94/721677) exhibit highly potent antifungal activity when administered intravenously. FK463 and MK0991 are currently under clinical trial by i.v. infusion. However, their clinical utility could be rather limited especially for out-patients due to lacking an oral formulation. Namely, these antifungal cyclic peptides can only little be absorbed intact from the mucous membrane of the intestine because of the decomposition by proteases existing in the digestive system and/or their rather high molecular weight and polarity.

Therefore, there exists a strong demand to develop a method for administering physiologically active cyclic peptides via a non-injection route and, more preferably, methods which enable patients to safely administer such physiologically active cyclic peptides by themselves, with a simple administration method and a low frequency. Nasal application could be an alternative administration route to an oral administration when considered the patient's compliance.

Recently, some nasally administrable powdery preparations having improved absorbability have been proposed. They are prepared by adsorbing physiologically active linear polypeptide hormones, such as insulin and carcitonin, onto a polyvalence metal such as hydroxyapatite or calcium carbonate (EP 0 681 833 A2). However, in those instances, the attained plasma concentration of the physiologically active peptides is still very low (pico gram to nano gram/ml) and its plasma half life is short. Nevertheless, it is sufficient for exerting the biological activity because of its high efficacy.

On the other hand, much higher plasma concentration of the bioactive peptide and longer half life is usually required for chemotherapy, e.g. the treatment of systemic fungal infections. It is reported that peptidic compounds can be metabolized by peptidases located at nasal mucosa (A. Husain et al, Biochem. Biophys. Res. Commun. (1985) 133, 923-928). Up to now, no nasal formulations have been developed to attain such a high plasma concentration of peptidic drugs for chemotherapeutic treatment. The nasally administrable preparations so far proposed are not satisfactory because of poor absorbability of the active ingredient or local irritation so that they are not commercially available yet.

As a result of extensive studies on nasal formulations of physiologically active peptides, the present inventors have discovered a nasally administrable composition of an antifungal cyclic peptide or a pharmaceutically acceptable salt thereof that is unlikely to be administered orally, with higher bioavailability and less irritation than those of other nasally administrable preparations so far proposed for linear peptides, and completed the present invention.

In particular, the present invention relates to a nasally administrable composition comprising (i) an antifungal cyclic peptide, (ii) a physiologically acceptable powdery or crystalline carrier containing a polyvalence metal or an organic carrier, and (iii) an absorption enhancer, wherein a physiologically effective amount of said physiologically active cyclic peptide is dispersed homogeneously in and adsorbed homogeneously onto said physiologically acceptable powdery or crystalline polyvalence metal carrier or organic carrier, whose mean particle size is in the range of 20 to 500 µm.

Furthermore, the present invention relates to the use of the above nasally administrable compositions for the manufacture of a medicament for the treatment of disease such as systemic fungal and bacterial infections, cardiovascular disorder, acromegaly and cancer or for controlling immune system by intranasal administration.

The physiologically active cyclic peptide to be used in the present invention may be any cyclic peptide having antifungal activity. Some examples of those physiologically active cyclic peptides will be described in more detail later.

The polyvalence metal which is one of the components of the physiologically acceptable powdery or crystalline carrier used in the present invention may be metal compounds having more than 2 valency, and may include, for example, aluminum compounds, calcium compounds, magnesium compounds, silicon compounds, iron compounds and zinc compounds. Such metal compounds are commonly used as excipients, stabilizers, filing agents, disintegrants, lubricants, adsorbents and coating agents for medical preparations.

The aluminum compound to be used in the present invention may include, for example, dry aluminum hydroxy gel, aluminum hydroxychloride, synthetic aluminum silicate, light aluminum oxide, colloidal aluminum silicate hydrate, aluminum magnesium hydroxide, aluminum hydroxide, aluminum hydroxide gel, aluminum sulfate, dihydroxyaluminum aminoacetate, aluminum stearate, natural aluminum silicate, aluminum monostearate and potassium aluminum sulfate. Among them, the preferable aluminum compound is aluminum hydroxide.

The calcium compound may include, for example, apatite, hydroxyapatite, calcium carbonate, calcium disodium EDTA, calcium chloride, calcium citrate, calcium glycerophosphate, calcium gluconate, calcium silicate, calcium oxide, calcium hydroxide, calcium stearate, calcium phosphate tribasic, calcium lactate, calcium pantothenate, calcium oleate, calcium palmitate, calcium D-pantothenate, calcium alginate, calcium phosphate anhydride, calcium hydrogenphosphate, calcium primary phosphate, calcium acetate, calcium saccharate, calcium sulfate, calcium secondary phosphate, calcium para-aminosalicylate and bio-calcilutite compounds. Bio-calcilutite compounds such as crystalline calcium pyrophosphate (Ca₂(P₂O₇) 2H₂O), calcium secondary phosphate (CaHPO₄ 2H₂O), octacalcium phosphate (Ca₈H₂(PO₄) 5H₂O), tricalcium phosphate (Ca₃-(PO₄)₂) and crystalline calcium oxalate (CaC₂O₄ H₂O) are analogous to hydroxyapatite (Ca₁₀(PO₄)₆(OH)₂), and may also be used as a physiologically acceptable powdery or crystalline carrier of the present invention. Preferable calcium compounds are hydroxyapatite, calcium carbonate or calcium lactate.

Furthermore, the magnesium compound which is one of the component of the physiologically acceptable powdery or crystalline carrier used in the present invention includes, for example, magnesium L-aspartate, magnesium chloride, magnesium gluconate, magnesium aluminate silicate, magnesium silicate, magnesium oxide, magnesium hydroxide, magnesium stearate, magnesium carbonate, magnesium aluminate metasilicate, magnesium sulfate, sodium magnesium silicate and synthetic sodium magnesium silicate. Among them, preferable magnesium compound is magnesium stearate.

Other metal compounds with more than 2 valency may be silicon compounds such as silicon oxide hydrate, light silicic anhydride, synthetic hydrotalcite, diatomaceous earth and silicon dioxide; iron compounds such as ferrous sulfate; and zinc compounds such as zinc chloride, zinc stearate and zinc sulfate.

The above metal compounds may be used alone or in combination of two or more.

Preferably, the physiologically acceptable powdery or crystalline carrier of the present invention may have a mean particle size in the range of 20 to 250 µm, more preferably in the range of 20 to 100 µm, most preferably in the range of 20 to 60 µm.

Preferable organic carrier which is one of the components of the physiologically acceptable powdery carrier used in the present invention may be fine grain powder of rice, wheat, buck wheat, barley, soybean, corn, millet, foxtail millet and the like.

Preferably, the mean particle size of the organic carrier is not more than 300 µm, more preferably in the range of 20 to 180 µm.

Preferable absorption enhancer which is one of the components of the nasally administrable composition according to the present invention is a pharmaceutically acceptable natural (e.g. cellulose, starch and their derivatives) or unnatural polymer material. These compounds are normally used as a binder, but nothing has been so far reviewed about the applicability of an absorption enhancer for a nasally administrable preparation.

A preferable embodiment of the cellulose and its derivatives is microcrystalline cellulose, methyl cellulose, ethyl cellulose, hydroxypropyl cellulose, hydroxypropylmethyl cellulose, hydroxypropylmethyl cellulose phthalate, cellulose acetate, cellulose acetate phthalate, carboxymethyl cellulose, low carboxymethyl cellulose sodium, carboxymethylehtyl cellulose and the like.

A preferable embodiment of the starch and its derivatives is corn starch, potato starch, rice starch, glutinous rice starch, wheat starch, pregelatinized starch, dextrin, sodium carboxymethyl starch, hydroxypropyl starch, pullulan and the like.

Other natural polymers such as agar, sodium alginate, chitin, chitosan, egg yolk lecithin, gum arabic, tragacanth, gelatine, collagen, casein, albumin, fibrinogen, and fibrin may also be used as absorption enhancer.

A preferable embodiment of the unnatural polymer is sodium polyacrylate, polyvinyl pyrrolidone, and the like.

Preferable absorption enhancers are fine powder of rice, glutinous rice, starch, gelatine, dextrin, hydroxypropyl cellulose, hydroxypropylmethyl cellulose, polyvinyl pyrrolidone, egg yolk lecithin, gum arabic, tragacanth or a mixture thereof. More preferable absorption enhancers are fine powder of glutinous rice, starch, gelatine, hydroxypropyl cellulose, hydroxypropylmethyl cellulose, polyvinyl pyrrolidone, tragacanth or a mixture thereof. Even more preferable absorption enhancers are fine powder of glutinous rice or hydroxypropyl cellulose. Most preferable absorption enhancer is fine powder of glutinous rice.

The mean particle size of the absorption enhancer is preferably not more than 250 µm, more preferably from 20 to 180 µm.

The above absorption enhancers may be used alone or in combination of two or more absorption enhancers in the physiologically acceptable powdery or crystalline carrier of the present invention.

For nasally administrable preparations, it has been thought so far that a water-soluble carrier would help to attain a good absorption of the active substance into the body. However, it has been found that an excellent absorption of active substances can be obtained by homogeneously dispersing the active substance in a water-insoluble carrier, for example, hydroxyapatite, calcium carbonate, calcium lactate, aluminum hydroxide or magnesium stearate, preferably in the presence of an absorption enhancer, and homogeneously adsorbing said cyclic peptide thereonto.

The hydroxyapatite used in the present invention includes synthetic hydroxyapatite and hydroxyapatite obtained from organisms (bio-hydroxyapatite). The bio-hydroxyapatite may be prepared by using bones or teeth of animals from which organic materials are removed.

Calcium carbonate, calcium lactate, aluminum hydroxide or magnesium stearate is usually used as a stabilizer, lubricant, agent to add luster, excipient, dispersing agent or coating agent for a pharmaceutical preparation; however, it has been found that these compounds having a mean particle size of not more than 500 µm can be used as a carrier for the compositions of the present invention, and offers the effect of promoting the absorption of physiologically active substances into the body by nasal administration.

Physiologically active cyclic peptides in accordance with the present invention are antifungal cyclic peptides [e.g. aerothricins (as described later in this specification), echinocandin and pneumocandin analogs (typical analogs are described in: Current Pharmaceutical Design, 1996, 2, 209-224) and aureobacidines (JP 03044398)], antibacterial cyclic peptides [e.g. vancomycin, daptomycin (GB 2,120,257), and the like], cyclosporin A, lanreotide (WO 9504752: growth hormone release inhibiting factor), vapreotide (US 4,650,787: growth hormone release inhibiting factor), vasopressin antagonist (US 5,095,003), eptifibatide (US 3,67,509: fibrinogen gpIIb/IIIa receptor antagonist) and the like.

Examples of above antifungal cyclic peptides are aerothricins of the following formula (I): 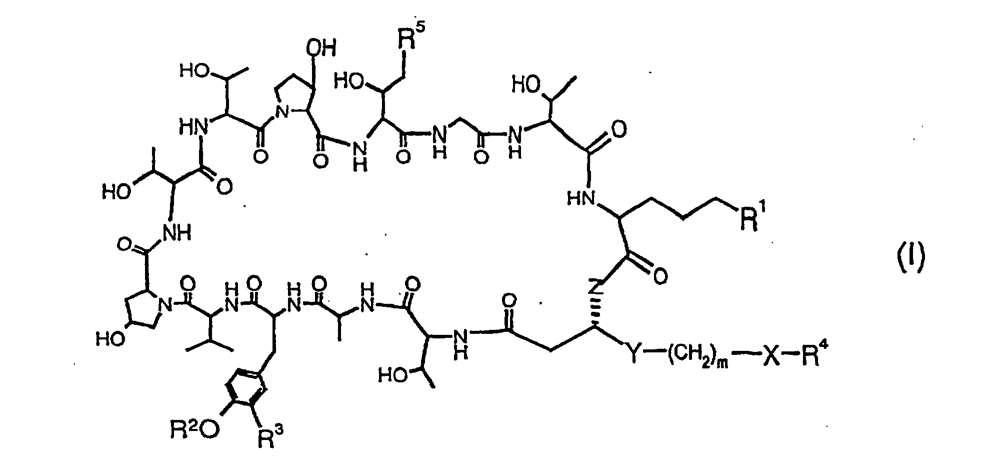 wherein
R¹ is guanidino, tri-lower alkylammonio, -N(R¹⁰)-R¹¹, -N(R¹⁵)-CO-R¹⁴, -N(R¹⁵)-CO-CH[N(R¹⁰)R¹¹]-R¹³, -NHCOCH(R¹³)-NHCOCH(NH₂)-R¹³, 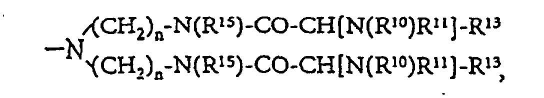 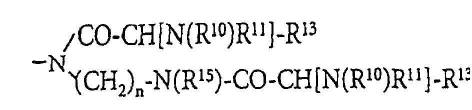 or 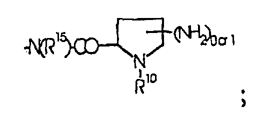 R¹⁰ and R¹¹ are each independently selected from hydrogen; heteroaryl substituted with one or two amino; lower alkyl optionally substituted with one or more, preferably one or two, amino, amino-lower alkyl, cyano, guanidino, nitrogen containing heterocycle(s) or phenyl group(s) containing an amino, amidino or guanidino group;
R¹³ is a residue derived from natural or unnatural amino acids;
R¹⁴ is lower alkyl substituted with one or more, preferably one or two, amino, guanidino, nitrogen containing heterocycle(s) or phenyl group(s) containing an amino, amidino or guanidino group;
R¹⁵ is hydrogen, lower alkyl optionally substituted with one or more, preferably one or two, amino, guanidino, nitrogen containing heterocycle(s) or phenyl group (s) containing an amino, amidino or guanidino group;
R² is hydrogen, hydroxysulfonyl, lower alkyl or lower alkenyl, wherein lower alkyl and lower alkenyl may be optionally substituted with acyl, carbamoyl, amino, mono-lower alkylamino or di-lower alkylamino;
R³ is hydrogen, hydroxy, nitro, amino, acylamino, (lower alkylcarbamoyl)amino, carboxyl, lower alkoxy, lower alkoxycarbonyl, lower alkyl, lower alkenyl or lower alkynyl, wherein lower alkyl, lower alkenyl and lower alkynyl may be optionally substituted with hydroxy, amino, mono-lower alkylamino, di-lower alkylamino, lower alkoxycarbonyl or carbamoyl;
R⁴ is alkyl, alkenyl, alkoxy or alkenyloxy which may be optionally substituted with lower alkyl, aryl, cycloalkyl or fluorine atom(s);
R⁵ is -CONH₂, -CN or -CH₂NH₂;
X is a single bond, or an aryl, biphenyl or terphenyl group optionally containing one or more hetero atom(s) and/or being substituted with halogen atom(s) or lower alkyl;
Y is a single bond, -CH2-, -CH(lower alkyl)-, -CONH- or -CON(lower alkyl)-;
Z is -O-, -NH- or -N(lower alkyl)-;
m is an integer of 0 to 4; and
n is an integer of 2 to 5;
and pharmaceutically acceptable salts thereof.

The compounds of above formula (I) are new, provided that R¹ is not amino, R² and R³ are not hydrogen, R⁵ is not -CONH₂, and Z is not -O- or -NH- at the same time when -Y-(CH₂)ₘ-X-R⁴ is unsubstituted alkyl or aralkyl.

In this specification, the term "lower" is used to mean a group consisting of 1 to 6, preferably 1 to 4 carbon atom(s), unless otherwise indicated.

The term "alkyl" refers to a branched or straight chain monovalent saturated aliphatic hydrocarbon radical of one to twenty carbon atoms, preferably of one to sixteen carbon atoms. The term "lower alkyl" refers to a branched or straight chain monovalent alkyl radical of one to six carbon atoms, preferably one to four carbon atoms. This term is further exemplified by such radicals as methyl, ethyl, *n*-propyl, isopropyl, *n*-butyl, *i*-butyl, *tert*-butyl and the like.

The term "alkenyl" refers to an alkyl group containing one or more double bond(s) in the alkylene chain.

The term "alkynyl" refers to an alkyl group containing one or more triple bond(s) in the alkylene chain.

The term "alkoxy" refers to the group -O-R', where R' is an alkyl. The term "lower alkoxy" refers to the group -O-R', where R' is a lower alkyl.

The term "alkenyloxy" refers to an alkoxy group which contains one or more double bond(s) in the alkylene chain.

The term "acyl" refers to the group -C(O)-R', where R' is a lower alkyl. The term "acylamino" refers to an acyl group attached to an imino radical, i.e., -NH-.

The term "mono-lower alkylamino" refers to a lower alkyl group attached to an imino radical, i.e., -NH-. The term "di-lower alkylamino" refers to two independently selected lower alkyl groups attached to a nitrogen atom, i.e., -N(-lower alkyl)-lower alkyl. The term "tri-lower alkylammonio" means tri-lower alkylammonio containing three independently selected C₁₋₃-alkyl groups.

The term "lower alkoxycarbonyl" refers to the group -C(O)OR', where R' is a lower alkyl.

The term "(lower alkylcarbamoyl)amino" refers to the group -NHCONH-R', where R' is a lower alkyl.

The term "halogen atom" refers to fluorine, chlorine, bromine and iodine.

The term "aryl" refers to a monovalent carbocyclic aromatic radical (e.g. phenyl), or two condensed carbocyclic rings (e.g. naphtyl) optionally mono-, di- or tri-substituted, independently, with lower alkyl, trifluoromethyl, halogen and the like.

The term "nitrogen containing heterocycle" refers to a saturated, unsaturated or aromatic monovalent cyclic radical containing at least one nitrogen atom.

The term "heteroaryl" refers to an aromatic monovalent mono- or poly-carbocyclic radical containing at least one heteroatom, i.e. nitrogen, sulfur or oxygen. Examples of heteroaryl residues with one or more nitrogen atoms are pyridyl, pyrazinyl, pyrimidinyl, pyridazinyl, triazinyl and imidazolyl.

The term "cycloalkyl" refers to a monovalent carbocyclic radical of three to ten carbon atoms, preferably of three to six carbon. atoms.

The term "pharmaceutically acceptable salts" embraces salts of the Aerothricis of the Formula (I) with inorganic or organic acid such as hydrochloric acid, hydrobromic acid, nitric acid, sulfuric acid, phosphoric acid, citric acid, formic acid, maleic acid, acetic acid, trifluoroacetic acid, succinic acid, tartaric acid, methanesulfonic acid, p-toluenesulfonic acid and the like, which are non-toxic to living organisms.

Each substituent of Formula (I) in the above is explained in more detail hereafter.

In the definition of R¹, the term "tri-lower alkylammonio" preferably means trimethylammonio and triethylammonio.

In the definition of R¹⁰ and R¹¹, the term "heteroaryl" preferably means 2-pyridyl, 2-pyrazinyl, 2-pyrimidinyl, 2-pyridazinyl, 2-triazinyl, 2-imidazolyl and the like, more preferably 2-pyridyl and 2-imidazolyl, most preferably 2-pyridyl. The term "lower alkyl" preferably means an alkyl chain consisting of 1 to 6 carbon atoms such as methyl, ethyl, *n*-propyl, *iso*-propyl, *n*-butyl, *iso*-butyl, *sec*-butyl, *n*-pentyl, neopentyl, *tert*-pentyl, and *n*-hexyl; preferably methyl, ethyl, *n*-propyl or *n*-butyl, most preferably methyl, ethyl or *n*-propyl. The term "nitrogen containing heterocycles" preferably means morpholino, piperazinyl, N-methylpiperazinyl, pyrrolidinyl, piperidinyl, imidazolidinyl, pyrazolidinyl, imidazolyl, pyrazolyl, triazolyl, pyridinyl, pyrazinyl and the like, more preferably piperazinyl and morpholino, most preferably piperazinyl. The term "phenyl group(s) containing an amino, amidino or guanidino group" preferably means 4-aminophenyl, 4-amidinophenyl, 4-guanidinophenyl and the like.

In the definition of R¹³, the term "a residue derived from natural or unnatural amino acids" preferably means hydrogen or lower alkyl which may be substituted with hydroxy, amino, guanidino, methylthio, mercapto, carbamoyl, carboxy, phenyl, hydroxyphenyl, aminophenyl, imidazolyl or indolyl and the like. Preferable embodiment of R¹³ is lower alkyl substituted with amino or guanidino such as aminomethy, 2-aminoethyl, 3-aminopropyl, 4-aminobutyl, 4-guanidinobutyl.

In the definition of R¹⁴, the term "lower alkyl" means the same as defined for R¹⁰ and R¹¹. Preferably, it means an alkyl chain consisting of 2 to 5 carbon atoms such as ethyl, propyl, butyl and pentyl. The term "nitrogen containing heterocycles" means the same as defined for R¹⁰ and R¹¹. Preferably, it means morpholino, piperazinyl, N-methylpiperazinyl, pyrrolidinyl, piperidinyl, imidazolidinyl, pyrazolidinyl, imidazolyl, pyrazolyl, triazolyl, pyridinyl, pyrazinyl and the like, more preferably piperazinyl and morpholino. The term "phenyl group(s) containing an amino, amidino or guanidino group" preferably means 4-aminophenyl, 4-amidinophenyl, 4-guanidinophenyl and the like. Preferable embodiment of R¹⁴ is 2-aminoethyl, 3-aminopropyl, 4-aminobutyl, 2-guanidinoethyl, 3-guanidinopropyl, 2-piperazinoethyl, 2-morpholinoethyl, 4-aminophenethyl and the like.

In the definition of R¹⁵, the terms "lower alkyl", "nitrogen containing heterocycles" and "phenyl group(s) containing an amino, amidino or guanidino group" are the same as defined for R¹⁴. Preferable embodiment of R¹⁵ is 2-aminoethyl, 3-aminopropyl, 4-aminobutyl, 2-guanidinoethyl, 3-guanidinopropyl, 2-piperazinoethyl, 2-morpholinoethyl, 4-aminophenethyl and the like.

Preferable embodiments of -N(R¹⁰)-R¹¹ [wherein R¹⁰ and R¹¹ are as defined above] are amino, 5-aminopyrid-2-ylamino, methylamino, ethylamino, propylamino, (2-aminoethyl)amino, (3-aminopropyl)amino, [3-[(3-aminopropyl)amino]propyl]amino, (2-piperazinylethyl)amino, (2-morpholinoethyl)amino, N,N-dimethylamino, N,N-diethylamino, N,N-dipropylamino, N,N-ethylmethylamino, N,N-bis(2-aminoethyl)amino, N,N-bis(3-aminopropyl)amino, N,N-bis(4-aminobutyl)amino, N,N-bis(2-piperazinylethyl)amino, N,N-bis(2-morpholinoethyl)amino, N,N-bis(2-guanidinoethyl)amino, N,N-bis(3-guanidinopropyl)amino, N,N-bis(2-pyridin-2-ylethyl)amino, N,N-bis(imidazol-2-ylmethyl)amino, N-(2-aminoethyl)-N-(3-aminopropyl)amino, N-(3-aminopropyl)-N-(2-piperazinylethyl)amino, N-(3-aminopropyl)-N-(2-pyridin-2-ylethyl)amino and the like. More preferable embodiments are amino, 5-aminopyrid-2-ylamino, N,N-dimethylamino, (2-aminoethyl)amino, (3-aminopropyl)amino, [3-[(3-aminopropyl)amino]propyl]amino, (2-piperazinylethyl)amino, N,N-bis(2-aminoethyl)amino, N,N-bis(3-aminopropyl)amino, N,N-bis(4-aminobutyl)amino, N,N-bis(2-piperazinylethyl)amino, N,N-bis(2-guanidinoethyl)amino, N,N-bis(3-guanidinopropyl)amino, N-(2-aminoethyl)-N-(3-aminopropyl)amino, N-(3-aminopropyl)-N-(2-piperazinylethyl)amino and the like. Most preferable embodiments are (3-aminopropyl)amino, N,N-bis(2-aminoethyl)amino, N,N-bis(3-aminopropyl)amino and N,N-bis(2-piperazinylethyl)amino.

In the definition of -N(R¹⁵)-CO-CH[N(R¹⁰)R¹¹]-R¹³, the group -CO-CH[N(R¹⁰)R¹¹]-R¹³ [wherein R¹⁰ and R¹¹ are hydrogen; R¹³ is a residue derived from natural or unnatural amino acids] preferably means sarcosyl, glycyl, alanyl, ornitinyl, lysyl, valyl, leucyl, isoleucyl, tryptophyl, phenylalanyl, methionyl, seryl, tyrosyl, threonyl, cysteinyl, asparaginyl, glutaminyl, aspartyl, glutamyl, arginyl, histidyl, 2,3-diaminopropionyl, 2,4-diaminobutyryl, 2-amino-4-triazol-1-ylbutyryl and the like.

Preferable embodiments of -N(R¹⁵)-CO-CH[N(R¹⁰)R¹¹]-R¹³ are acylamino groups derived from basic amino acids. Examples of such acylamino groups are ornitinylamino, lysylamino, arginylamino, histidylamino, 3-aminoprolylamino, 2,3-cliaminopropionylamino, 2,4-diaminobutyrylamino, 2-amino-4-triazol-1-ylbutyrylamino, [3-amino-2-[bis(2-aminoethyl)amino]propionyl]amino, [4-amino-2-[bis(2-aminoethyl)amino]butyryl]amino, [5-amino-2-[bis(2-aminoethyl)amino]valeryl]amino, N-(3-aminopropyl)-N-(2,3-diaminopropionyl)amino, N-(3-aminopropyl)-N-(2,4-diaminobutyryl)amino, N-(3-aminopropyl)-N-(2,5-diaminovaleryl)amino, N-(3-aminopropyl)-N-(2,6-diaminohexanoyl)amino and the like; more preferably ornitinylamino, lysylamino, arginylamino, histidylamino, 2,3-diaminopropionylamino, 2,4-diaminobutyrylamino, [3-amino-2-[bis(2-aminoethyl)amino]propionyl]amino, [4-amino-2-[bis(2-aminoethyl)amino]butyryl]amino, [5-amino-2-[bis(2-aminoethyl)amino]valeryl]amino, N-(3-aminopropyl)-N-(2,3-diaminopropionyl)amino, N-(3-aminopropyl)-N-(2,4-diaminobutyryl)amino, N-(3-aminopropyl)-N-(2,5-diaminovaleryl)amino and N-(3-aminopropyl)-N-(2,6-diaminohexanoyl)amino, most preferably ornitinylamino, lysylamino, 2,4-diaminobutyrylamino, [4-amino-2-[bis(2-aminoethyl)amino]butyryl]amino, [5-amino-2-[bis(2-aminoethyl)amino]valeryl]amino, N-(3-aminopropyl)-N-(2,4-diaminobutyryl)amino, N-(3-aminopropyl)-N-(2,6-diaminohexanoyl)amino, N-(3-aminopropyl)-N-[(2S)-2,5-diaminovaleryl]amino, N-(3-aminopropyl)-N-[(2R)-2,5-diaminovaleryl]amino, N-(3-aminopropyl)-N-[(2S)-5-amino-2-[N,N-bis(2-aminoethyl)amino]valeryl]amino, N-(3-aminopropyl)-N-[(2R)-5-amino-2-[N,N-bis(2-aminoethyl)amino]valeryl]amino, N-(3-aminopropyl)-N-[(2S)-5-amino-2-[N-(3-aminopropyl)amino]valeryl]amino, N-(2-aminoethyl)-N-[(2S)-5-amino-2-[N,N-bis(2-aminoethyl)amino]valeryl]amino and N-(2-aminoethyl)-N-[(2R)-5-amino-2-[N,N-bis(2-aminoethyl)amino]valeryl]amino.

In the definition of R¹, preferable embodiment of 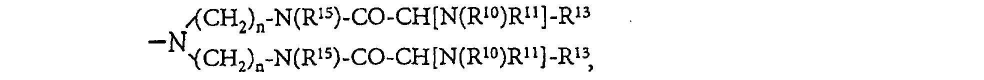 is bis[2-(ornitylamino)ethyl]amino, bis-[3-(ornitylamino)propyl]amino,
[2-(lysylamino)ethyl]amino, bis-[3-(lysylamino)propyl]amino and the like.

In the definition of R¹, preferable embodiment of 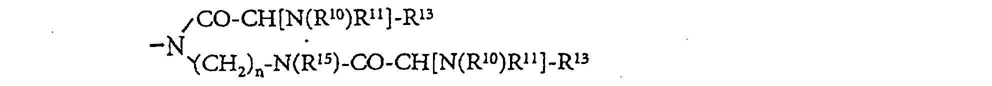 is N-ornityl-N-[2-(ornitylamino)ethyl]-amino, N-ornityl-N-[3-(ornitylamino)propyl]-amino, N-ornityl-N-[3-(lysylamino)propyl]amino, N-ornityl-N-[3-(lysylamino)propyl]-amino, N-lysyl-N- [2-(ornitylamino)ethyl]amino, N-lysyl-N-[3-(ornitylamino)propyl]amino, N-lysyl-N-[2-(lysylamino)ethyl]amino, N-lysyl-N-[3-(lysylamino)propyl]amino and the like.

In the definition of R¹, preferable embodiment of 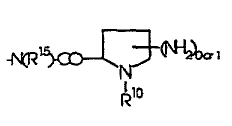 is prolylamino, 3-aminoprolylamino, 4-aminoprolylamino, N-(3-aminoprop-yl)-N-prolylamino, (2-aminoethyl)prolylamino and the like.

The term "-NHCOCH(R¹³)-NHCOCH(NH₂)-R¹³" [wherein R¹³ is as defined above] preferably means ornityl-ornitylamino, lysyl-ornitylamino, ornityl-lysylamino, lysyl-lysylamino and the like.

In the term "-N(R¹⁵)-CO-R¹⁴" [wherein R¹⁴ and R¹⁵ are as defined above], the term "nitrogen containing heterocycle" and the term "phenyl group(s) containing an amino, amidino or guanidino group" are as defined above.

Preferable embodiments of -N(R¹⁵)-CO-R¹⁴ are 3-aminopropionylamino, 3-guanidinopropionylamino, 3-piperazinylpropionylamino, (3-pyridin-3-ylpropionyl)amino, [3-(4-aminophenyl)propionyl] amino, N-(3-aminopropionyl)-N-(3-aminopropyl)amino and the like.

In a preferred aspect, R¹ is -N(R¹⁰)-R¹¹, wherein R¹⁰ and R¹¹ are as defined above. In another preferred aspect, R¹ is -N(R¹⁵)-CO-CH[N(R¹⁰)R¹¹]-R¹³, wherein R¹⁰, R¹¹, R¹³ and R¹⁵ are as defined above. In another preferred aspect, R¹ is -N(R¹⁵)-CO-R¹⁴, wherein R¹⁴ and R¹⁵ are as defined above. In another preferred aspect, R¹ is 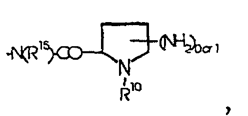 wherein R¹⁰ and R¹⁵ are as defined above. In another preferred aspect, R¹ is -NHCOCH(R¹³)-NHCOCH(NH₂)-R¹³, wherein R¹³ is as defined above. In another preferred aspect, R¹ is tri-lower alkylammonio. In still another preferred aspect, R¹ is amino or guanidino.

In the definition of R², the term "lower alkyl optionally substituted with acyl, carboxy, carbamoyl, amino, mono-lower alkylamino or di-lower alkylamino" preferably means methyl, ethyl, *n*-propyl, isopropyl, butyl, oxo-lower alkyl, carboxy-lower alkyl, carbamoyl-lower alkyl, amino-lower alkyl and the like, more preferably methyl, ethyl, *n*-propyl, *n*-butyl, 2-oxopropyl, carboxymethyl, carbamoylmethyl, 3-aminopropyl and the like. The term "lower alkenyl optionally substituted with acyl, carboxy, carbamoyl, amino, mono-lower alkylamino or di-lower alkylamino" preferably means allyl, 2-butenyl, 3-butenyl and the like, more preferably allyl.

In a preferred aspect, R² is hydrogen, hydroxysulfonyl or lower alkyl such as methyl or ethyl.

In the definition of R³, the term "acylamino" preferably means lower alkylcarbonylamino such as acetylamino, propionylamino or isobutyrylamino, or an arylamino group derived from natural or unnatural amino acids such as sarcosylamino, glycylamino, alanylamino, ornitylamino, lysylamino, prolylamino, valylamino, leucylamino, isoleucylamino, tryptophylamino, phenylalanylamino, methionylamino, serylamino, tyrosylamino, threonylamino, cysteinylamino, asparaginylamino, glutamylamino, aspartylamino, glutamylamino, arginylamino, histidylamino and the like; preferably sarcosylamino, glycylamino, alanylamino, lysylamino, prolylamino and the like. The term "(lower-alkylcarbamoyl)amino" preferably means methylcarbamoylamino, ethylcarbamoylamino, propylcarbamoylamino, butylcarbamoylamino and the like, more preferably methylcarbamoylamino or ethylcarbamoylamino. The term "lower alkoxy" preferably means methoxy, ethoxy, propoxy, butoxy and the like, more preferably methoxy and ethoxy. The term "lower alkoxycarbonyl" preferably means methoxycarbonyl, ethoxycarbonyl, propoxycarbonyl, butoxycarbonyl and the like, more preferably methoxycarbonyl and ethoxycarbonyl. The term "lower alkyl which may be optionally substituted with hydroxy, amino, mono-lower alkylamino, di-lower alkylamino, lower alkoxycarbonyl or carbamoyl" preferably means methyl, ethyl, propyl, aminomethyl, aminoethyl, aminopropyl, hydroxymethyl, hydroxyethyl, methylaminomethyl, 2-(methylamino)ethyl, 3-(methylamino)propyl, dimethylaminomethyl, 2-(dimethylamino)ethyl, 3-(dimethylamino)propyl, 2-(methoxycarbonyl)ethyl, 2-(carbamoyl)ethyl and the like. The term "lower alkenyl which may be optionally substituted with hydroxy, amino, mono-lower alkylamino, di-lower alkylamino, lower alkoxycarbonyl or carbamoyl" preferably means vinyl, 2-(methoxycarbonyl)vinyl, 2-(carbamoyl)vinyl and the like. The term "lower alkynyl which may be optionally substituted with hydroxy, amino, mono-lower alkylamino, di-lower alkylamino, lower alkoxycarbonyl or carbamoyl" preferably means ethynyl, propynyl, hydroxypropynyl, aminopropynyl, diethylaminopropynyl and the like.

In a preferred aspect, R³ is hydrogen, hydroxy, nitro, amino or acylamino. In another preferred aspect R³ is (lower alkylcarbamoyl)amino, carboxyl, lower alkoxy or lower alkoxycarbonyl.

In the definition of R⁴, the term "alkyl, alkenyl, alkoxy or alkenyloxy" preferably means an alkyl, alkenyl, alkoxy or alkenyloxy group containing 3 to 16 carbon atoms, such as propyl, butyl, pentyl, hexyl, heptyl, octyl, oct-4-enyl, oct-6-enyl, nonanyl, decyl, undecyl, dodecyl, tridecyl, tetradecyl, pentadecyl, hexadecyl, propoxy, butoxy, pentyloxy, hexyloxy, heptyloxy, octyloxy, oct-4-enyloxy, oct-6-enyloxy, nonanyloxy, non-5-enyloxy, decyloxy and the like. The term "lower alkyl" preferably means methyl, ethyl, propyl, butyl, pentyl, more preferably methyl or ethyl. The term "aryl" means an aryl group which may optionally be substituted with lower alkyl, trifluoromethyl or halogen atom(s) such as phenyl, naphtyl, 3-fluorophenyl, 3-bromophenyl, 3-chlorophenyl, 4-fluorophenyl, 4-bromophenyl, 4-chlorophenyl, 3-methylphenyl, 4-methylphenyl, 4-trifluoromethylphenyl. The term "cycloalkyl" preferably means cyclopropyl, cyclobutyl, cyclopentyl, cyclohexyl, adamantyl and the like. The term "alkyl, alkenyl, alkoxy or alkenyloxy which may be optionally substituted with lower alkyl, aryl, cycloalkyl or fluorine atom(s)" preferably means 5-methylhexyl, 1-methyltridecyl, 2-ethylbutoxy, 4-methylpentyloxy, 2-propylpentyloxy, 2-ethylhexyloxy, 3,7-dimethyloctyloxy, 2-phenylethoxy, 2-(4-fluorophenyl)ethoxy, 2-(4-chlorophenyl)ethoxy, 2-(3-fluorophenyl)ethoxy, 2-(4-trifluorophenyl)ethoxy, 3-phenylpropoxy, 2-naphtylethoxy, 3-naphtylpropoxy, 2-cyclopropylethoxy, 2-cyclobutylethoxy, 2-cyclopentylethoxy, 3-cyclopentylpropoxy, 2-cyclohexylethoxy, 3-cyclohexylpropoxy, 3,3-diphenylpropoxy, 3,3,3- trifluoropropoxy, 4,4,4-trifluorobutoxy, 5,5,5-trifluoropentyloxy and the like.

In a preferred aspect, R⁴ is alkyl or alkoxy which may be optionally substituted with lower alkyl, aryl, cycloalkyl or fluorine atom(s).

Preferable embodiments of R⁵ are -CONH₂ or -CH₂NH₂.

In the definition of X, the term "hetero atom" preferably means nitrogen, sulfur and oxygen. The term "aryl, biphenyl or terphenyl optionally containing one or more hetero atom(s)" preferably means 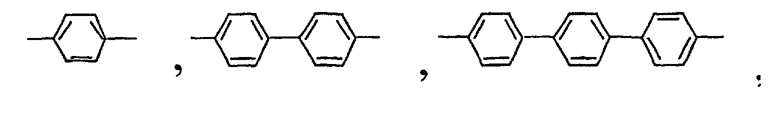 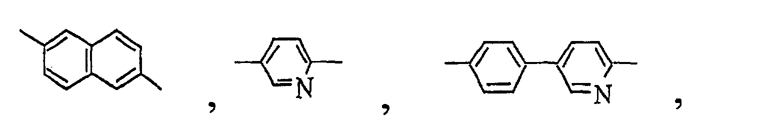 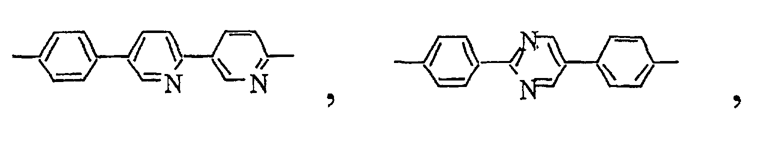 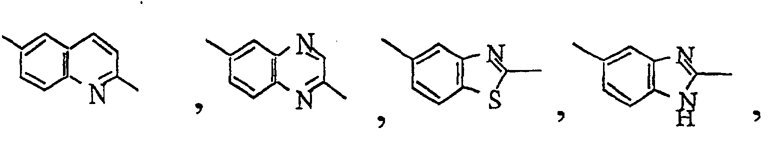 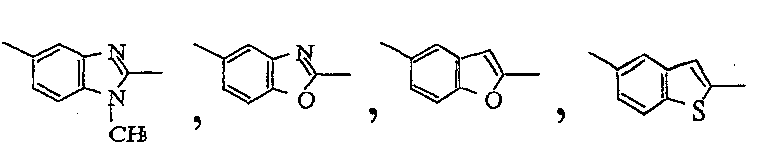 or 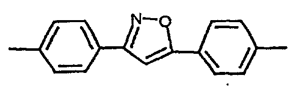 and the like, which may be further substituted with halogen atom(s) or lower alkyl. The open-ended lines in the formulas above indicate the preferred linkage in the corresponding position.

The most preferable embodiment of X is a single bond, 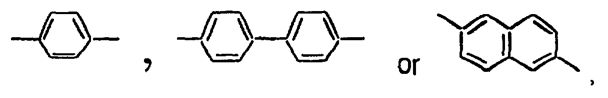 which may be further substituted with halogen atom(s) or lower alkyl, preferably methyl.

In the definition ofY, the term "lower alkyl" preferably means an alkyl group consisting of 1 to 3 carbon atoms, e.g. methyl, ethyl or propyl. The preferable embodiment ofY is a single bond, -CH₂-, -CH(CH₃)-, -CONH- or -CON(CH₃)-, more preferably a single bond, -CH(CH₃)- or -CONH-.

In the definition of Z, the, term "-N(lower alkyl)-" preferably means an N-alkyl group consisting of 1 to 3 carbon atoms, e.g. N-methyl, N-ethyl or N-propyl. A preferable embodiment of Z is -O- ; another preferable embodiment of Z is -NH-.
m is an integer of 0 to 4, preferably 0 to 2.

Preferred Aerothricins in accordance with the present invention are Aerothricins 2 and 4 to 137 as exemplified in the following Table 1.

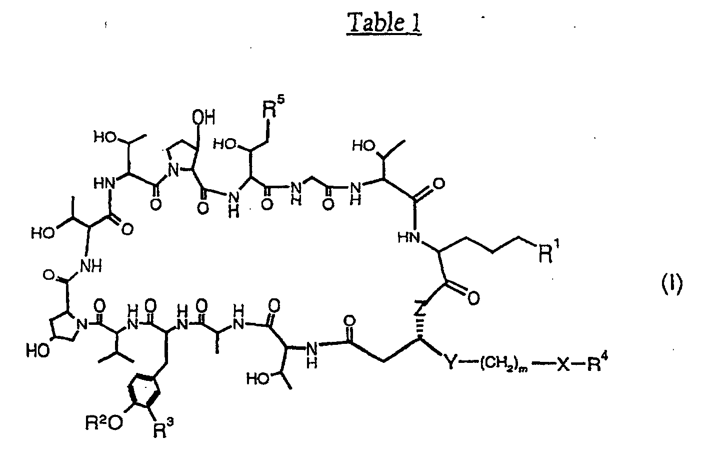

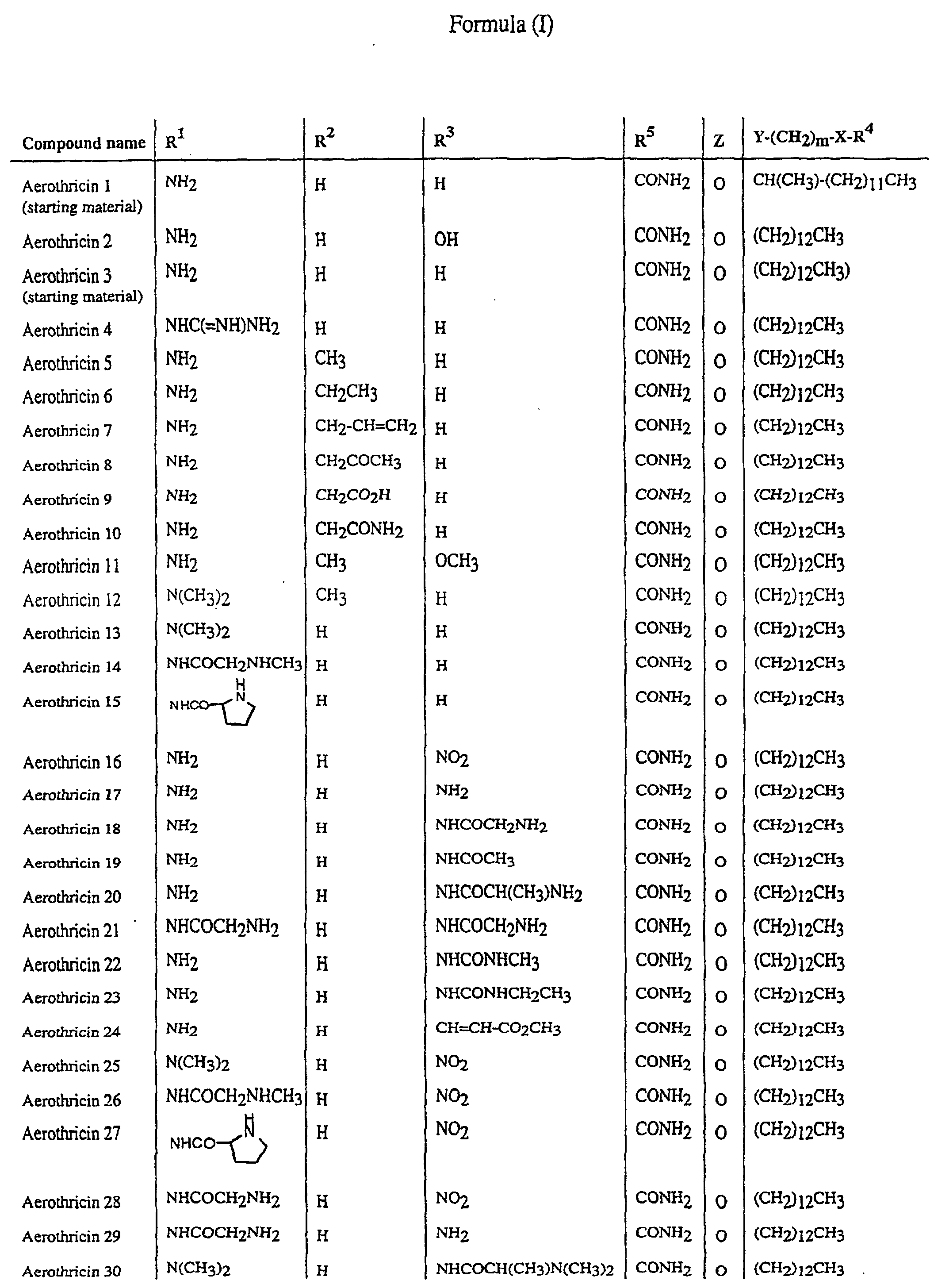

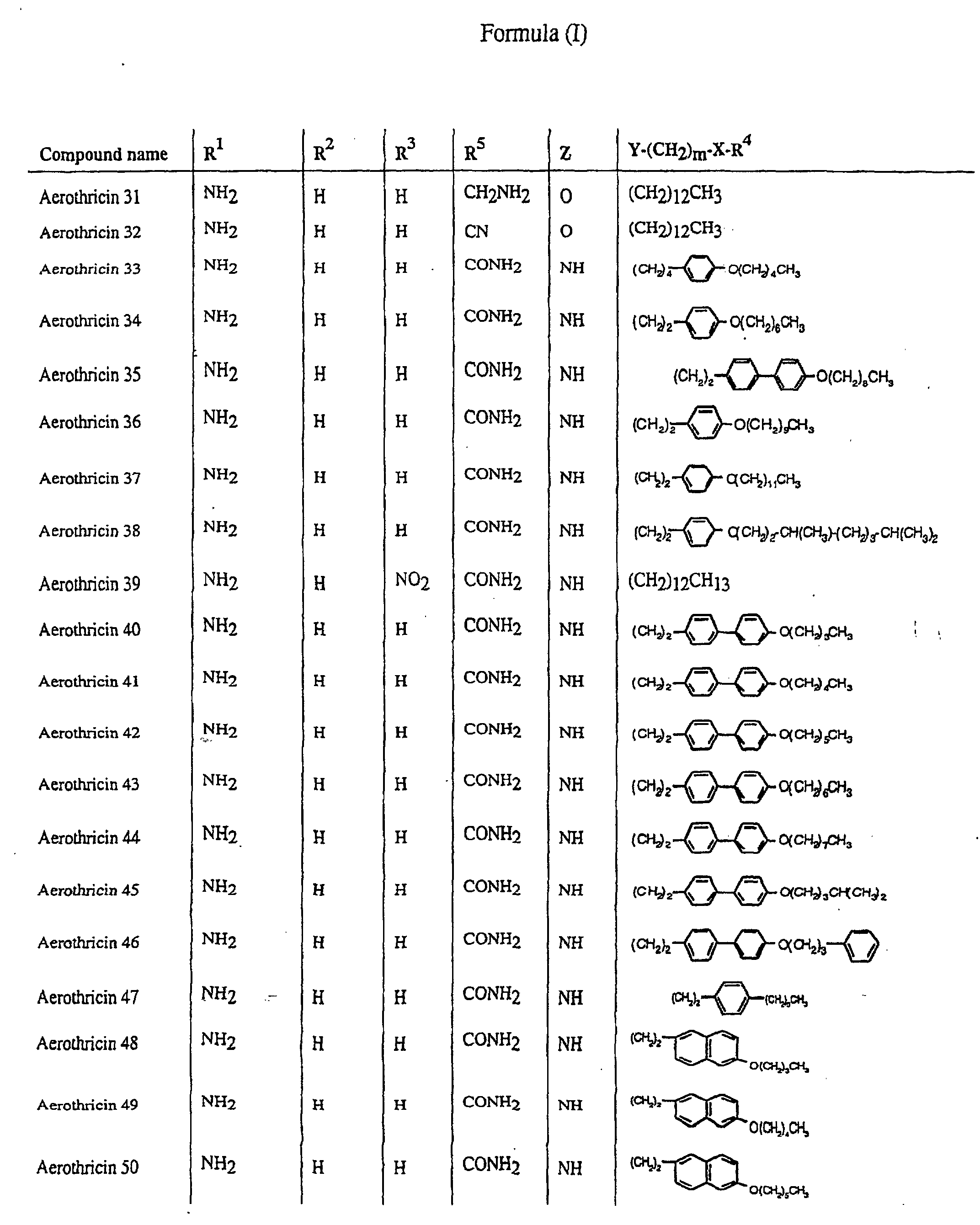

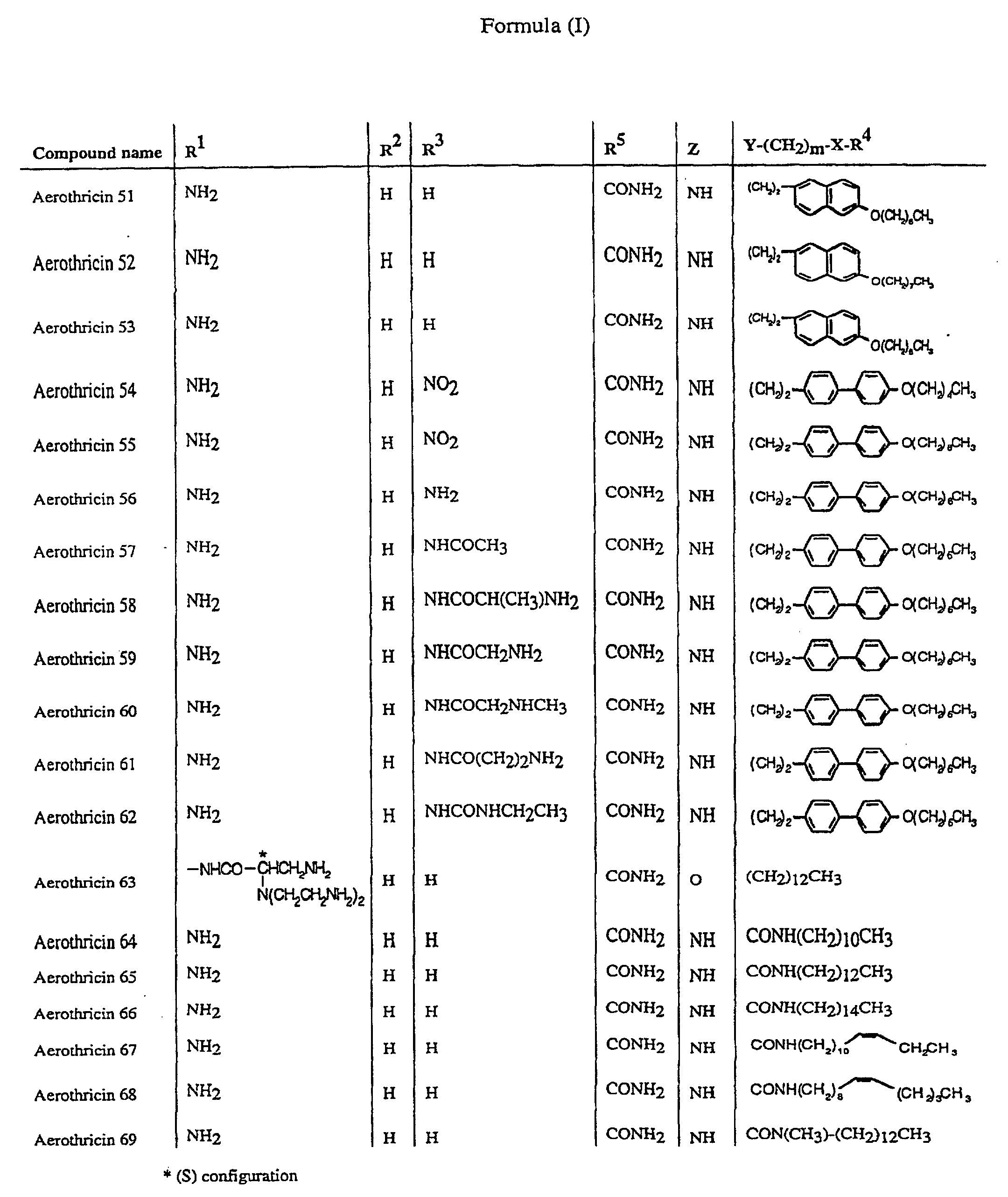

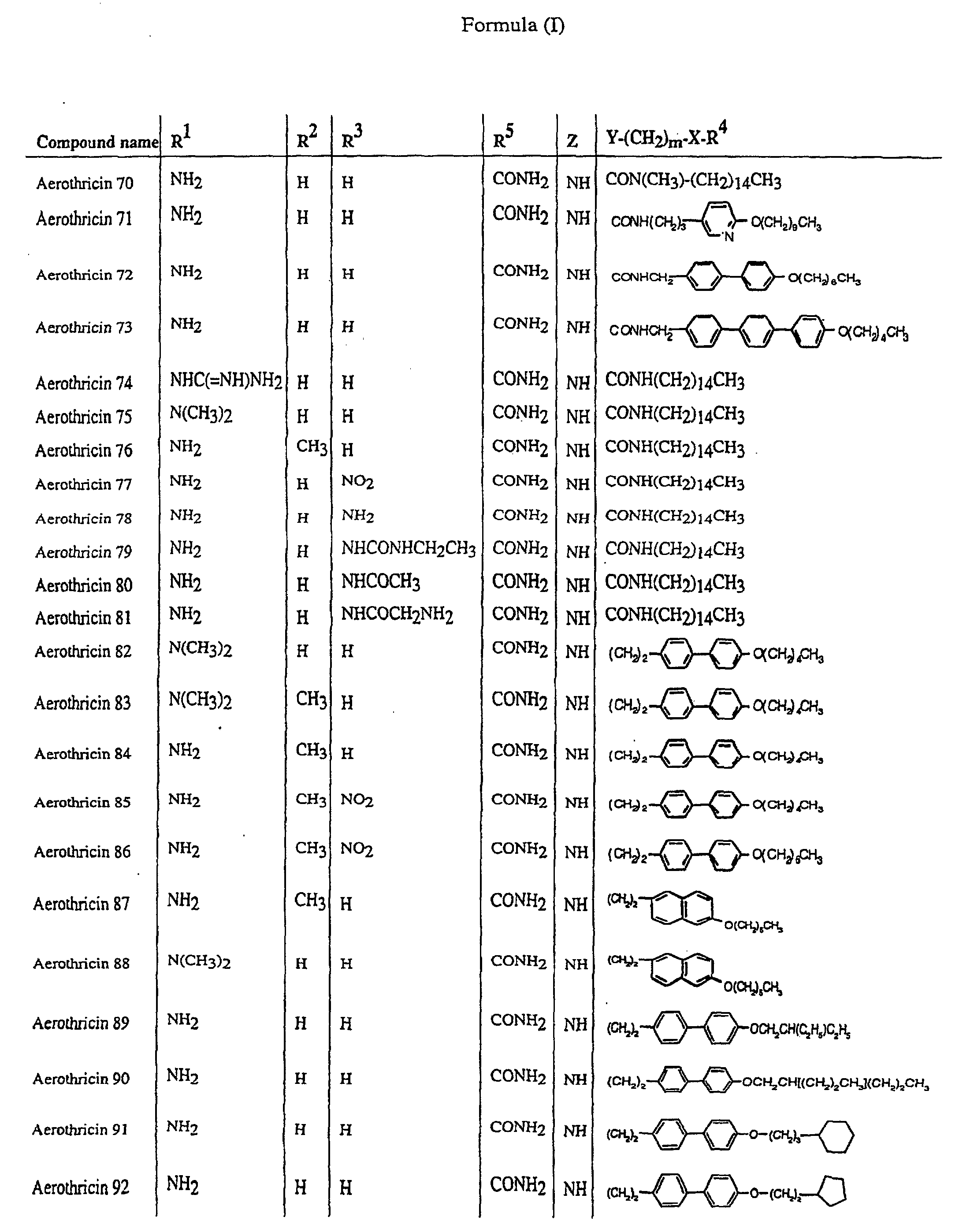

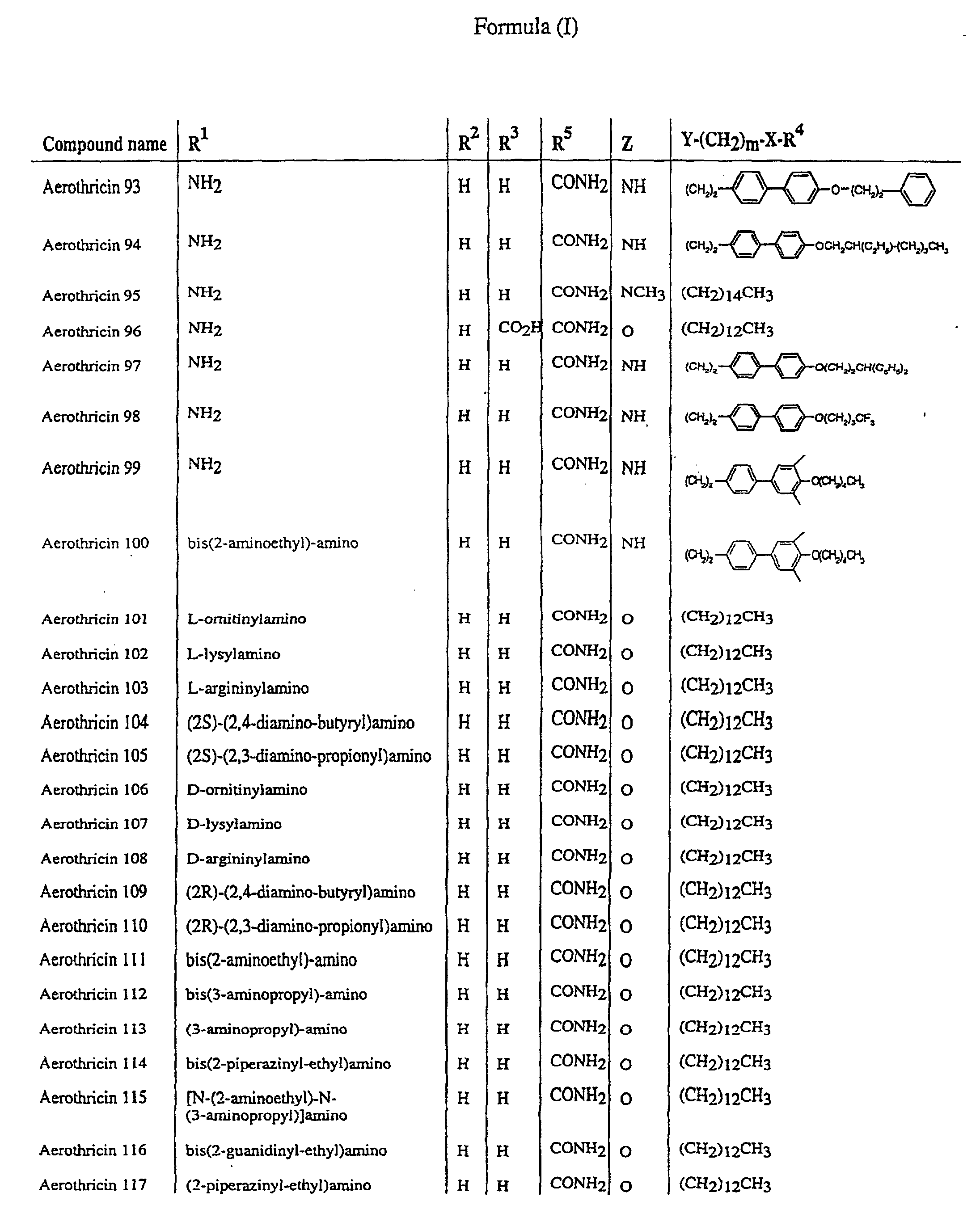

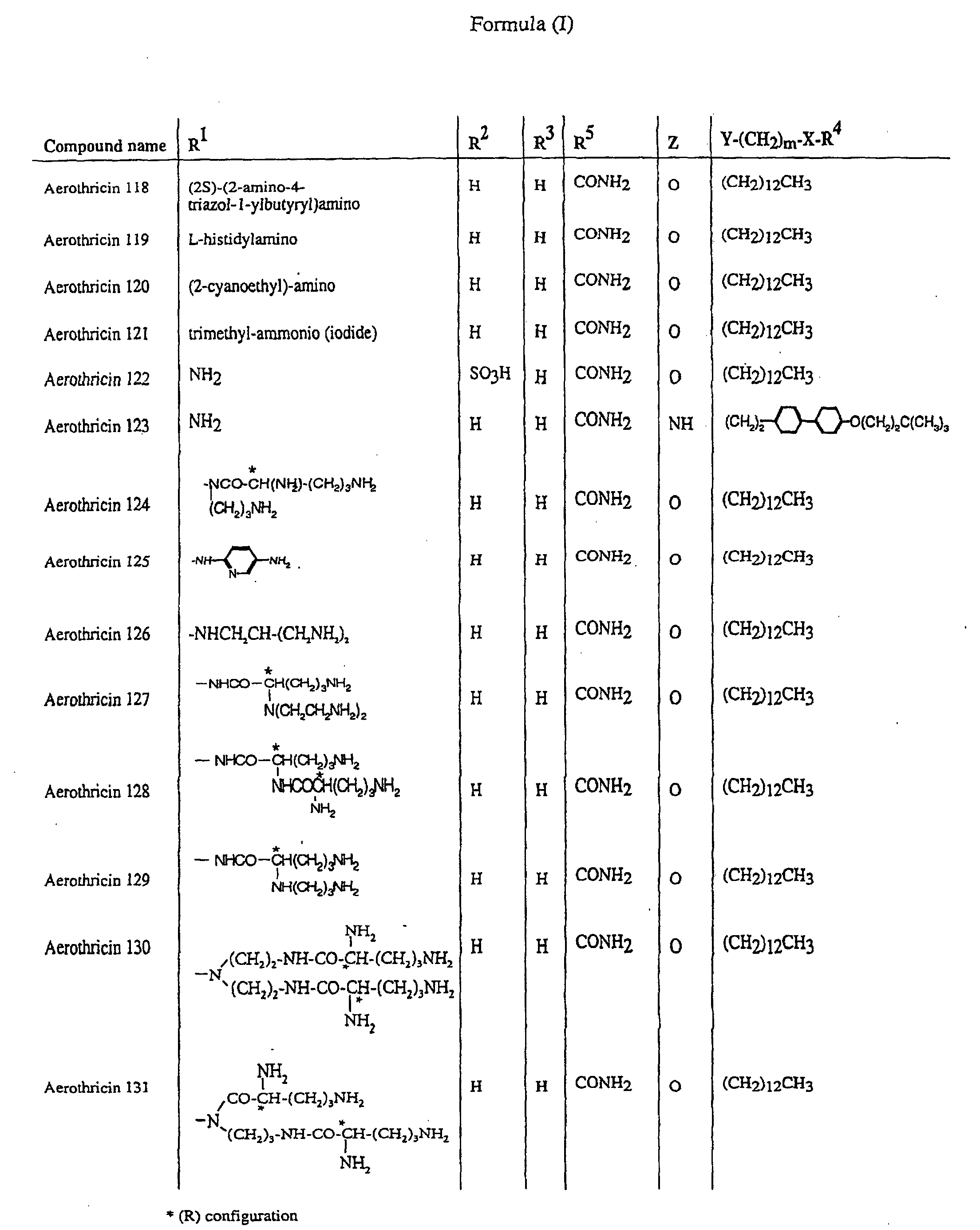

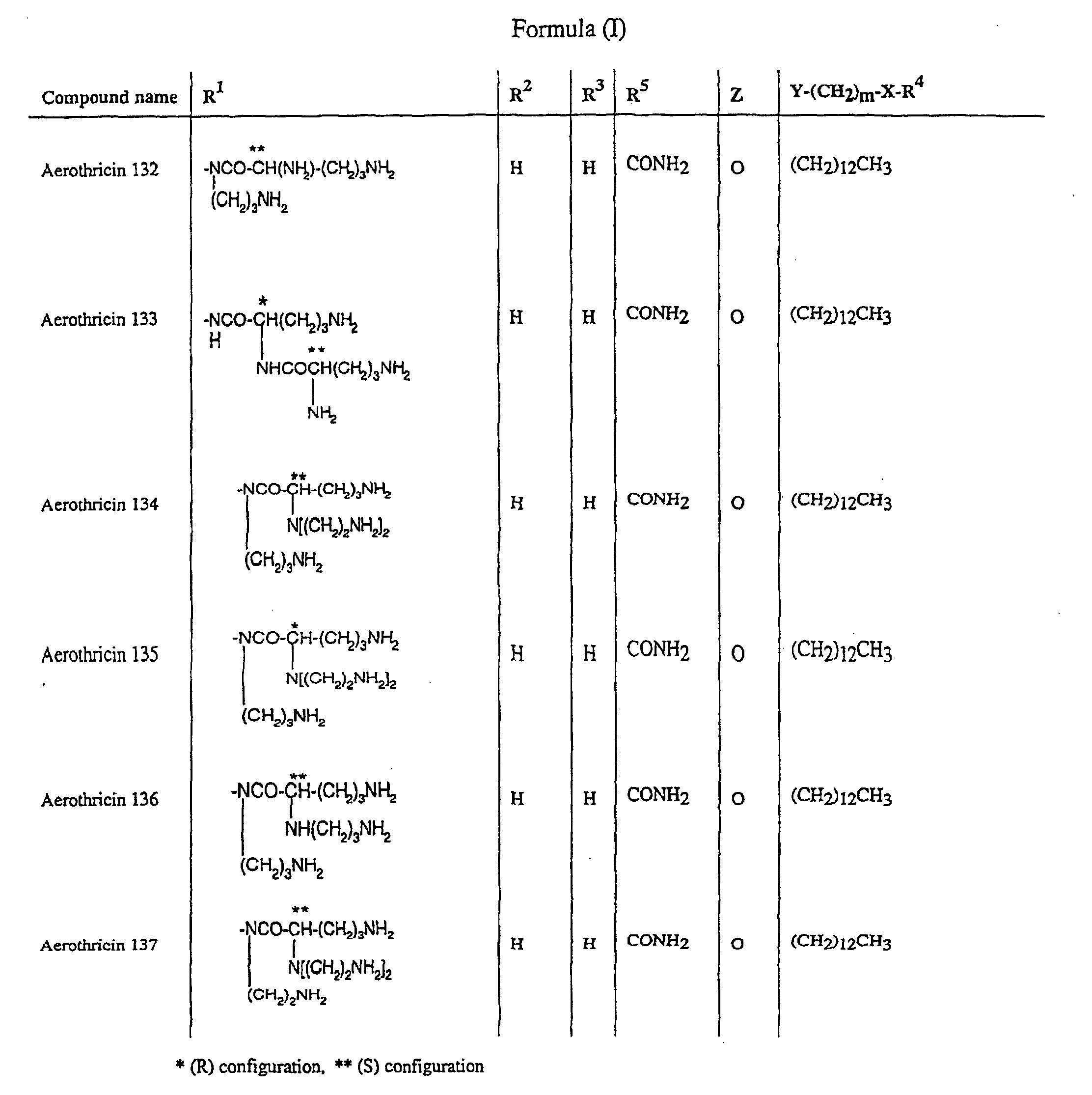

Further examples of above antifungal cyclic peptides are echinocandin analogs (e.g. LY303366: EP 736 541, FK463 and its analogs as described in WO 98/23637 and WO 99/40108) and pneumocandin analogs (e.g. MK0991 as described in WO 94/21677):

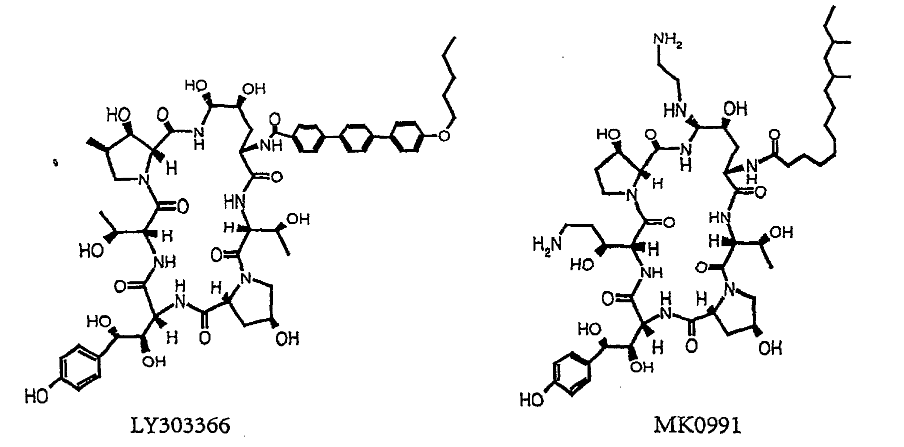

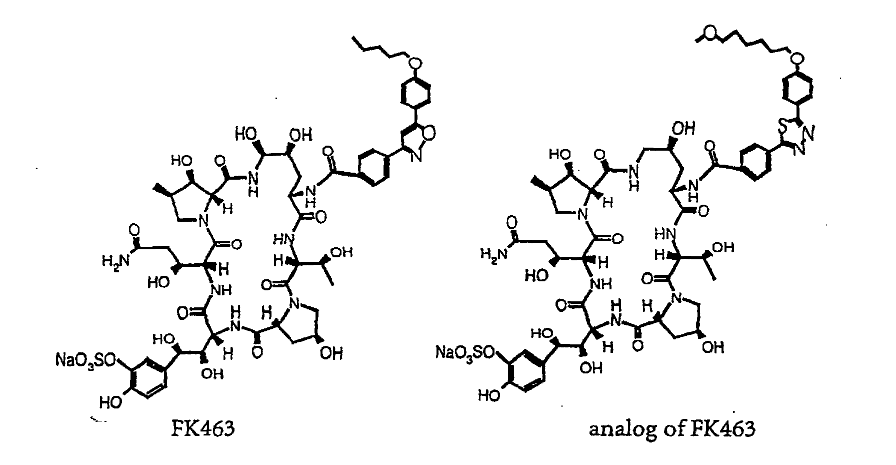

More preferable aerothricins in connection with the nasally administrable composition of the present invention are aerothricins of the aforementioned formula (I), wherein
R¹ is -N(R¹⁰)-R¹¹, -N(R¹⁵)-CO-R¹⁴, -N(R¹⁵)-CO-CH[N(R¹⁰)R¹¹]-R¹³,
-NHCOCH(R¹³)-NHCOCH(NH₂)-R¹³, 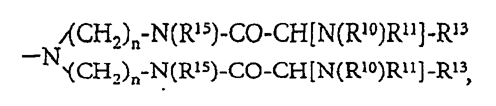 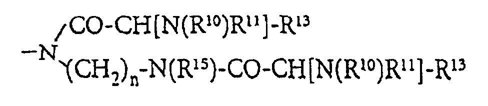 or 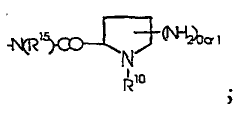 R¹⁰ and R¹¹ are each independently selected from hydrogen; lower alkyl optionally substituted with one or more, preferably one or two, amino, amino-lower alkyl, cyano, guanidino, or nitrogen containing heterocycle(s) preferably selected from morpholino, piperazinyl, N-methylpiperazinyl, pyrrolidinyl, piperidinyl, imidazolidinyl, pyrazolidinyl, imidazolyl, pyrazolyl, triazolyl, pyridinyl, pyrazinyl and the like, more preferably selected from piperazinyl and N-methylpiperazinyl.
R¹³ is a residue derived from natural or unnatural amino acids, preferably selected from hydrogen or lower alkyl which may be substituted with hydroxy, amino, dimethylamino, guanidino, methylthio, mercapto, carbamoyl, carboxy, phenyl, hydroxyphenyl, aminophenyl, imidazolyl or indolyl and the like, more preferably selected from lower alkyl substituted with amino or guanidino such as aminomethy, 2-aminoethyl, 3-aminopropyl, 3-(dimethylamino)propyl, 4-aminobutyl or 4-guanidinobutyl.
R¹⁴ is lower alkyl substituted with one or more, preferably one or two, amino, dimethylamino, guanidino, or nitrogen containing heterocycle(s) preferably selected from morpholino, piperazinyl, N-methylpiperazinyl, pyrrolidinyl, piperidinyl, imidazolidinyl, pyrazolidinyl, imidazolyl, pyrazolyl, triazolyl, pyridinyl, pyrazinyl and the like, more preferably selected from piperazinyl, N-methylpiperazinyl and imidazolyl.
R¹⁵ is hydrogen, lower alkyl optionally substituted with one or more, preferably one or two, amino, dimethylamino, guanidino, or nitrogen containing heterocycle(s) preferably selected from morpholino, piperazinyl, N-methylpiperazinyl, pyrrolidinyl, piperidinyl, imidazolidinyl, pyrazolidinyl, imidazolyl, pyrazolyl, triazolyl, pyridinyl, pyrazinyl and the like, more preferably selected from piperazinyl and N-methylpiperazinyl.
R² is hydrogen, hydroxysulfonyl or lower alkyl;
R³ is hydrogen, hydroxy, or amino;
R⁴ is alkyl
R⁵ is -CONH₂, -CN or -CH₂NH₂;
X is a single bond;
Y is a single bond, -CH₂-, -CH(lower alkyl)-;
Z is -O-;
m is an integer of 0 to 4; and
n is an integer of 2 to 5.
and pharmaceutically acceptable salts thereof.

Still more preferable aerothricins in connection with the nasally administrable composition of the present invention are aerothricins 1-5, 14, 15, 17, 31, 32, 63, 96, 101-122, 124, 126-137 as exemplified in above Table 1. Most preferable aerothricins in connection with the nasally administrable composition of the present invention are aerothricins 132-137.

Aerothricins represented by Formula (I) can be produced according to the following methods:

### Process A

Aerothricins of the Formula (II) can be produced by cultivating a microorganism belonging to *Deuteromycotina* capable of producing Aerothricins 1, 2 and 3 [Aerothricin 3 (= WF11243) is described in Reference Example 1] under aerobic conditions in an aqueous or a solid medium and isolating Aerothricins 1, 2 and 3 from the culture. 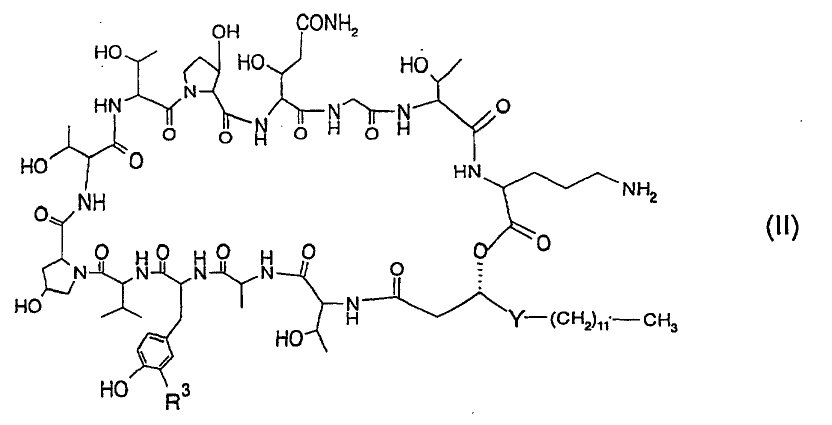 [wherein R³ is hydrogen or hydroxy, Y is -CH(CH₃)- or -CH₂-]

### Process B

Aerothricins of the Formula (I) [wherein R¹ is amino; Y is -CONH-, -CON(lower alkyl)-, -CH₂- or a single bond; Z is -NH- or -N(lower alkyl)-; R², R³, R⁴, R⁵, X and m are as defined above] can be prepared by condensation of a compound of the Formula (III), 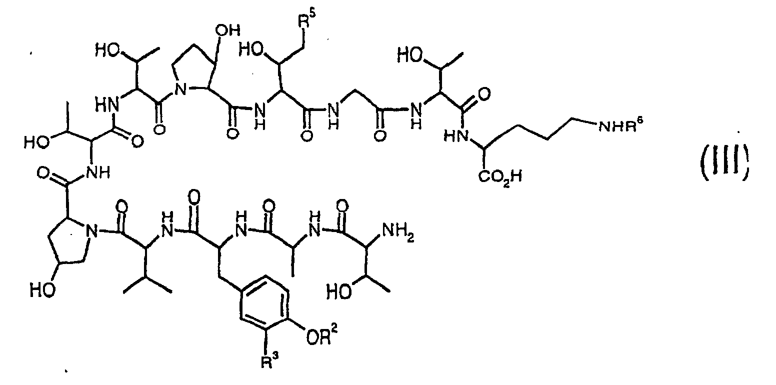 [wherein R⁶ is an amino protecting group; R², R³ and R⁵ are as defined above],
with a compound of the Formula (IV), 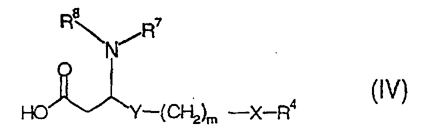 [wherein R⁷ is an amino protecting group; R⁸ is hydrogen or lower alkyl; R⁴, X, Y and m are as defined above],
using a carboxy activating agent for peptide synthesis, followed by selective removal of the amino protecting group R⁷ of the resulting linear peptide, the successive cyclization with a carboxy activating agent for peptide synthesis, and removal of the amino protecting group R⁶.

### Process C

Aerothricins of the Formula (I) [wherein R³ is a nitro group; R¹, R², R⁴, R⁵, X, Y, Z and m are as defined above] can be prepared by nitration of Aerothricins of the Formula (I) [wherein R³ is hydrogen; R¹, R², R⁴, R⁵, X, Y, Z and m are as defined above].

### Process D

Aerothricins of the Formula (I) [wherein R³ is an amino group; R¹, R², R⁴, R⁵, X, Y, Z and m are as defined above] can be prepared by reduction of the nitro group of Aerothricins of the Formula (I) [wherein R³ is a nitro group; R¹, R², R⁴, R⁵, X, Y, Z and m are as defined above].

### Process E

Aerothricins of the Formula (I) [wherein R³ is acylamino or (lower alkylcarbamoyl)amino; R¹, R², R⁴, R⁵, X, Y, Z and m are as defined above] can be prepared by acylation of the amino group of Aerothricins of the Formula (I) [wherein R³ is an amino group; R¹, R², R⁴, R⁵, X, Y, Z and m are as defined above] with acid chloride, acid anhydride, carboxylic acid/condensation agent or lower alkylcarbamoyl chloride, followed, if necessary, by removal of the amino protecting group.

### Process F

Aerothricins of the Formula (I) [wherein R¹ is (3-aminopropyl)amino, (2-cyanoethyl)amino, 3-amino-2-(aminomethy)propyl]amino or -N(R¹⁵)-COCH[NH(CH₂)₃NH₂]-R¹³ [wherein R¹³ and R¹⁵ are as defined above] can be prepared by reacting the amino group of Aerothricins of Formula (1) [wherein R¹ is an amino group or -N(R¹⁵)-COCH(NH₂)-R¹³ [wherein R¹³ and R¹⁵ are as defined above]; R², R³, R⁴, R⁵, X, Y, Z and m are as defined above] with acrylonitrile, ethoxymethylenemalononitrile or (1-ethoxyethylidene)malononitrile, followed by reduction of the resulting nitrile group(s) into amino group(s), and if necessary by removal of protecting group(s).

### Process G

Aerothricins of the Formula (I) [wherein R¹ is -N(R¹⁰)-R¹¹ [wherein R¹⁰ and R¹¹ are each independently selected from hydrogen, lower alkyl optionally substituted with one or more amino, guanidino, nitrogen containing heterocycle(s) or phenyl group(s) containing an amino, amidino or guanidino group] or -N(R¹⁵)-CO-CH[N(R¹⁰)R¹¹]-R¹³ [wherein R¹⁰ and R¹¹ are each a lower alkyl optionally substituted with one or more amino, amino-lower alkyl, guanidino, nitrogen containing heterocycle(s) or phenyl group(s) containing an amino, amidino or guanidino group; R¹³ and R¹⁵ are as defined above]; R², R³, R⁴, R⁵, X, Y, Z and m are as defined above] can be prepared by reductive alkylation of the amino group ofAerothricins of the Formula (I) [wherein R' is amino, (2-cyanoethyl)amino or -N(R¹⁵)-CO-CH[N(R¹⁰)R¹¹]-R¹³ [wherein R¹⁰ and R¹¹ are each independently a hydrogen atom or (2-cyanoethyl)amino; R¹³ and R¹⁵ are as defined above]; R², R³, R⁴, R⁵, X, Y, Z and m are as defined above] with an aldehyde of the Formula (V),

R⁹-CHO (V)

[wherein R⁹ is hydrogen, lower alkyl which may be further substituted with one or more protected amino, nitrogen containing heterocycle(s) or phenyl group(s) containing a protected amino group],
followed, if necessary, by removal of amino protecting group(s) or reduction of a cyano group.

### Process H

Aerothricins of the Formula (I) [wherein R¹ is -N(R¹⁰)-R¹¹ [wherein R¹⁰ and R¹¹ are each independently selected from hydrogen or heteroaryl substituted with one or two amino group(s)]; R², R³, R⁴, R⁵, X, Y, Z and m are as defined above] can be prepared by reacting the amino group of Aerothricins of the Formula (I) [wherein R¹ is an amino group; R², R³, R⁴, R⁵, X, Y, Z and m are as defined above] with a compound of the Formula (VI),

R¹²-Q (VI)

[wherein R¹² is a nitrogen containing heteroaryl which may be further substituted with a protected amino or nitro group, Q is a halogen atom such as chloro or bromo],
followed, if necessary, by removal of an amino protecting group or reduction of a nitro group.

### Process I-1

Aerothricins of the Formula (I) [wherein R¹ is 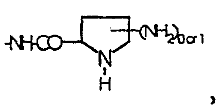 - NHCO-CH(NH₂)-R¹³ [wherein R¹³ is a residue derived from natural or unnatural amino acids] or -NHCO-R¹⁴ [wherein R¹⁴ is as defined above]; R², R³, R⁴, R⁵, X, Y, Z and m are as defined above] can be prepared by acylation of the amino group of Aerothricins of the Formula (I) [wherein R¹ is an amino group; R², R³, R⁴, R⁵, X, Y, Z and m are as defined above] with an acid of the Formula (VII) or (VII'),

HO(O=)C-CH(NH-R⁷)-R¹³ (VII)

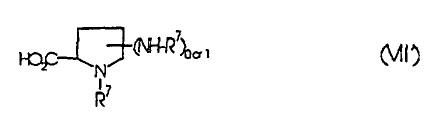

[wherein R¹³ is a residue derived from natural or unnatural amino acids whose functional group is suitably protected, R⁷ is an amino protecting group],
or an acid of the Formula (VIII),

HO(O=)C-R¹⁴ (VIII)

[wherein R¹⁴ is lower alkyl having one or more protected amino group(s), nitrogen containing heterocycle(s) or phenyl group(s) containing protected amino group];
followed, if necessary, by removal of the protecting group(s).

### Process I-2

Aerothricins of the Formula (I) wherein R¹ is 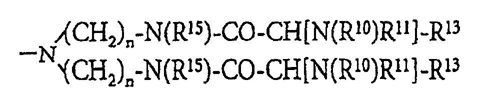 [wherein R¹⁰, R¹¹, R¹³, R¹⁵, and m are as defined above], or 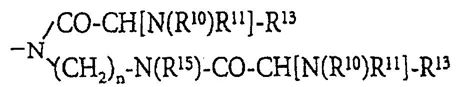 [wherein R¹⁰, R¹¹, R¹³, R¹⁵, and m are as defined above]
can be prepared by acylation of the amino group ofAerothricins of the Formula (I), wherein R¹ is -N(R¹⁰)-R¹¹ [wherein R¹⁰ and R¹¹ are both lower alkyl substituted with an amino group] or -N(R¹⁵)-CO-CH[N(R¹⁰)R¹¹]-R¹³ [wherein R¹⁵ is lower alkyl substituted with an amino group; R¹⁰, R¹¹, and R¹³ are as defined in Claim 1 with the proviso that the amino group(s) present in R¹⁰, R¹¹ and R¹³ are protected), with an acid of the Formula (VII)

HO(O=)C-CH(NH-R⁷)-R¹³ (VII)

[wherein R¹³ is a residue derived from natural or unnatural amino acids whose functional group is suitably protected, R⁷ is an amino protecting group];
followed by removal of the protecting group(s).

### Process J

Aerothricins of the Formula (I) [wherein R¹ is -N(R¹⁵)-CO-CH[N(R¹⁰)R¹¹]-R¹³ [wherein R¹⁰ and R¹¹ are hydrogen, R¹³ is as defined above and R¹⁵ is lower alkyl optionally substituted with one or more amino, guanidino, nitrogen containing heterocycle(s) or phenyl group(s) containing an amino, amidino or guanidino group], 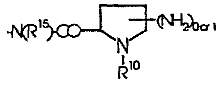 [wherein R¹⁰ is hydrogen and R¹⁵ is lower alkyl optionally substituted with one or more amino, guanidino, nitrogen containing heterocycle(s) or phenyl group(s) containing an amino, amidino or guanidino group], or -N(R¹⁵)-CO-R¹⁴ [wherein R¹⁵ is lower alkyl optionally substituted with one or more amino, guanidino, nitrogen containing heterocycle(s) or phenyl group(s) containing an amino, amidino or guanidino group, R¹⁴ is as defined above]; R², R³, R⁴, R⁵, X, Y, Z and m are as defined above] can be prepared by mono N-alkylation of the amino group of Aerothricins of the Formula (I) [wherein R¹ is an amino group; R², R³, R⁴, R⁵, X, Y, Z and m are as defined above] as described in process F, followed by acylation with a corresponding compound of the Formula (VII), (VII') or (VIII) as described in the process I, followed, if necessary, by removal of the protecting group(s).

### Process K

Aerothricins of the Formula (I) [wherein R¹ is a guanidino group, -N(R¹⁰)-R¹¹ [wherein R¹⁰ and R¹¹ are each independently selected from lower alkyl substituted with guanidino or phenyl group(s) containing a guanidino group], -N(R¹⁵)-CO-CH[N(R¹⁰)R¹¹]-R¹³ [wherein R¹⁰, R¹¹ and R¹³ are as defined above and R¹⁵ is lower alkyl optionally substituted with one or more guanidino group(s), nitrogen containing heterocycle(s) or phenyl group(s) containing a guanidino group] or -N(R¹⁵)CO-R¹⁴ [wherein R¹⁴ is lower alkyl substituted with one or more guanidino group(s), nitrogen containing heterocycle(s) or phenyl group(s) containing a guanidino group; R², R³, R⁴, R⁵, X, Y, Z and m are as defined above] can be prepared by reacting Aerothricins of the Formula (I) [wherein R¹ is an amino group; -N(R¹⁰)-R¹¹ [wherein R¹⁰ and R¹¹ are each independently selected from lower alkyl substituted with amino group(s) or phenyl group(s) containing an amino group], -N(R¹⁵)-CO-CH[N(R¹⁰)R¹¹]-R¹³ [wherein R¹⁰, R¹¹ and R¹³ are as defined above and R¹⁵ is lower alkyl optionally substituted with one or more amino group(s), nitrogen containing heterocycle(s) or phenyl group(s) containing an amino group]; or -NHCO-R¹⁴ [wherein R¹⁴ is lower alkyl substituted with one or more amino group(s), nitrogen containing heterocycle(s) or phenyl group(s) containing an amino group; R², R³, R⁴, R⁵, X, Y, Z and m are as defined above] with an activated amidine derivative.

### Process L

Aerothricins of the Formula (I) [wherein R² is lower alkyl or lower alkenyl optionally substituted with acyl, carboxy, carbamoyl, hydroxy, amino, mono-lower alkylamino or di-lower alkylamino; R¹, R³, R⁴, R⁵, X, Y, Z and m are as defined above] can be prepared by O-alkylation of the phenolic hydroxyl group of Aerothricins of the Formula (I) [wherein R² is hydrogen; R¹, R³, R⁴, R⁵, X, Y, Z and m are as defined above] with an alkylating agent.

### Process M

Aerothricins of the Formula (I) [wherein R³ is carboxyl, lower alkoxycarbonyl, lower alkyl, alkenyl or alkynyl which may be optionally substituted with hydroxy, amino, mono-lower alkylamino, di-lower alkylamino, lower alkoxycarbonyl or carbamoyl; R² is hydrogen; R¹, R⁴, R⁵, X, Y, Z and m are as defined above] can be prepared by iodination of Aerothricins of the Formula (I) [wherein R² and R³ are hydrogen; R¹, R⁴, R⁵, X, Y, Z and m are as defined above] with an iodination agent, followed by palladium(0) catalyzed coupling of the resulting iodo derivative of the Formula (I) [wherein R³ is an iodo; R¹, R², R⁴, R⁵, X, Y, Z and m are as defined above] with carbon monoxide, methyl acrylate and the like, and if necessary, by removal of the protecting group(s).

### Process N

Aerothricins of the Formula (I) [wherein R⁵ is -CN; R¹, R², R³, R⁴, X, Y, Z and m are as defined above] can be prepared by dehydration of the carbamoyl group of Aerothricins of the Formula (I) [wherein R⁵ is -CONH₂; R¹, R², R³, R⁴, X, Y, Z and m are as defined above] with a dehydrating agent, and if necessary, by removal of the amino protecting group(s).

### Process O

Aerothricins of the Formula (1) [wherein R⁵ is -CH₂NH₂; R¹, R², R³, R⁴, X, Y, Z and m are as defined above] can be prepared by reduction of the carbamoyl or cyano group of Aerothricins of the Formula (1) [wherein R⁵ is -CONH₂ or -CN; R¹, R², R³, R⁴, X, Y, Z and m are as defined above] with a reducing agent, and if necessary, by removal of the amino protecting group(s).

### Process P

Aerothricins of the Formula (I) [wherein R² is hydroxysufonyl; R¹, R³, R⁴, R⁵, X, Y, Z and m are as defined above] can be prepared by hydroxysulfonation of the tyrosine residue of Aerothricins of the Formula (I) [wherein R² is hydrogen; R¹, R³, R⁴, R⁵, X, Y, Z and m are as defined above], followed by removal of protecting group(s).

### Process O

Aerothricins of the Formula (I) [wherein -Y-(CH₂)m-X-R⁴ is *n*-tridecanyl or 1-methytridecanyl, R⁵ is -CONH₂, Z is an oxygen atom and R¹, R², and R³ are as defined above] can be prepared from the linear peptide of the Formula (IX) by the method outlined in Scheme 1.

The compound of above formula (III), wherein R², R³ and R⁵ are as defined above and R⁶ is an amino protecting group, with the proviso that when R⁵ is -CONH₂, then R² or R³ are other than hydrogen, and salts thereof are new and are also subject of the present invention. Furthermore, the linear peptides of Formulas (IX), (X) and (XII) shown in Scheme 1 and optionally salts thereof are new and are also subject of the present invention.

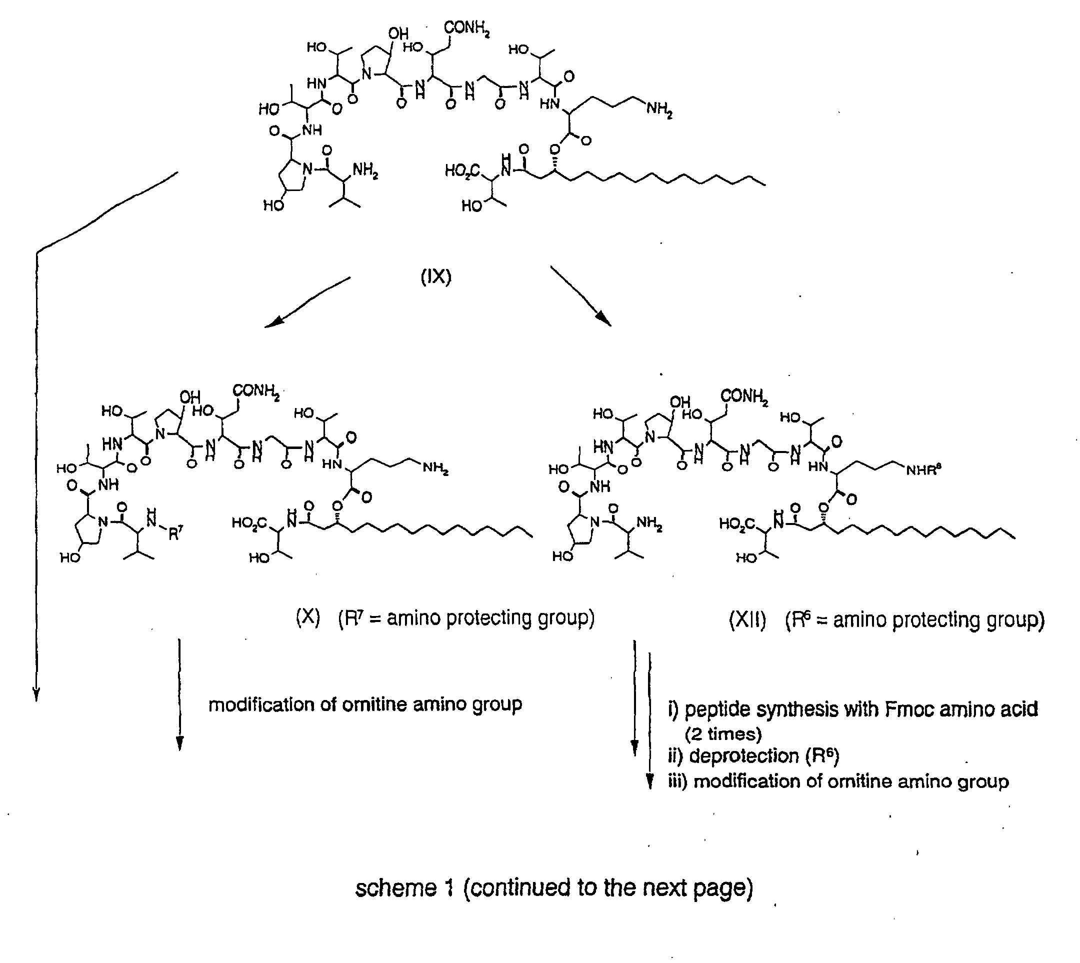

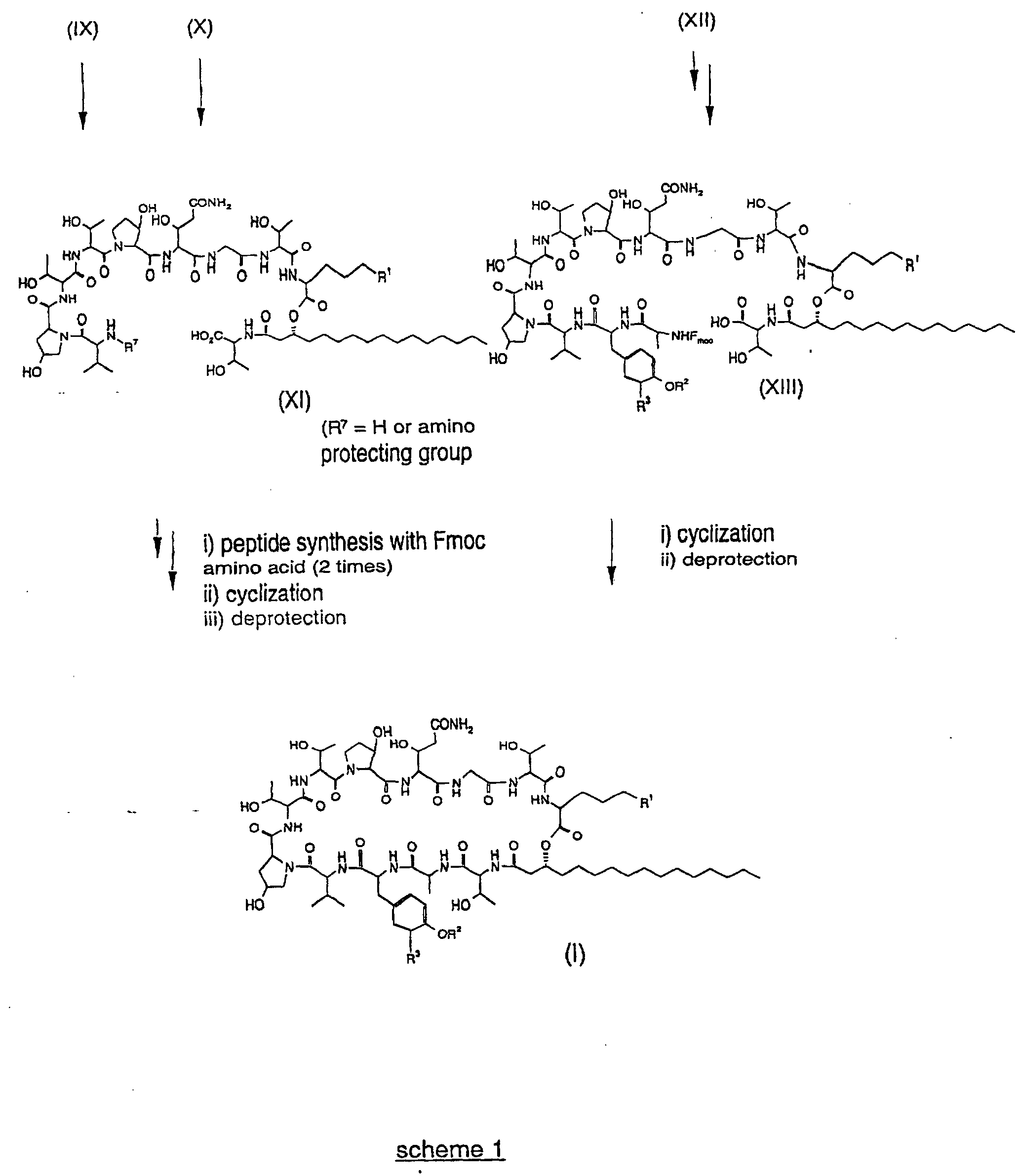

The Processes A to Q can be illustrated in more detail as follows:

### Process A

The microorganism used in the present invention can be any strains including mutants and variants belonging to *Deuteromycotina* capable of producing Aerothricins 1, 2 and 3. Especially preferred is strain NR 7379 which was isolated from fallen leaves collected at Kagoshima pref. in Japan, and identified as a strain belonging to *Deuteromycotina*.

The cultural and morphological characteristics of strain NR 7379 are as follows:

### 1. Cultural characteristics

Corn meal agar (CMA): Growth was not extensive. The colonies reached 11 mm in diameter from inoculum (4.5 mm diam. agar plug) after 14 days at 25°C. They were plane and pale cream yellow. The reverse side was pale cream yellow. Colorless and mucilaginous exudates were present.

Miura's medium (LCA): Growth was not extensive. The colonies reached 11 mm in diameter from inoculum after 14 days at 25°C. They were plane and pale cream yellow. The reverse side was pale cream yellow. Exudates were absent.

Malt extract agar (MEA): Growth was not extensive. The colonies were pustuliform and attained a diameter of 18 mm from inoculum after 14 days at 25°C. The color of colonies was light yellowish brown. The reverse side was of the same color. Exudates were colorless and mucilaginous.

Potato-dextrose agar (PDA): Growth was not extensive. The colonies were pustuliform and reached 14 mm in diameter from inoculum after 14 days at 25°C. The color and texture of colonies were similar to those on MEA. Exudates were colorless and mucilaginous.

Germination was observed between 5°C and 30°C on CMA, LCA, MEA, and PDA.

### 2. Morphological characteristics

Mycelia were partly immersed, partly superficial, branched, septate, and pale brown to cream yellow. Conidiophores were formed from immersed mycelium. They were hyaline, septate, branched, irregular. Conidiogenous cells were on distinct conidiophores or irregular hyphae. They were enteroblastic, phialidic, terminal or subterminal. Terminal or subterminal phialides were variable in length and shape. They were cylindrical to lageniform and their length and width were up to 5.5 to 10 µm and 2.5 to 5.5 µm respectively. Irregularly filiform Conidiophores with lateral conidiogenous cells immediately below septa were often formed. Conidia were one-celled, hyaline, smooth, globose to subglobose, 2.0 to 5.5 µm in length and 2.0 to 5.0 µm in width.

On the basis of these distinct cultural and morphological characteristics, the present strain belonged to *Deuteromycotina* designated as Deuteromycotina NR 7379.

The strain denoted as Deuteromycotina NR 7379 has been deposited with the National Institute of Bioscience and Human-Technology, Agency of Industrial Science and Technology, Japan in the name of Nippon Roche K.K., of 6-1, Shiba 2-chome, Minato-ku Tokyo, 105 Japan on June 16, 1998 under the Budapest Treaty as follows:
Deuteromycotina NR 7379 (PERM BP-6391).

The cultivation in accordance with the process provided by the present invention can be carried out in a culture medium which contains customary nutrients usable by the microorganism being cultivated. As carbon sources there can be mentioned, for example, glucose, sucrose, starch, glycerol, molasses, dextrin and mixtures thereof Nitrogen sources are, for example, soybean meal, cottonseed meal, meat extract, peptone, dried yeast, yeast extract, corn steep liquor, ammonium sulfate, sodium nitrate and mixtures thereof. Moreover, there may be added to the culture medium other organic or inorganic substances for promoting the growth of the microorganism and for increasing the production of Aerothricin 1. Examples of such substances are inorganic salts, such as calcium carbonate, sodium chloride, phosphates and the like.

The cultivation is carried out under aerobic conditions preferably in a liquid medium by submerged fermentation, or in a solid medium by static fermentation. A temperature of 20°C to 30°C, with an optimal temperature of 27°C is suitable for cultivation. The cultivation is preferably carried out at a pH of 3 to 9. The cultivation time depends on the conditions under which the cultivation is carried out. In general, it is sufficient to carry out the cultivation for 20 to 360 h.

For harvesting the objective Aerothricins 1, 2 and 3 from the cultures, separation methods which are usually employed to isolate metabolites produced by microbes from their cultures can be properly used. For example, Aerothricin 1, which is a methanol extractable amphoteric substance, is recovered advantageously by the following procedures.

That is, the whole culture solid obtained by solid state fermentation is extracted with an appropriate solvent to recover the proposed product. The solvents which can be used to extract the objective compound from the whole cultured solid include water-soluble organic solvents or hydrous solutions of water-soluble organic solvents, such as methanol, ethanol and hydrous alcohols.

For removing salts, water soluble substances, etc. from the resulting extract, use is made of, with advantage, solvent partition between water and water-immiscible organic solvents, such as *n*-butanol, ethyl acetate, etc. For removing coloring substances, fat-soluble substance or the like from the extract, use is made of, with advantage, solvent purification by methanol, ethanol, a mixture of acetonitrile-0.1% aqueous trifluoroacetic acid, etc.

For complete purification of Aerothricins, column chromatography is used with advantage. Carriers which can be used in such a column chromatography are such as YMC-GEL ODS (Yamamura Chemical Laboratories, Japan) or Preparative C18 (Waters Millipore Corporation). As an eluent, use is made of a solvent system consisting a mixture of aqueous trifluoroacetic acid and appropriate water-soluble organic solvents such as methanol, ethanol, acetonitrile, etc. The eluate fraction thus purified, which contains each component, can be subjected to concentration or freeze-drying to pulverize Aerothricins 1, 2 and 3.

Aerothricins 1, 2 and 3 were isolated as a trifluoroacetic acid salt, but the free Aerothricins 1, 2 and 3 can be prepared by the following procedure. Namely, Aerothricins 1, 2 and 3 trifluoroacetic acid salt are dissolved in water, to which was added one equivalent of sodium hydroxide, and the mixture is subjected to Sephadex LH-20 column Chromatography, followed by elution with a hydrous alcohol such as methanol-water, etc. to thereby obtain Aerothricins 1, 2 and 3 (free form), respectively.

### Process B

The starting compound of the Formula (III) can be prepared from Aerothricins of the Formula (I) [which includes Aerothricins 1 to 3 as well as those converted from Aerothricins 1 to 3 by use of a process selected from the processes C to Q] by the method similar to that described in WO 96/30399. This method comprises alkaline hydrolysis of the lactone ring followed by enzymatic cleavage of the fatty acid chain. The preferable amino protecting groups for R⁶ in the Formula (III) and R⁸ in the Formula (IV) are *tert*-butoxycarbony (Boc) and 9-fluorenylmethyloxycarbonyl (Fmoc), respectively.

The starting compound of the Formula (III) can also be prepared from the linear peptide of the Formula (IX), obtained by fermentation of *Deuteromycotina*, by conventional peptide synthesis mentioned herein after.

The starting compound of the Formula (IV) [wherein Y is -CONH-; R⁴, R⁸, and X are as defined above) can be prepared by condensation of the compound of the Formula (XIV), 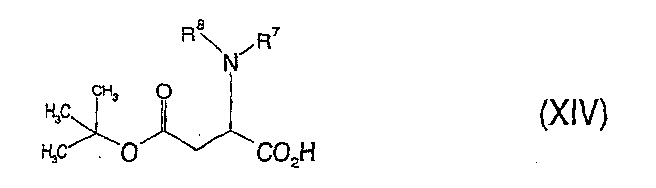 [wherein R⁷ is an amino protecting group, such as a Fmoc group, and R⁸ is as defined above],
with a compound of the Formula (XV),

R⁸NH-(CH₂)ₘ -X-R⁴ (XV)

[wherein R⁴, R⁸, X and m are as defined above],
followed by removal of the *tert*-butyl group. The compound of the Formula (XIV) is commercially available.

The starting compounds of the Formula (XV) [wherein X is a single bond, aryl, biphenyl or terphenyl group optionally containing one or more hetero atom(s) and/or being substituted with halogen atom(s) or lower alkyl] are commercially available or can be prepared by the methods similar to those described in EP 736 541 and Scheme 2: for example, LiAlH₄ reduction of the carboxyamide prepared from the carboxylic acid intermediates in Scheme 2 mentioned herein after, followed by protection of amino group with Fmoc chloride and the like.

The representative compounds of the Formula (IV) [wherein Y is -CONH- or -CON(lower alkyl)-; R⁴, R⁷, R⁸ and X are as defined above] are

HO₂C-CH₂CH(NHFmoc)-CONH-(CH₂)₁₀CH₃ ,

HO₂C-CH₂CH(NHFmoc)-CONH-(CH₂)₁₂CH₃ ,

HO₂C-CH₂CH(NHFmoc)-CONH-(CH₂)₁₄CH₃ ,

HO₂C-CH₂CH(NHFmoc)-CONH-(CH₂)₁₁CH(CH₃)₂ ,

HO₂C-CH₂CH(NHFmoc)-CONH-(CH₂)₁₀-CH=CH-CH₂CH₃ ,

HO₂C-CH₂CH(NHFmoc)-CONH-(CH₂)₈-CH=CH-(CH₂)₃CH₃ ,

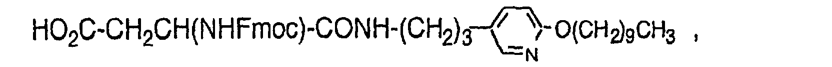

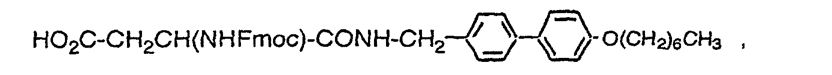

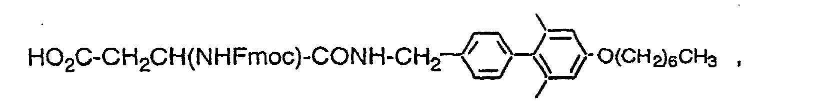

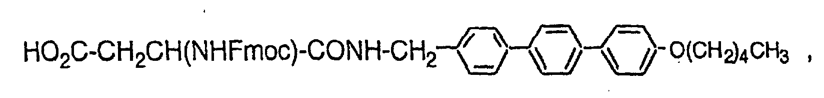

HO₂C-CH₂CH(NHFmoc)-CON(CH₃)-(CH₂)₁₂CH₃ ,

HO₂C-CH₂CH(NHFmoc)-CON(CH₃)-(CH₂)₁₄CH₃ ,

and the like.

The starting compound of the Formula (IV) [wherein Y is a single bond or -CH₂-; R⁴, R⁸, and X are as defined above] can be prepared by Michael addition of (R)-(+)-N-benzyl-1-phenylethylamine to a compound of the Formula (XVI), 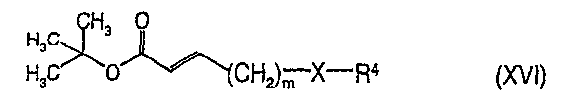 [wherein R⁴, X and m are as defined above] in the presence of strong base such as LDA [cf. Tetrahedron Asymmetry, 2 (3), 183 (1991)], followed by i) N-debenzylation by catalytic hydrogenation, ii) protection of the resulting primary amine with Fmoc chloride and the like, and iii) removal of *tert*-butyl group.

The starting compounds of the Formula (XVI) can be prepared by the method outlined in the following Scheme 2.

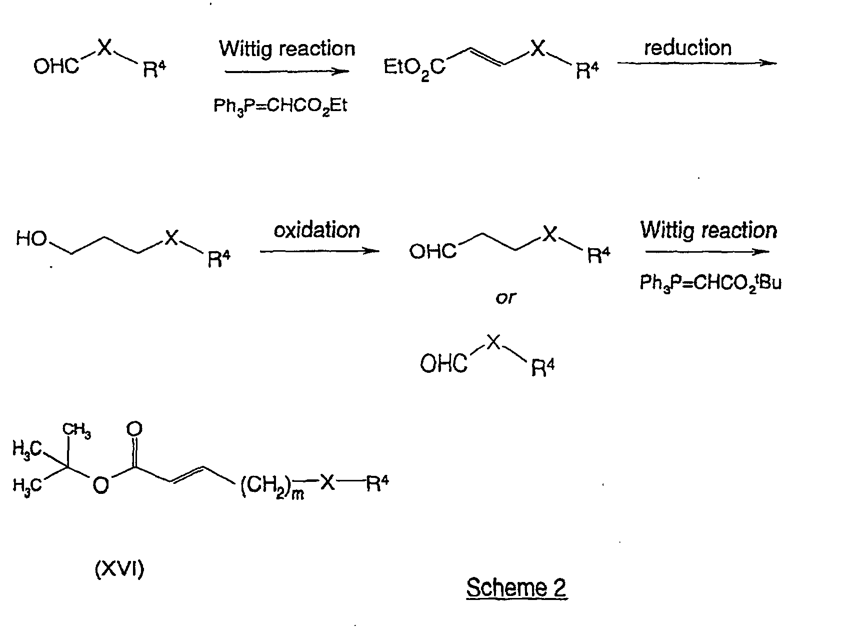

The compounds of the Formula (XVI), wherein m is 4, can be prepared by repeating the steps 1 to 3 in Scheme 2 before the last Wittig reaction.

The representative compounds of the Formula (IV) [wherein Y is a single bond or -CH₂-; R⁴, R⁷, and X are as defined above] are:

HO₂C-CH₂CH(NHFmoc)-(CH₂)₁₂CH₃ ,

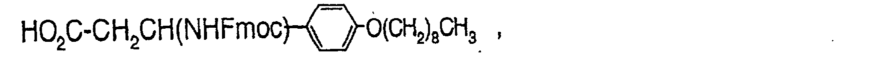

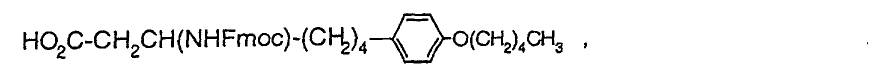

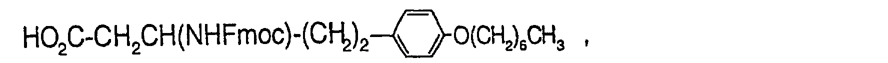

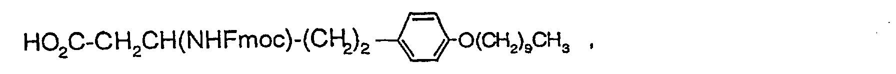

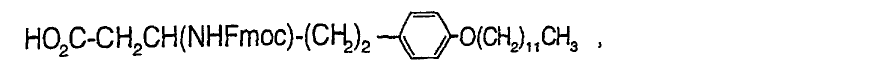

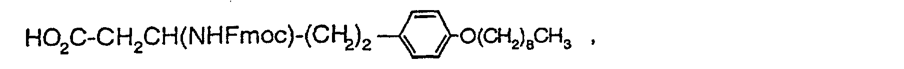

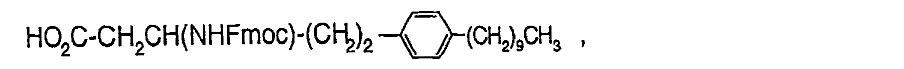

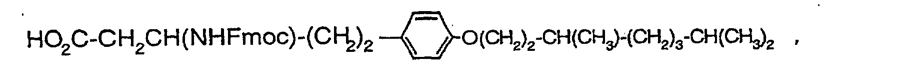

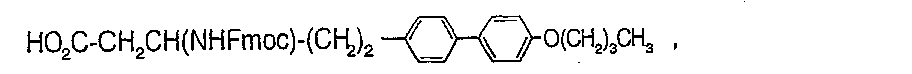

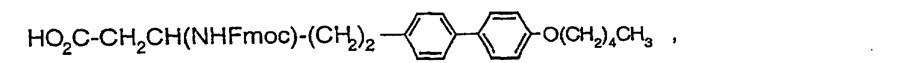

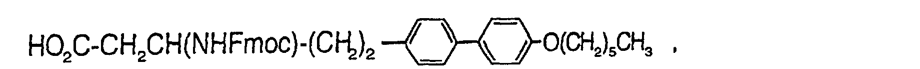

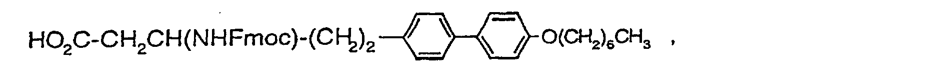

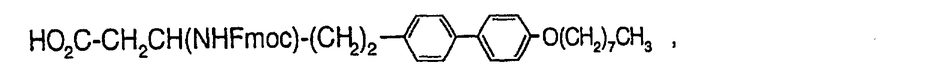

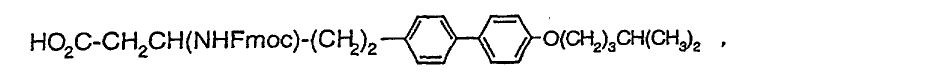

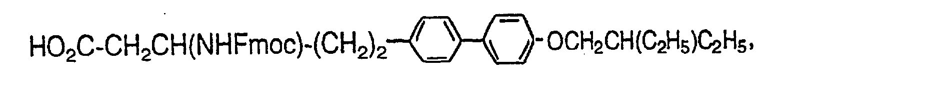

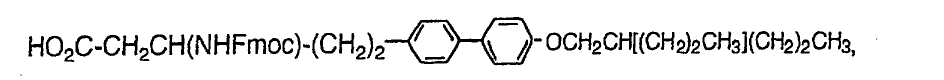

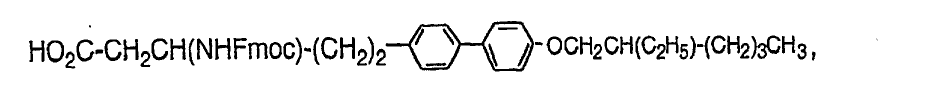

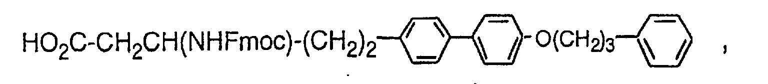

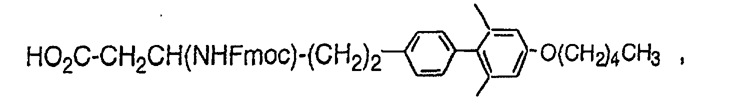

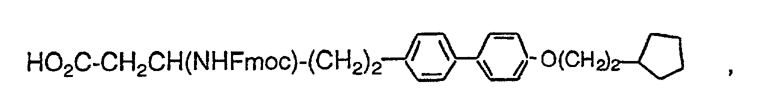

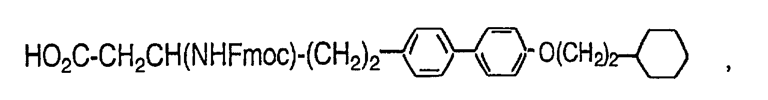

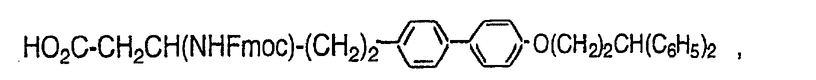

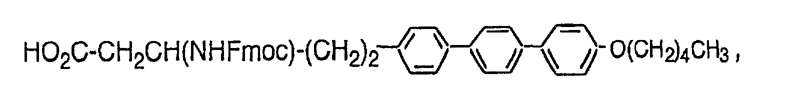

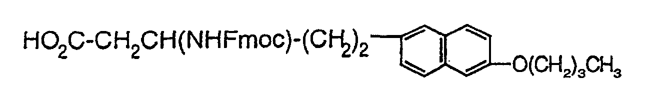

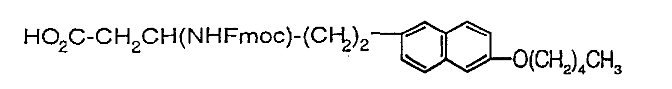

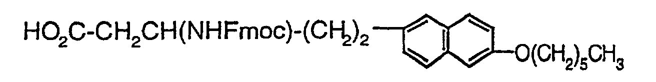

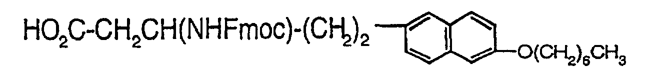

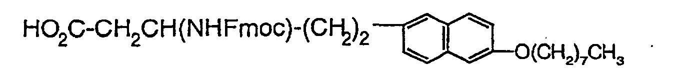

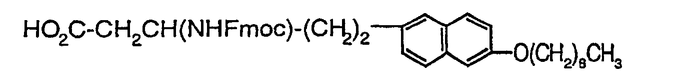

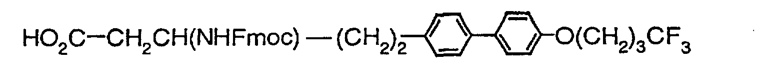

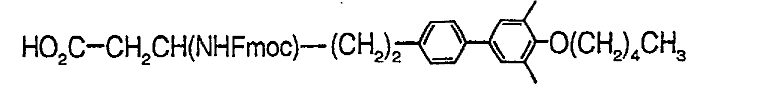

HO₂C-CH₂CH(N(CH₃)Fmoc)-(CH₂)₁₄CH₃

and the like.

The first peptide bond formation reaction as well as the cyclization of the resulting linear peptide can be performed by the method known to those skilled in the peptide chemistry [cf. The practice of Peptide Synthesis, M. Bodansky and A. Bodansky / 2nd ed., 1994 (Springer-Verlag)]. The preferable condensation agent is BOP-HOBt, PyBOP™-HOBt, PyBroP™-HOBt and the like [coupling reagents: commercially available (cf. The Combinatorial Chemistry Catalog, Feb., 1997; Novabiochem.)].

The reaction can be carried out in a solvent such as methanol, ethanol, pyridine, N,N-dimethylformamide, N-methylpyrrolidone and the like in the presence or absence of a base such as triethylamine, di-isopropylethylamine, pyridine and the like at a temperature between -20°C and +50°C, preferably at 0°C to +25°C.

### Process C

Nitration of the Aerothricin of the Formula (I) can be performed by the method known to those skilled in the art; typically by sodium nitrite/acetic acid, tetranitromethane/pyridine and the like.

The reaction can be carried out at a temperature between -20° and 0°C, preferably at 0°C.

### Process D

Reduction of nitro group(s) can be done by the method known to those skilled in the art; typically by catalytic hydrogenation using a catalyst such as palladium-C, platinum oxide and the like.

The reaction can be carried out at room temperature in a solvent such as methanol, ethanol, acetic acid, and the like.

### Processes E and I

N-acylation of an amino group existing in R¹ or R³ of the Formula (I) can be done with acid anhydride or carbamoyl chloride by the method known to those skilled in the art, or with carboxylic acid using condensation agents such as dicyclohexylcarbadiimide, BOP, HBTU, TNTU, PyBroP™, PyBOP™, TBTU, TSTU, HOBt and the like, or the combination of two of them.

The reaction can be carried out in a solvent such as methanol, ethanol, pyridine, N,N-dimethylformamide, N-methylpyrrolidone and the like in the presence or absence of a base such as triethylamine, di-isopropylethylamine, pyridine and the like at a temperature between -20°C and +50°C, preferably at 0°C to +25°C.

The removal of the amino protecting group, when using N-protected amino acid for the condensation reaction, can be done by the method known to those skilled in the art, e.g. treatment with trifluoroacetic acid for Boc group, or piperidine for Fmoc group.

### Process F

N-monoalkylation of an amino group existing in R¹ of the Formula (I) can be done using acrylonitrile, ethoxymethylene-malononitrile or (1-ethoxyethylidene)malononitrile according to the method described in Organic Synthesis col. Vol. III, page 93, followed by reduction of the resulting nitrile group by catalytic hydrogenation or reduction with sodium borohydride/cobalt chloride, borane-methylsulide complex and the like [cf. J. Med. Chem., 37, 222 (1994)].

### Process G

N-alkylation of the primary or secondary amino group existing in R¹ of the Formula (I) can be done by the conventional reductive alkylation with aldehyde derivatives of the Formula (V) using a reducing agent such as sodium cyanoborohydride in the presence or absence of weak acid such as acetic acid.

The reaction can be carried out at room temperature in a solvent such as methanol, ethanol, acetic acid and the like.

### Process H

Examples of the compound (R¹²-Q) of Formula (VI) for the substitution reaction are 2-bromo-5-nitropyridine, 2-chloropyrimidine, chloropyrazine and the like.

The substitution reaction can be carried out at a temperature between -20°C and +50°C, preferably at 0°C to +25°C, in a solvent such as acetonitrile,
N,N-dimethylformamide and the like in the presence or absence of acid scavenger such as potassium carbonate, triethylamine, di-isopropylethyamine and the like.

### Process J

The first mono-N-alkylation of an amino group existing in R¹ of the Formula (I) can be done by the method described in Process F. The successive N-acylation can be done by the method described in Process E and I.

### Process K

The conversion of an amino group existing in R¹ of the Formula (I) into a guanidino group can be done by an activated amidine derivative such as 3,5-dimethyl-1H-pyrazole-1-carboxamidine, formamidinesulfonic acid, benztriazol-1-carboxamidinium tosylate and the like.

The reaction can be carried out in a solvent such as methanol, ethanol, water, N,N-dimethylformamide and the like at a temperature between 0°C and ∼50°C, preferably at 20°C to ∼30°C.

### Process L

O-alkylation of a hydroxy group of the tyrosine residue in the Formula (I) can be done by the method known to those skilled in the art in the presence of acid scavenger such as sodium carbonate, diisopropylethylamine and the like [Org. Synth., Coll. vol. IV 836 (1963)].

The reaction can be carried out in a solvent such as methanol, ethanol, acetone, N,N-dimethylformamide and the like at a temperature between 0°C and +50°C, preferably at 0°C to +25°C.

### Process M

Iodination at the ortho position of the phenol group in a tyrosine residue can be done by treatment of Aerothricins of the Formula (I), wherein R² is hydrogen, with iodine monochloride or sodium iodide/aqueous sodium hypochlorite in a solvent such as methanol, ethanol and the like at room temperature.

The palladium(0) catalyzed coupling reaction with carbon monoxide, methyl acrylate and the like can be carried out using a palladium(0) catalyst such as Pd(OAc)₂, Pd(OAc)₂(dppp)₂ in a solvent such as methanol, ethanol, N,N-dimethylformamide, acetonitrile and the like in the presence of base such as triethylamine at a temperature between 20°C and +100°C, preferably at 20°C to +70°C [Bioorg. Med. Chem. Lett., 7 (22), 2879 (1997)].

### Process N

Dehydration of the carbamoyl group (R⁵) of the Formula (I) can be done by Burgess reagent [available from Aldrich], cyanuric chloride, oxalyl chloride and the like [cf. J. Med. Chem., 37, 222 (1994)].

The reaction can be carried out in a solvent such as N,N-dimethylformamide, N-methylpyrrolidone and the like at room temperature.

### Process O

The reduction of the carbamoyl or cyano group (R⁵) of the Formula (I) can be done by sodium borohydride/cobalt chloride, borane-methylsulfide complex and the like [cf. J. Med. Chem., 37, 222 (1994)].

The reaction can be carried out in a solvent such as methanol, ethanol and the like at room temperature.

### Process P

The hydroxysulfonation of the tyrosine residue of the Formula (I) can be carried out by sulfurtrioxide-DMF complex, sulfurtrioxide-pyridine complex or sulfurtrioxide-triethylamine complex in a solvent such as N,N-dimethylformamide,
N-methylpyrrolidone, 1,4-dioxane, tetrahydrofuran and the like at a temperature between -30 to +70 °C, preferably at room temperature [cf. J. Chem. Soc. Perkin Trans, (6) 1739 (1990)].

### Process Q

The reactions involved in this process can be done by the methods similar to those described in the process B - O.

The starting material, a linear peptide of the Formula (IX) can be obtained by cultivating a microorganism belonging to *Deuteromycotina* under aerobic conditions in an aqueous or a solid medium and isolating a linear peptide of Formula (IX) from the culture.

The microorganism used in the present invention can be any strains including mutants and variants belonging to *Deuteromycotina* capable of producing a linear peptide of Formula (IX). Especially preferred is strain NR 7379 which was isolated from fallen leaves collected at Kagoshima pref. in Japan, and identified as a strain belonging to *Deuteromycotina*.

The strain denoted as Deuteromycotina NR 7379 has been deposited with the National Institute of Bioscience and Human-Technology, Agency of Industrial Science and Technology, Japan on June 16, 1998 under the Budapest Treaty as follows:
Deuteromycotina NR 7379 (FERM BP-6391).

The cultivation in accordance with the process provided by the present invention can be carried out in a culture medium which contains customary nutrients usable by the microorganism being cultivated. As carbon sources there can be mentioned, for example, glucose, sucrose, starch, glycerol, molasses, dextrin and mixtures thereof. Nitrogen sources are, for example, soybean meal, cottonseed meal, meat extract, peptone, dried yeast, yeast extract, corn steep liquor, ammonium sulfate, sodium nitrate and mixtures thereof. Moreover, there may be added to the culture medium other organic or inorganic substances for promoting the growth of the microorganism and for increasing the production of a linear peptide of Formula (IX). Examples of such substances are inorganic salts such as, calcium carbonate, sodium chloride, phosphates and the like.

The cultivation is carried out under aerobic conditions preferably in a liquid medium by submerged fermentation, or in a solid medium by static fermentation. A temperature of 20°C to 30°C, with an optimal temperature of 27°C is suitable for cultivation. The cultivation is preferably carried out at a pH of 3 to 9. The cultivation time depends on the conditions under which the cultivation is carried out. In general, it is sufficient to carry out the cultivation for 120 to 672 h.

For harvesting the objective linear peptide of Formula (IX) from the cultures, separation methods which are usually employed to isolate metabolites produced by microbes from their cultures can be properly used. For example, a linear peptide of Formula (IX), which is a methanol extractable amphoteric substance, is recovered advantageously by the following procedures.

That is, the cultured broth obtained by liquid fermentation is extracted with an appropriate solvent to recover the proposed product. The solvents which can be used to extract the objective compound from the cultured broth include water-soluble organic solvents or hydrous solutions of water-soluble organic solvents, such as methanol, ethanol and hydrous alcohols, or water-immiscible organic solvent such as *n*-BuOH.

For removing salts, water soluble substances, etc. from the resulting extract, use is made of, with advantage, solvent partition between water and water-immiscible organic solvents, such as *n*-butanol, ethyl acetate, etc. For removing coloring substances, fat-soluble substance or the like from the extract, use is made of, with advantage, solvent purification by methanol, ethanol, a mixture of acetonitrile-0.1% aqueous trifluoroacetic acid, etc.

For complete purification of a linear peptide of Formula (IX), column chromatography is used with advantage. Carriers which can be used in such a column chromatography are such as Capcel Pak C18 UG80 (Shiseido Co. LTD, Japan). As an eluent, use is made of a solvent system consisting of a mixture of aqueous trifluoroacetic acid and appropriate water-soluble organic solvents such as methanol, ethanol, acetonitrile, etc. The eluate fraction thus purified, which contains a linear peptide of Formula (IX), can be subjected to concentration or freeze-drying to pulverize a linear peptide of Formula (IX).

A linear peptide of Formula (IX) was isolated as a trifluoroacetic acid salt, but the free linear peptide of Formula (IX) can be prepared by the following procedure. Namely, the linear peptide of Formula (IX) trifluoroacetic acid salt is dissolved in water, to which was added one equivalent of sodium hydroxide, and the mixture is subjected to Sephadex LH-20 column chromatography, followed by elution with a hydrous alcohol such as methanol-water, etc. to thereby obtain a linear peptide of Formula (IX).

The linear peptide of Formula (IX) provided by the present invention does not exhibit any fungicidal activity against various fungi, however, can be a key intermediate to produce potent antifungal agent such as Aerothricins.

The present invention is also concerned with acid addition salts of Aerothricins. The acid addition salt can be obtained as trifluoroacetic acid salt after normal course of isolation. The salt thus obtained may be dissolved in water and passed through an anion exchange column bearing the desired anion. The eluate containing the desired salt may be concentrated to recover the salt as a solid product.

The Aerothricins of Formula (I) may be converted to a corresponding salt by virtue of the presence of the tertiary nitrogen atoms.

The acid addition salt of Aerothricins of Formula (I) can be obtained by treatment of the free base of Aerothricins with at least a stoichiometric amount of an appropriate acid, such as mineral acids, e.g., hydrochloric acid, hydrobromic acid, sulfuric acid, nitric acid, phosphoric acid, and the like, and organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Typically, the free base is dissolved in an inert organic solvent such as ethanol, methanol, and the like, and the acid added in a similar solvent. The temperature is maintained at about 40°C. The resulting salt precipitates spontaneously or may be brought out of solution with a less polar solvent.

The acid addition salts of the Aerothricins of Formula (I) may be converted to the corresponding free base by treatment with at least a stoichiometric amount of a suitable base such as sodium or potassium hydroxide, potassium carbonate, sodium bicarbonate, ammonia, and the like.

Above aerothricins exhibit broad fungicidal activity against various fungi and can be used as agents for treatment and prophylaxis of fungal infectious diseases. The *in vitro* and *in vivo* antifungal activity (see Tables 2 and 3) as well as the toxicity to hepatocytes (see Table 4) of the representative Aerothricins of Formula (I) are shown as follows:

### 1. In vitro antifungal activities

The *in vitro* antifungal activities of the representative Aerothricins of the present study were evaluated by determining the 50% inhibitory concentration (IC₅₀), which was calculated as the lowest concentration of an antifungal to inhibit the growth of fungus to 20% turbidity compared with the drug-free control growth spectrophotometrically.

The IC₅₀ values were determined by the broth micro-dilution procedure based on NCCLS Approved Standard with the following minor modifications (National Committee for Clinical Laboratory Standards. (1997) Reference method for broth dilution antifungal susceptibility testing for yeasts. Approved standard. Document M27-A). Yeast Nitrogen Base (YNB; Difco Lab.) supplemented with 1% glucose and 0.25% K₂HPO₄ was used as testing medium for yeast, the same medium solidified with 0.2% low melting point agarose (BRL) was used for filamentous fungi. Inoculum size was 1-3 x 10⁴ cells/ml, and incubation was performed for 1-2 days at 35°C.

**Table 2:**

| *In vitro* Antifungal activity, IC₅₀ (µg/ml) | | | |
|---|---|---|---|
| | *Candida albicans* CY1002 | *Aspegillus fumigatus* CF1003 | *Fusarium solani* CF1088 |
| Aerothricin 1 | 0.03 | 0.06 | 0.21 |
| Aerothricin 5 | 0.03 | 0.07 | 0.19 |
| Aerothricin 12 | 0.09 | 0.10 | 2.20 |
| Aerothricin 31 | 0.07 | 0.49 | 0.70 |
| Aerothricin 36 | 0.08 | 0.05 | 1.00 |
| Aerothricin 39 | 0.09 | 0.17 | 0.70 |
| Aerothricin 41 | 0.08 | 0.03 | 2.40 |
| Aerothricin 43 | 0.05 | 0.04 | 0.70 |
| Aerothricin 45 | 0.07 | 0.08 | 2.30 |
| Aerothricin 46 | 0.09 | 0.08 | 1.80 |
| Aerothricin 47 | 0.09 | 0.11 | 1.40 |
| Aerothricin 53 | 0.11 | 0.09 | 2.30 |
| Aerothricin 54 | 0.15 | 0.17 | 0.74 |
| Aerothricin 55 | 0.04 | 0.04 | 0.39 |
| Aerothricin 57 | 0.14 | 0.05 | 1.30 |
| Aerothricin 75 | 0.15 | 0.10 | 1.40 |
| Aerothricin 77 | 0.13 | 0.10 | 0.67 |
| Aerothricin 95 | 0.14 | 0.10 | 0.74 |
| Aerothricin 132 | 0.30 | 0.10 | 0.19 |
| Aerothricin 134 | 0.12 | 0.19 | 0.03 |
| Aerothricin 135 | 0.18 | 0.20 | 0.05 |
| Aerothricin 136 | 0.20 | 0.28 | 0.04 |

### 2. In vivo antifungal efficacy

### 2-1: Murine systemic candidiasis

*In vivo* antifungal efficacy of Aerothricins of the present invention against systemic candidiasis is shown in the following Table 3-1. Mice of a conventional immunocompetent mouse strain, Crj: CD-1 (ICR) were used for experimental infection models of systemic candidiasis. 4 weeks old Crj: CD-1 (ICR) mice were used for systemic candidiasis by injecting *Candida albicans* 5x10⁶ conidia/mouse via the tail vein. Treatments were given twice (0, 4 h after infection) on the first day and once daily on following 2 days for systemic candidiasis (b.i.d x 1 day followed by q.d. x 2 days), intravenously (i.v.). 50% of effective dose (ED₅₀) values was calculated from the survival number at each dose on day 14.

**Table 3-1:**

| *In vivo* antifungal activity against systemic candidiasis in mice, ED₅₀ (mg/kg) on day 14 | |
|---|---|
| Aerothricin 5 | 0.3 |
| Aerothricin 16 | 0.3 |
| Aerothricin 18 | 0.6 |
| Aerothricin 36 | 0.6 |
| Aerothricin 41 | 0.3 |
| Aerothricin 42 | 0.6 |
| Aerothricin 45 | 0.3 |
| Aerothricin 46 | 0.4 |
| Aerothricin 50 | <0.3 |
| Aerothricin 55 | <0.3 |
| Aerothricin 65 | 0.6 |

### 2-2: Murine pulmonary aspergillosis

*In vivo* antifungal efficacy of Aerothricins of the present invention against pulmonary aspergillosis is shown in the following Table 3-2. Murine pulmonary aspergillosis was created in cortisone-treated (250mg/kg, twice sub-cutaneous treatments on 3 days before and on the infection day) ICR male mouse. Conidia of *A*.*fumigatus* (2.5 x 10⁵ conidia/mouse) were infected intratracheally to these mice, and treatments were carried out once daily for 4 days. The efficacy of each drug was determined from the survival number, and 50% of effective dose (ED₅₀) was calculated from the survival number at each dose on the 14 days.

**Table 3-2:**

| *In vivo* antifungal activity against pulmonary aspergillosis in mice, ED₅₀ (mg/kg) on day 14 | |
|---|---|
| Aerothricin 132 | 5.2 |
| Aerothricin 134 | 5.8 |
| Aerothricin 135 | 8.6 |

### 3. In vitro hepatotoxicity test

The mouse hepatocytes were isolated by a collagenase digestion and cultured in microtest plates. The hepatocyte monolayers were exposed to the test Aerothricins in the culture system for 1 day. After the culture period, the hepatocytes were observed under a microscope and evaluated morphologically. The degree of the morphological alteration (degeneration) of the hepatocytes by the test Aerothricins were compared with WF11243 and LY303366.

**Table 4:**

| Cytotoxicity to hepatocyte (µg/ml) | |
|---|---|
| Aerothricin 14 | >100 |
| Aerothricin 15 | >100 |
| Aerothricin 21 | >100 |
| Aerothricin 34 | >100 |
| Aerothricin 38 | >100 |
| Aerothricin 45 | >100 |
| Aerothricin 47 | >100 |
| Aerothricin 48 | >100 |
| Aerothricin 53 | >100 |
| Aerothricin 65 | >100 |
| Aerothricin 67 | >100 |
| Aerothricin 72 | >100 |
| Aerothricin 81 | >100 |
| Aerothricin 132 | >100 |
| Aerothricin 134 | >100 |
| Aerothricin 135 | >100 |
| WF11243 (= Aerothricin 3) | 100 |
| LY303366 | 10 |

5 mg/kg and 30 mg/kg of Aerothricin 1 administration to mice for 4 weeks showed no acute toxicity.

Therefore, the novel Aerothricins of Formula (I) as well as pharmaceutically acceptable salts thereof exhibit potent antifungal activity against various fungal infections, including Aspergillosis, in mice over a wide range of dosages and are useful as antifungal agents. Moreover, the Aerothricins provided by this invention are much less cytotoxic to hepatocytes than the known cyclic peptide derivatives (WF11243 and LY303366).

Aerothricins of the present invention may also be useful for inhibiting or alleviating *Pneumocystis carinii* infections in immune-compromised patients.

The novel Aerothricins of Formula (I) as well as pharmaceutically acceptable salts thereof are highly active fungicidal agents. They are active against a variety of fungal species including *Candida spp*., *Aspergillus spp*., *Fusarium spp., Mucor spp*. and *Absidia spp*..

The daily dosage level of Aerothricins of Formula (I) is from 0.1 to 50 mg/kg (in divided doses) when administered by either the oral or parenteral route. Thus tablets or capsules of Aerothridns can be expected to contain from 5 mg to 0.5 g of active compound for administration singly or two or more at a time as appropriate. In any event the actual dosage can be determined by the physician and it may be varied upon the age, weight and response of the particular patient

Therefore, a preferable embodiment of the composition according to the present invention is a nasally administrable composition comprising a physiologically active cyclic peptide and a physiologically acceptable powdery or crystalline polyvalence metal carrier, wherein an effective amount of any one of cyclic peptides such as cyclospoline A, vancomycin, daptomycin, aerothricins, echinocandins and pneumocandins is dispersed homogeneously in and adsorbed homogeneously onto the polyvalence metal carrier whose mean particle size is in the range of 20 to 250 µm, preferably in the range of 20 to 100 µm and more preferably in the range of 20 to 60 µm, in the presence of an absorption enhancer whose mean particle size is not more than 250 µm, preferably from 20 µm to 180 µm.

Hence, one preferable embodiment of the composition according to the present invention is a physiologically active cyclic peptide composition in powdery form, which is formulated into a nasally administrable preparation, in which a physiologically effective amount of a cyclic peptide is homogeneously dispersed in and adsorbed onto a divalence metal carrier selected from aluminum compound, calcium compound, magnesium compound, silicon compound, iron compound and zinc compound, whose mean particle size is not more than 250 µm, preferably not more than 100 µm and more preferably 20 µm to 60 µm, in the presence of an absorption enhancer selected from fine powder of rice, glutinous rice, starch, gelatine, dextrin, hydroxypropyl cellulose, hydroxypropylmethyl cellulose, polyvinyl pyrrolidone, egg yolk lecithin, gum arabic, tragacanth and a mixture thereof. More preferable absorption enhancer is fine powder of glutinous rice, starch, gelatine, hydroxypropyl cellulose, hydroxypropylmethyl cellulose, polyvinyl pyrrolidone, tragacanth and a mixture thereof. The most preferable absorption enhancer is fine powder of glutinous rice. The mean particle size of the absorption enhancer is not more than 250 µm, preferably from 20 µm to 180 µm.

Another preferable embodiment of the composition according to the present invention is a nasally administrable physiologically active composition in powdery form, in which a physiologically effective amount of a cyclic peptide is homogeneously dispersed in and adsorbed onto the carrier selected from hydroxyapatite, calcium carbonate, calcium lactate, magnesium stearate, preferably calcium carbonate whose mean particle size is in the range of 20 to 100 µm, in the presence of an absorption enhancer selected from the fine powder of rice, glutinous rice, starch, gelatine, dextrin, hydroxypropyl cellulose, hydroxypropylmethyl cellulose, polyvinyl pyrrolidone, egg yolk lecithin, gum arabic, tragacanth and a mixture thereof, and most preferably fine powder of glutinous rice. The mean particle size of the absorption enhancer is not more than 300 µm, preferably from 20 µm to 180 µm.

Another preferable embodiment of the composition according to the present invention is a nasally administrable physiologically active composition in powdery form, in which a physiologically effective amount of a cyclic peptide is homogeneously dispersed in and adsorbed onto the organic carrier selected from the fine grain powder of rice, wheat, buck wheat, barley, soybean, corn, millet, foxtail millet and the like. The mean particle size of the organic carrier is not more than 300 µm, preferably from 20 µm to 180 µm.

Furthermore, the most preferable embodiment of the composition according to the present invention is a nasally administrable antifungal cyclic peptide composition in powdery form, in which a physiologically effective amount of peptide selected from aerothricins, echinocandins and pneumocandins is homogeneously dispersed in and adsorbed onto the carrier selected from hydroxyapatite, calcium carbonate, calcium lactate, magnesium stearate, preferably calcium carbonate whose mean particle size ranges from 20 µm to 60 µm, in the presence of an absorption enhancer selected from the fine powder of rice, glutinous rice, starch, gelatine, dextrin, hydroxypropyl cellulose, hydroxypropylmethyl cellulose, polyvinyl pyrrolidone, egg yolk lecithin, gum arabic and tragacanth, preferably glutinous rice, whose mean particle size is not more than 250 µm, preferably from 20 µm to 180 µm, or a mixture thereof.

The physiologically effective amount of the cyclic peptide to be contained in the composition according to the present invention may vary with factors such as the active substance to be chosen, the disease to be treated, desired number of administration, desired effect of therapy, and so on. When administering the composition of the present invention through the nasal cavity, the physiologically effective amount of the cyclic peptide may be determined on the basis of a comparison of its bioavailability relative to other known preparations containing the same active substance.

The physiologically active cyclic peptide composition according to the present invention may contain a physiologically active cyclic peptide at a rate of from approximately 5% to approximately 50%, preferably from approximately 10% to approximately 40%, more preferably from approximately 20% to approximately 30%, with respect to the total weight of the preparation.

The physiologically active peptide composition according to the present invention can achieve a high extent of nasal absorption when it contains carrier (for example, hydroxyapatite, calcium carbonate, calcium lactate, magnesium stearate as typical carrier) at a rate of from 50% to approximately 95%, preferably from approximately 60% to approximately 95%, more preferably from approximately 70% to approximately 90%, with respect to the total weight of the preparation.

The physiologically active peptide composition according to the present invention can achieve a high extent of nasal absorption when it contains absorption enhancer (for example, fine powder of rice, glutinous rice, corn starch, and hydrxypropyl cellulose-H as a typical enhancer) at a rate of from 0.5% to approximately 15%, preferably from approximately 1% to approximately 10%, more preferably from approximately 1% to approximately 5%, with respect to the total weight of the preparation.

The physiologically active peptide composition according to the present invention can be prepared by homogeneously dispersing a physiologically effective amount of the cyclic peptide in a physiologically acceptable powdery or crystalline carrier containing either a polyvalence metal or organic carrier, preferably in a physiologically acceptable powdery or crystalline water insoluble polyvalence metal carrier having a mean particle size in the range of 20 to 250 µm, in the presence of an absorption enhancer whose mean particle size is not more than 250 µm, preferably from 20 to 180 µm, and adsorbing said active substance thereonto.

For example, to prepare the composition according to the present invention, an antifungal cyclic peptide as active substance is admixed with a carrier [e.g., hydroxyapatite, calcium carbonate or calcium lactate as calcium compound; magnesium stearate as magnesium compound; or aluminum hydroxide as aluminum compound] and, an absorption enhancer [e.g. fine powder of rice, glutinous rice, starch, gelatine, dextrin, hydroxypropyl cellulose, hydroxypropylmethyl cellulose, polyvinyl pyrrolidone, egg yolk lecithin, gum arabic, tragacanth or a mixture thereof]. Then distilled water is added to the mixture at a rate of from 10% to approximately 60%, preferably from approximately 10% to approximately 40%, more preferably from approximately 10% to approximately 30%, with respect to the total weight of the preparation, and mixed well until the mixture becomes pasty solid. The mixture is then dried *in vacuo* or freeze-dried at a temperature between -5 and -30°C. The resulting powdery residue is, if necessary, mixed with a lubricant such as calcium stearate at a rate of from 0.1% to approximately 5%, preferably from approximately 1% to approximately 5%, with respect to the total weight of the preparation, and passed through mesh with 180 to 250 µm in diameter, preferably 180 µm.

The carrier to be used in the present invention may have a mean particle size of 20 to 250 µm, preferably of 20 to 100 µm and most preferably of approximately 20 µm to approximately 60 µm. On the other hand, it is preferred that the physiologically active cyclic peptide is pulverized to the smallest possible particles, the mean particle size being smaller than 20 µm, preferably smaller than 10 µm.

More specifically, when aerothricin is selected as antifungal cyclic peptide, a physiologically effective amount of the aerothricin is admixed with calcium carbonate. Then distilled water is added to the mixture at a rate of from approximately 10% to approximately 30%, preferably approximately 25% with respect to the total weight of the preparation, and mixed well until the mixture becomes pasty solid. The mixture is then dried *in vacuo* or freeze-dried at a temperature between -5 and -30°C. Calcium stearate or magnesium stearate is added as a lubricant to the resulting powdery residue at a rate of from 0.1% to approximately 5%, preferably from approximately 1% with respect to the total weight of the preparation, and passed through the mesh with 180 to 250 µm in diameter, preferably 180 µm.

Another embodiment, when aerothricin is selected as antifungal cyclic peptide and glutinous rice powder as an absorption enhancer, a physiologically effective amount of the aerothricin is admixed with calcium carbonate and fine powder of glutinous rice. Then distilled water is added to the mixture at a rate of approximately 25% with respect to the total weight of the preparation, and mixed well until the mixture become pasty solid. The mixture is then dried *in vacuo* or freeze-dried at a temperature between -5 and -30°C. Calcium stearate or magnesium stearate is added as a lubricant to the resulting powdery residue at a rate of from 0.1% to approximately 5%, preferably from approximately 1% with respect to the total weight of the preparation, and passed through the mesh with 180 to 250 µm in diameter, preferably 180 µm.

In order to prevent loss of activity of the physiologically active cyclic peptide, the nasally administrable composition may then be filled in capsules of a low-grease type and packaged in an appropriate form, preferably in a closed form, by combining blister packing with aluminum packaging.

The absolute bioavailability (=AUC (i.n.)/AUC (i.v.)) of the representative nasally administrable antifungal cyclic peptide composition according to the present invention was determined in monkeys after single intranasal administration, and the results are shown in table 5. The preparation of each composition is described in the working examples. A nasally applicable composition of the antifungal cyclic peptides was intranasally administered in cynomolgus monkeys using a jetmizer at a dose of 80 mg (total weight of composition which contained 20 mg of the active substance)/body. Blood samples were collected via a limb vein in heparinized syringes at pre-dose, and at 10 min, 30 min, 1, 2, 4, 8, 12 and 24 hours after the administrations. The drug concentration was measured by means of LC-MS. To calculate the bioavailability, 20 mg of the corresponding active substance was administered intravenously (i.v.) to the monkey, and the area under the curve (AUC) value was compared with that obtained after intranasal (i.n.) administration.

**Table 5**

| Composition | Active substance | Carrier | Absorption enhancer | Cmax (µg/ml) | AUC inf. (µg.hr/ml) | Bioavailability (%) |
|---|---|---|---|---|---|---|
| Example 32 | Aerothricin 106 | CaCO₃ | none | 5.62 | 61.7 | 20.2 |
| Example 33 | Aerothricin 106 | CaCO₃ | glutinous rice | 7.06 | 97.0 | 31.8 |
| Example 34 | Aerothricin 106 | rice powder | none | 4.80 | 41.6 | 13.6 |
| Example 35 | Aerothricin 106 | corn starch | none | 5.68 | 53.6 | 17.6 |
| Example 36 | Aerothricin 133 | CaCO₃ | none | 5.38 | 53.2 | 13.5 |
| Example 37 | Aerothricin 132 | CaCO₃ | none | 2.51 | 55.5 | 22.6 |

The highest absolute bioabailability was attained when the nasally administrable antifungal cyclic peptide composition consisting of calcium carbonate and glutinous rice powder (Example 33) is used. The plasma concentration of the active substance exceeded the therapeutic concentration for 24 hours at the above mentioned dosage.

Therefore, the nasally administrable physiologically active cyclic peptide composition in the present invention can be used for treatment of disease such as systemic fungal infections.

The following examples illustrate some preferred physiologically active cyclic peptides used in accordance with the present inventiona as well as preferred methods for the preparation of the nasally administrable physiologically active cyclic peptide composition in the present invention.

In the following Examples, the products were analyzed and purified by HPLC using a reverse phase column selected from those listed below. The mixed solivent consisted of 0.05% trifluoroacetic acid-water: 0.005% trifluoroacetic acid-acetonitrile with the appropriate ratio described in each working Examle.

### HPLC column:

| | |
|---|---|
| Column A | CAPCELL PAK18, UG-120, 4.6x250nm |
| Column B | CAPCELL PAK18, UG-120, 10x250nm |
| Column C | CAPCELL PAK18, UG-80, 20x250nm |
| Column D | CAPCELL PAK18, SG-120, 4.6x250nm |
| Column E | CAPCELL PAK18, SG-120, 10x250nm |
| Column F | ODS-80Ts, 10x250nm |

In the following working Examples, Aerothricins were obtined as trifluoroacetic acid salts unless otherwise indicated.

### Example 1

### Preparation of (R)-3-(9-fluorenylmethoxycarbonylamino)-5-(4'-heptyloxybiphenyl-4-yl)-pentanoic acid

a) Preparation of 4-bromo-4'-heptyloxybiphenyl
   To a stirred solution of 4-bromo-4'-hydroxybiphenyl (5.05 g, 20.2 mmol) in DMF (100 ml) were added K₂CO₃ (4.20 g, 30.4 mmol) and 1-bromoheptane (4.14 ml, 26.4 mmol), and then the mixture was heated at 80°C. After being stirred at 80°C for 20 h, the mixture was cooled to room temperature. The mixture was diluted with Et₂O (250 ml) and then the solution was washed with sat. brine (150 ml x 2). The organic layer was dried over anhydrous Na₂SO₄ and concentrated *in vacuo*. The residue was recrystallized from CH₂Cl₂-petroleum ether to give 4-bromo-4'-heptyloxybiphenyl (6.21 g, 88%) as a white solid; FAB-MS: m/z 347[MH⁺].
b) Preparation of 4-formyl-4'-heptyloxybiphenyl
   To a cold (0°C) stirred solution of 4-bromo-4'-heptyloxybiphenyl (6.21 g, 17.9 mmol) in THF (120 ml) was added *n*-BuLi (1.66 M solution in hexane, 32.3 ml, 53.6 mmol). After the mixture was stirred at 0°C for 20 min., DMF (4.85 ml, 62.6 mmol) was added at -78°C. The mixture was stirred at -78°C for additional 20 min., and then quenched with sat. aqueous NH₄Cl. The mixture was diluted with EtOAc (220 ml), and then successively washed with sat. aqueous NH₄Cl. (125 ml) and sat. brine (100 ml). The organic layer was dried over anhydrous Na₂SO₄ and concentrated *in vacuo*. The residue was purified by column chromatography on silica gel (EtOAc/hexane, 1:20) to give 4-formyl-4'-heptyloxybiphenyl (2.21 g, 42%) as a white amorphous powder.
c) Preparation of 3-(4'-heptyloxybiphenyl-4-yl)acrylic acid ethyl ester
   To a stirred solution of 4-formyl-4'-heptyloxybiphenyl (2.21 g, 7.46 mmol) in benzene (40 ml) was added Ph₃P=CHCOOEt (5.19 g, 14.9 mmol), and then the mixture was heated at 60°C. After being stirred at 60°C for 3 h, the mixture was cooled to room temperature and concentrated *in vacuo*. The residue was purified by column chromatography on silica gel (CH₂Cl₂/hexane, 1:2) to give 3-(4'-heptyloxybiphenyl-4-yl)acrylic acid ethyl ester (2.66 g, 97%) as a white amorphous powder.
   FAB-MS: m/z 367[MH⁺],
   ¹H NMR: δ 0.90 (t, *J*=6.8Hz, 3H), 1.25-1.55 (m, 8H), 1.35 (t, *J*=7.1Hz, 3H), 1.81 (quint, *J*=6.6Hz, 2H), 4.00 (t, *J*=6.4Hz, 2H), 4.28 (q, *J*=7.1Hz, 2H), 6.46 (d, *J*=16.0Hz, 1H), 6.94-7.00 (m, 2H), 7.50-7.60 (m, 6H), 7.72 (d, *J*=16.0Hz, 1H).
d) Preparation of 3-(4'-heptyloxybiphenyl-4-yl)propionic acid ethyl ester
   To a stirred solution of 3-(4'-heptyloxybiphenyl-4-yl)acrylic acid ethyl ester (2.65 g, 7.23 mmol) in CH₂Cl₂ (60 ml) was added palladium on activated carbon (Pd ca.10wt%, 1.07 g), and then the mixture was set under H₂ atmosphere. After being stirred for 2 h, the mixture was filtered through a pad of Celite and washed with CH₂Cl₂. Filtrate and washings were combined and concentrated *in vacuo* to give 3-(4'-heptyloxybiphenyl-4-yl)propionic acid ethyl ester (crude, 2.74 g) which was used for the next step without further purification.
   ¹H NMR: δ 0.90 (t, *J*=6.6Hz, 3H), 1.25 (t, *J*=7.3Hz, 3H), 1.29-1.56 (m, 8H), 1.75-1.86 (m, 2H), 2.65 (t, *J*=7.8Hz, 2H), 2.98 (t, *J*=7.8Hz, 2H), 3.99 (t, *J*=6.6Hz, 2H), 4.14 (q, *J*=7.3Hz, 2H), 6.93-6.98 (m, 2H), 7.25 (d, *J*=8.6Hz, 2H), 7.43-7.52 (m, 4H).
e) Preparation of 3-(4'-heptyloxybiphenyl-4-yl)propan-1-ol
   To a cold (0°C) stirred suspension of LiAlH4 (0.47 g, 12.4 mmol) in THF (20 ml) was added a solution of 3-(4'-heptyloxybiphenyl-4-yl)propionic acid ethyl ester (crude, 2.74 g) in THF (30 ml). After being stirred for 30 min. at room temperature, the mixture was quenched with H₂O at 0°C. The mixture was filtered through a pad of Celite and washed with CH₂Cl₂. The filtrate and washings were combined and concentrated in *vacuo*. The residue was purified by column chromatography on silica gel (EtOAc/hexane, 2:3) to give 3-(4'-heptyloxy-biphenyl-4-yl)propan-1-ol (2.27 g, 96% for 2 steps) as a white amorphous powder.
   EI-MS: m/z 326[M⁺],
   ¹H NMR: δ 0.90 (t, *J*=6.8Hz, 3H), 1.21-1.55 (m, 8H), 1.81 (quint, *J*=6.6Hz, 2H), 1.86-2.00 (m, 2H), 2.75 (t, *J*=7.3Hz, 2H), 3.71 (t, *J*=6.6Hz, 2H), 3.99 (t, *J*=6.6Hz, 2H), 6.92-7.00 (m, 2H), 7.25 (d, *J*=7.9Hz, 2H), 7.44-7.55 (m, 4H).
f) Preparation of 3-(4'-heptyloxybiphenyl-4-yl)propionaldehyde
   To a cold (0°C) stirred solution of 3-(4'-heptyloxybiphenyl-4-yl)propan-1-ol (2.26 g, 6.92 mmol) in CH₂Cl₂ (60 ml) were added molecular sieves 4A powder (5.17 g) and PCC (5.25 g, 24.4 mmol). After being stirred for 2 h at room temperature, Et₂O (20 ml) was added to the mixture. The reaction mixture was transferred to a short silica gel column and eluted with CH₂Cl₂. The eluate was concentrated in *vacuo* to give 3-(4'-heptyloxybiphenyl-4-yl)propionaldehyde (crude, 2.45 g) which was used for the next step without further purification.
g) Preparation of 3-(4'-heptyloxybiphenyl-4-yl)pent-2-enoic acid *tert*-butyl ester
   To a stirred solution of 3-(4'-heptyloxybiphenyl-4-yl)propionaldehyde (crude, 2.45 g) in benzene (150 ml) was added Ph₃P=CHCOO*t*-Bu (5.21 g, 13.8 mmol), and then the mixture was heated at 60°C. After being stirred for 30 min. at 60°C, the mixture was cooled to room temperature and concentrated *in vacuo*. The residue was purified by column chromatography on silica gel (EtOAc/hexane, 1:30) to give 3-(4'-heptyloxybiphenyl-4-yl)pent-2-enoic acid *tert*-butyl ester (1.95 g, 67% for 2 steps) as a white amorphous powder.
   EI-MS: m/z 422[M⁺],
   ¹H NMR: 8 0.90 (t, *J*=6.6Hz, 3H), 1.21-1.51 (m, 8H), 1.49 (s, 9H), 1.74-1.87 (m, 2H), 2.47-2.58 (m, 2H), 2.79 (t, *J*=7.3Hz, 2H), 3.99 (t, *J*=6.6Hz, 2H), 5.81 (d.t., *J*=1.5Hz, 15.5Hz, 1H), 6.87-7.01 (m, 3H), 7.23 (d, *J*=7.9Hz, 2H), 7.44-7.53 (m, 4H).
h) Preparation of (R)-3-[benzyl-((R)-1-phenylethyl)amino]-5-(4'-heptyloxybiphenyl-4-yl)pentanoic acid *tert*-butyl ester
   To a cold (0°C) stirred suspension of (R)-N-benzyl-1-phenylethylamine hydrochloride (3.28 g, 13.2 mmol) in THF (40 ml) was added *n*-BuLi (1.61 M solution in hexane, 15.0 ml, 24.2 mmol). After the mixture was stirred for 25 min. at 0°C, a solution of 3-(4'-heptyloxybiphenyl-4-yl)pent-2-enoic acid *tert*-butyl ester (1.94 g, 4.38 mmol) in THF (30 ml) was added at -78°C. After the mixture was stirred for additional 20 min. at -78°C, the reaction mixture was quenched with sat. aqueous NH₄Cl. and concentrated in *vacuo*. The residue was diluted with sat. aqueous NH₄Cl. (200 ml), and then extracted with CH₂Cl₂ (200 ml x 2). The combined extracts were dried over anhydrous Na₂SO₄ and concentrated *in vacuo*. The residue was purified by column chromatography on silica gel (EtOAc/hexane, 1:40) to give (R)-3-[benzyl-((R)-1-phenylethyl)amino]-5-(4'-heptyloxybiphenyl-4-yl)pentanoic acid *tert*-butyl ester (2.83 g, quant.) as a colorless oil.
   EI-MS: m/z 633[M⁺],
   ¹H NMR: δ 0.91 (t, *J*=6.6Hz, 3H), 1.24-1.55 (m, 13H), 1.38 (s, 9H), 1.57-2.04 (m, 6H), 2.52-2.69 (m, 1H), 2.97-3.10 (m, 1H), 3.37-3.49 (m, 1H), 3.55 (ABq, *J*=15.0 Hz, 1H), 3.85 (ABq, *J*=15.0Hz, 1H), 3.88 (q, *J*=6.9Hz, 1H), 4.00 (t, *J*=6.6Hz, 1H), 6.96 (d, *J*=8.6Hz, 2H), 7.16 (d, *J*=8.2Hz, 2H), 7.21-7.53 (m, 16H).
i) Preparation of (R)-3-amino-5-(4'-heptyloxybiphenyl-4-yl)pentanoic acid *tert*-butyl ester
   To a stirred solution of (R)-3-[benzyl-((R)-1-phenylethyl)amino]-5-(4'-heptyloxy-biphenyl-4-yl)pentanoic acid *tert*-butyl ester (2.82 g, 4.45 mmol) in EtOAc (50 ml) were added AcOH (2.5 ml) and Pd(OH)₂ on carbon (Pd(OH)₂ ca. 20wt%, 1.07 g), and then the mixture was set under H₂ atmosphere. After being stirred for 15 h, the mixture was filtered through a pad of Celite and washed with MeOH. The filtrate and washings were combined, and concentrated *in vacuo* to give (R)-3-amino-5-(4'-heptyloxybiphenyl-4-yl)pentanoic acid *tert*-butyl ester (crude, 3.14 g) which was used for the next step without further purification.
j) Preparation of (R)-3-(9-fluorenylmethoxycarbonylamino)-5-(4'-heptyloxybiphenyl-4-yl)pentanoic acid *tert*-butyl ester
   To a stirred suspension of (R)-3-amino-5-(4'-heptyloxybiphenyl-4-yl)pentanoic acid *tert*-butyl ester (crude, 3.14 g) in 50% aqueous 1,4-dioxane (40 ml) were added Na₂CO₃ (1.19 g, 11.2 mmol) and FmocCl (1.28 g, 4.95 mmol). After being stirred for 1 h, the mixture was diluted with sat. brine (100 ml) and extracted with CH₂Cl₂ (100 ml x 3). The combined extracts were dried over anhydrous Na₂SO₄ and concentrated *in vacuo* to give (R)-3-(9-fluorenylmethoxycarbonylamino)-5-(4'-heptyloxybiphenyl-4-yl)pentanoic acid tert-butyl ester (crude, 3.34 g) which was used for the next step without further purification.
   FAB-MS: m/z 668[M⁺+Li],
   ¹H NMR: δ 0.81 (t, *J*=6.6Hz, 3H), 1.15-1.44 (m, 8H), 1.35 (s, 9H), 1.62-1.93 (m, 4H), 2.29-2.68 (m, 4H), 3.84-4.02 (m, 1H), 3.88 (t, *J*=6.6Hz, 2H), 4.13 (t, *J*=6.8Hz, 1H), 4.25-4.41 (m, 2H), 5.27 (d, *J*=9.2Hz, 1H), 6.85 (d, *J*=8.6Hz, 2H), 7.06-7.42 (m, 10H), 7.51 (d, *J*=7.3Hz, 2H), 7.66 (d, *J*=7.6Hz, 2H).
k) Preparation of (R)-3-(9-fluorenylmethyloxycarbonylamino)-5-(4'-heptyloxybiphenyl-4-yl)pentanoic acid
   To a stirred solution of (R)-3-(9-fluorenylmethoxycarbonyl-amino)-5-(4'-heptyloxybiphenyl-4-yl)pentanoic acid *tert*-butyl ester (crude, 3.34 g) in CH₂Cl₂ (20 ml) was added TFA (20 ml) dropwise. After being stirred for 1 h at room temperature, the mixture was concentrated *in vacuo*. The residue was purified by column chromatography on silica gel (MeOH/CH₂Cl₂, 1:20) to give (R)-3-(9-fluorenylmethoxycarbonylamino)-5-(4'-heptyloxybiphenyl-4-yl)pentanoic acid (2.07 g, 77% in 3 steps) as a white amorphous powder.
   FAB-MS: m/z 606[MH⁺],
   ¹H NMR: δ 0.88 (t, *J*=6.6Hz, 3H), 1.21-1.51 (m, 8H), 1.64-2.04 (m, 2H), 1.78 (q, *J*=6.6Hz, 2H), 2.27-2.78 (m, 4H), 3.91-4.07 (m, 1H), 3.96 (t, *J*=6.6Hz, 2H), 4.20 (t, *J*=6.6Hz, 1H), 4.34-4.56 (m, 2H), 5.09-5.28 (m, 1H), 6.92 (d, *J*=8.9Hz, 2H), 7.10-7.49 (m, 10H), 7.57 (d, *J*=7.3Hz, 2H), 7.73 (d, *J*=7.3Hz, 2H).

The starting compounds of Formula (IV) [wherein Y is a single bond or -CH₂-] used in the process B were prepared according to the method similar to that described above.

### Example 2

### Preparation of (S)-3-(9H-fluorenylmethoxycarbonylamino)-N-undecylsuccinamic acid

a) To a solution of (S)-2-(9H-fluoren-9-ylmethoxycarbonyl-amino)succinic acid (150 mg, 0.36 mmol), BOP reagent (162 mg, 0.36 mmol) and HOBT hydrate (56 mg, 0.36 mmol) in N,N-dimethylformamide (0.2 ml) was added N,N-diisopropylethylamine (64 µl, 0.36 mmol). After being stirred for 30 min at room temperature, 1-aminoundecane (79 µl, 0.37 mmol) was added. The mixture was stirred at room temperature for 3 h. The reaction mixture was diluted with water and extracted with Et₂O. The combined extracts were washed with water, dried over anhydrous sodium sulfate, filtered and concentrated. Purification of the residue by silica gel column chromatography (using *n*-hexane:ethyl acetate = 3:1 as an eluent) gave (S)-3-(9H-fluoren-9-ylmethoxycarbonylamino)-N-undecylsuccinamic acid *tert*-butyl ester (169 mg, 82% yield) as a colorless amorphous solid.
   FAB-MS (m/z): 565[MH⁺],
   ¹H-NMR(CDCl₃) δ: 0.88 (3H, t, *J*=7Hz), 1.24 (16H, m), 1.45 (11H, m), 2.58 (1H, dd, *J*₁=17Hz, *J*₂=7Hz), 2.91 (1H, dd, *J*₁=17Hz, *J*₂=4Hz), 3.23 (2H, q, *J*=7Hz), 4.22 (1H, t, *J*=7Hz), 4.42-4.45 (3H, m), 5.94 (1H, broad d, *J*=8Hz), 6.43 (3H, broad s), 7.31 (2H, t, *J*=7Hz), 7.41 (2H, t, *J*=7Hz), 7.58 (2H, d, *J*=7Hz), 7.77 (2H, d, *J*=7Hz).
b) A solution of (S)-3-(9H-fluoren-9-ylmethoxycarbonylamino)-N-undecylsuccinamic acid *tert*-butyl ester (113 mg, 0.2 mmol) in TFA (2 ml) was stirred at room temperature for 30 min. After completion of the reaction, TFA was removed by evaporation *in vacuo*. Purification of the residue by silica gel column chromatography (using dichloromethane:methanol = 9:1 as an eluent) gave (S)-3-(9H-fluoren-9-ylmethoxycarbonylamino)-N-undecylsuccinamic acid (101 mg, 99% yield) as a colorless amorphous solid.
   FAB-MS (m/z): 507[MH⁺],
   ¹H-NMR(CDCl₃) δ: 0.87 (3H, t, *J*=7Hz), 1.23 (16H, m), 1.46 (2H, m), 2.62∼2.80 (1H, m), 2.90-3.05 (1H, m), 3.21 (2H, m), 4.20 (1H, t, *J*=7Hz), 4.44 (2H, d, *J*=6Hz), 4.53 (1H, broad s), 5.98 (1H, m), 6.52 (1H, broad s), 7.30 (2H, t, *J*=7Hz), 7.40 (2H, t, *J*=7Hz), 7.56 (2H, d, *J*=7 Hz), 7.76 (2H, d, *J*=7Hz).

The starting compounds of the general Formula (IV) [wherein Y is -CONH- or -CON(CH₃)-] used in the process B were prepared according to the method described above.

### Example 3

### Preparation of N-Boc-Aerothricin 3 (Compound A)

To a solution of Aerothricin 3 (10.0 g, 6.07 mmol) in MeOH (1500 ml) was added triethylamine (2.54 ml, 18.2 mmol), di-*tert*-butyl dicarbonate (13.9 ml, 60.7 mmol) successively. After the mixture was stirred at room temperature for 18 h, the solvent was evaporated *in vacuo*. The residue was dissolved in MeOH (ca. 10 ml) and the solution was added to the diethylether (1500 ml). The resultant precipitate was filtered and washed with diethylether to give 9.9 g of N-Boc-Aerothricin 3 (Compound A) as a pale yellow amorphous solid, which was used for further structural modification in the working examples described below without further purification.

### Example 4

### Preparation of Aerothricins 1, 2 and 3

a) Solid fermentation
   A 0.1 ml portion of the frozen culture of Deuteromycotina NR 7379 (FERM BP-6391) in 10% (v/v) glycerol solution was defrosted and inoculated into a 500-ml Erlenmeyer flask containing 100 ml of a medium consisting of 2% glucose, 1% potato starch, 1.5% glycerol, 1% Toast soya (Nissin Seiyu), 0.35% yeast extract (Nippon Seiyaku), 0.25% Polypepton (Nihon Seiyaku), 0.3% NaCl, 0.5% CaCO₃, 0.005% ZnSO₄·7H₂O, 0.0005% CuSO₄·5H₂O, and 0.0005% MnSO₄·4H₂O. The pH of the medium was not adjusted. The seed culture was incubated on a rotary shaker at 27°C for 7 days at 220 rpm. 2 ml of the seed culture was transferred into a 3-liter Erlenmeyer flask containing a solid medium consisting of 200 g pressed barley, 0.12 g yeast extract (Difco), 0.06 g sodium tartarate, 0.06 g KH₂PO₄, and 120 ml water. The fermentation was carried out at 27°C under static condition. The production reached maximum at around 240 h of fermentation and the culture was subjected to the isolation procedure of Aerothricins 1, 2 and 3.
   The cultured solid (10 kg) obtained was added methanol (40 L) and the mixture was stirred, followed by removal filtration to obtain methanol extract (39 L). The methanol extract thus obtained was concentrated to dryness under reduced pressure, and the residue (64.8 g) was added ethyl acetate (1 L) and water (1 L). And the mixture was stirred, followed by removal of the ethyl acetate layer.
   Furthermore, the aqueous layer was likewise washed with ethyl acetate (1 L) twice. The remaining aqueous layer was extracted with *n*-butanol (1 L) three times. The extracts thus obtained were combined and concentrated to dryness under reduced pressure, and the residue (28.5 g) was dissolved into a mixture (250 ml) of acetonitrile-0.1% aqueous trifluoroacetic acid (1:1). After removal of the insoluble materials by centrifugation, the solution thus obtained was evaporated to dryness under reduced pressure, and the residue was added methanol (300 ml) and the mixture was stirred, followed by removal filtration to obtain the methanol solution (280 ml). The methanol soluble materials (9.3 g) thus obtained were then subjected to a column chromatography on reversed phase silica gel C18 (1 L). The column was eluted stepwise using a mixture of methanol-0.1% aqueous trifluoroacetic acid (2:8, 4:6, 5:5, 6:4, 7:3, and 8:2). The Aerothricins 1, 2 and 3 eluted in this order with methanol-0.1% aqueous trifluoroacetic acid (7:3) were concentrated to dryness *in vacuo* to obtain white powdery Aerothricin 3 trifluoroacetic acid salt (731 mg) and Aerothricin 1 trifluoroacetic acid salt (747 mg), respectively. The fractions containing Aerothricin 2 was concentrated under reduced pressure and further purified by HPLC under the following conditions: column: Capcell Pak C18 (i.d. 30 x 250 mm, Shiseido Co., LTD.); mobile phase: acetonitrile-0.1% aqueous trifluoroacetic acid (45:55); flow rate: 40 ml/min.; detection: UV 220 nm. The appropriate eluates obtained under the above conditions were concentrated to dryness *in vacuo* to obtain white powdery Aerothricin 2 trifluoroacetic acid salt (42 mg).
b) Flask fermentation
   A 2 ml portion of the frozen culture of Deuteromycotina NR 7379 (FERM BP-6391) in 10% (v/v) glycerol solution was defrosted and inoculated into a 500-ml Erlenmeyer flask containing 100 ml of a medium consisting of 1% glucose, 1% oat flour, 4% tomato paste, 0.5% corn steep liquor (Ando kasei), 0.001% FeSO₄·7H₂O, 0.001% MnSO₄·4H₂O, 0.0001% CaCl₂, 0.0002% ZnSO₄·7H₂O, 0.00002% (NH₄)6MoO₂·4H₂O, and 0.00006% H₃BO₃. The pH of the medium was adjusted to 6.8 before sterilization. The seed culture was incubated on a rotary shaker at 27°C for 3 days at 220 rpm. 2 ml of the first seed culture was transferred into 500-ml Erlenmeyer flasks containing 100 ml of the same medium and incubated on a rotary shaker under the same conditions for 3 days. 2 ml of the second seed culture was inoculated into 500-ml Erlenmeyer flasks containing 100 ml of the medium consisting of 8.5% glycerol, 1% pectin from citrus, 0.4% peanuts powder, 0.4% casein from milk vitamin-free, 0.4% tomato paste, 0.4% corn steep liquor (Ando kasei), 0.2% glycine, and 0.2% KH₂PO₄· The pH of the medium was adjusted to 7.0 before sterilization. The fermentation was conducted at 27°C with agitation of 220 rpm. After 10 days cultivation, the production reached maximum and the whole culture was subjected to the isolation procedure of Aerothricins 1, 2 and 3.
c) Jar fermentation
   A 2 ml portion of the frozen culture of Deuteromycotina NR 7379 (PERM BP-6391) in 10% (v/v) glycerol solution was defrosted and inoculated into a 500-ml Erlenmeyer flask containing 100 ml of the same seed medium as described above. The flask was shaken at 220 rpm for 3 days at 27°C. 2 ml of the first seed culture was transferred into 500-ml Erlenmeyer flasks containing 100 ml of the same seed medium and incubated on a rotary shaker under the same conditions for 3 days. Six hundred ml of the second seed culture was inoculated into 50-liter jar fermentor containing 30 liters of the same production medium as described above and 0.4% disfoam (Nissan Disfoam CA-123). The fermentation was carried out at 27°C, with aeration of 30 liters/min. and agitation of 400 rpm. The production reached maximum at around 168 h of fermentation and the whole culture was subjected to the isolation procedure of Aerothricins 1, 2 and 3.

### Aerothricin 1

1) Appearance:
white solid

2) Molecular weight (FAB-MS method):
m/z 1547 (M+H)⁺

3) Molecular formula:

C₇₂H₁₁₈N₁₄O₂₃

4) High resolution mass spectroscopy (for M+H)⁺:
Found: 1547.8568
Calculated for C₇₂H₁₁₉N₁₄O₂₃: 1547.8572

5) UV spectrum (Fig. I): in methanol:
λ(ε)max (in MeOH): 225±5 (10600 sh), 270±5 (2000), 278±5 (2100)
λ(ε)max (in N/10 NaOH-MeOH): 240±5 (7700), 268±5 (1800), 298±5 (1800)

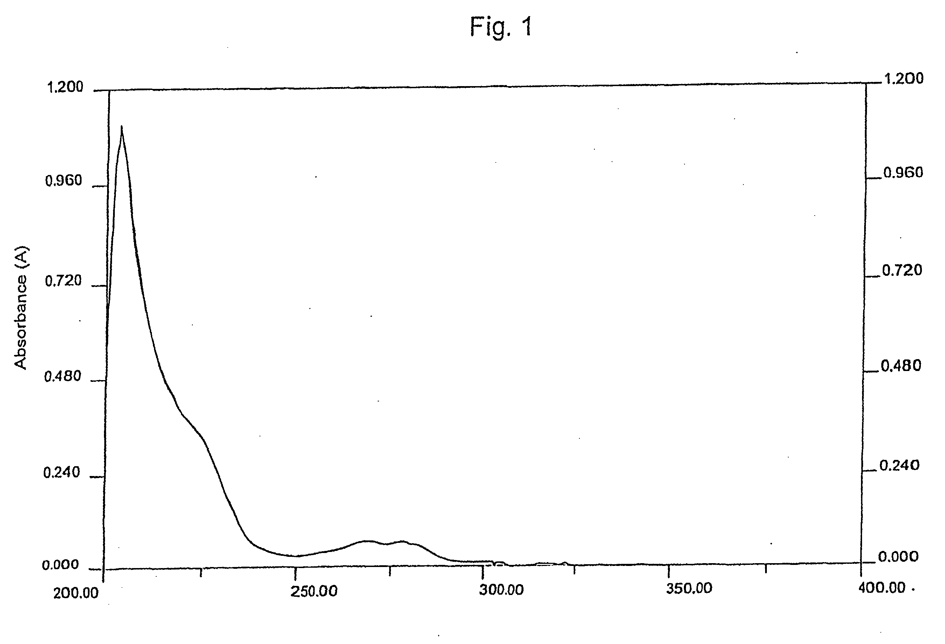

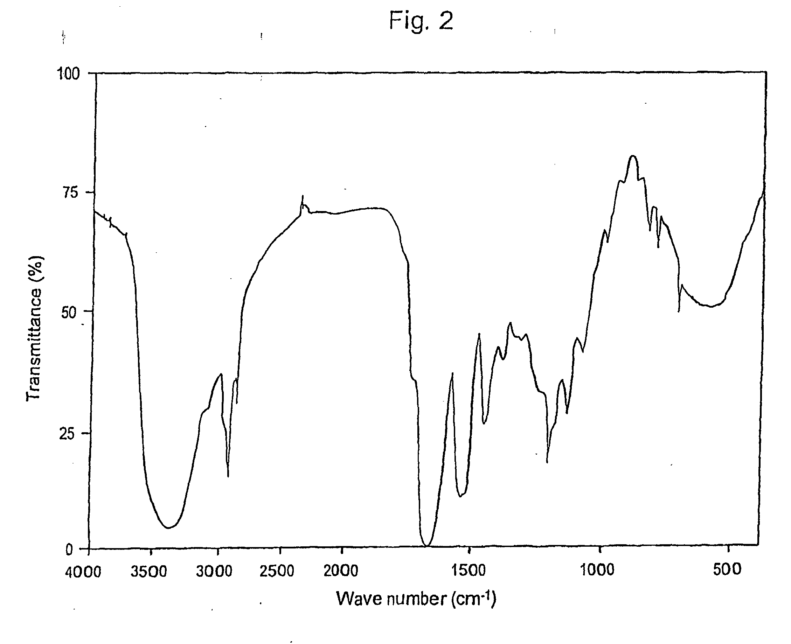

6) IR spectrum (KBr) (Fig. 2):
Main absorption wave numbers (cm⁻¹) are as follows:
   3379, 2927, 2855, 1740, 1660, 1535, 1453, 1203, 1139, 837

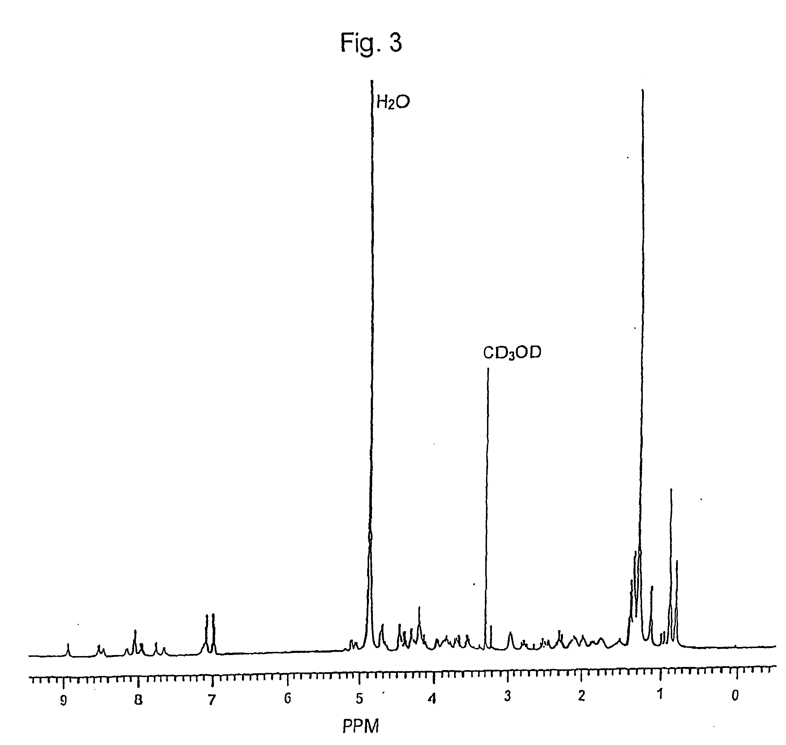

7) ¹H-NMR spectrum (Fig. 3):
400 MHz, in CD₃OD

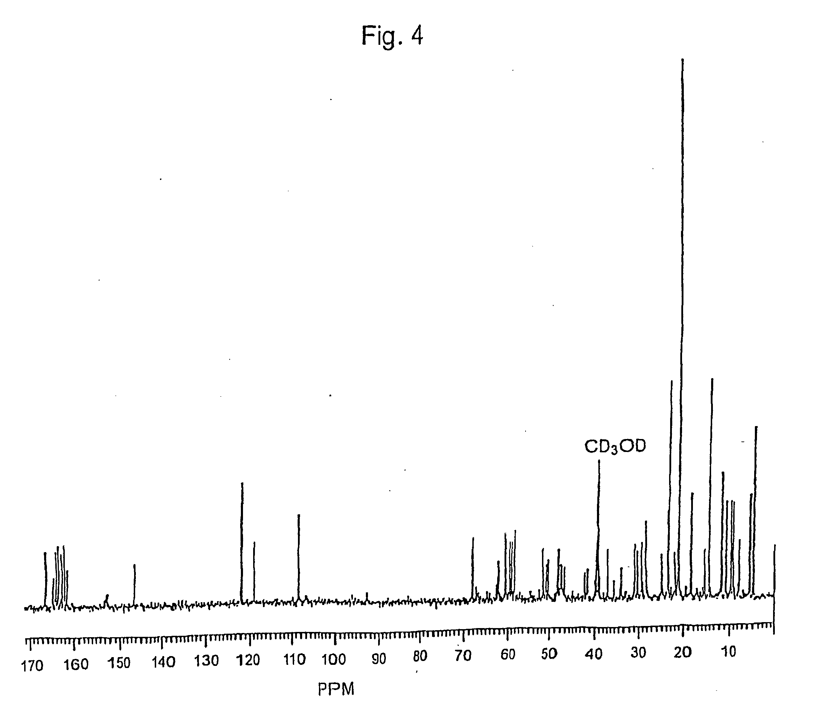

8) ¹³C-NMR spectrum (Fig. 4):
100 MHz, in CD₃OD

9) Solubility:
Soluble: water, methanol, dimethylsulfoxide

10) Color reaction:

| | |
|---|---|
| Positive | ninhydrin, anisaldehyde-sulfuric acid, iodine vapor, vanillin-sulfuric acid, Rydon-Smith reagent, molybdophosphoric acid |
| Negative | Sakaguchi reagent, Bromocresol green, 2,4-dinitrophenylhydrazine-sulfuric acid |

11) Thin-layer chromatography (TLC):

| Carrier | Solvent | Rf |
|---|---|---|
| silica gel F254^{*1} | *n*-BuOH: acetone:AcOH:H₂O (4:5:1:1) | 0.74 |
| | MeOH: H₂O (95:5) | 0.12 |

| | | |
|---|---|---|
| ^{*1} E. Merck AG., Germany | | |

12) High Performance Liquid Chromatography:
Carrier: Capcell Pak C18 gel S120A, 4.6x250mm (manufactured by Shiseido, Co., LTD.)
Mobile phase: Acetonitrile : 0.05% aqueous trifluoroacetic acid = 1:1
Flow rate: 1ml/min.
Rt = 12.1±0.5

13) Amino acid analysis:
Aerothricin 1 was heated at 120°C in 6N HCl for 24 h, followed by subjecting to amino acid analysis to detect threonine, 3 units of allo-threonine, glycine, alanine, valine, tyrosine, ornithine, 3-hydroxyproline, 4-hydroxyproline, 3-hydroxyglutamine.

### Aerothricin 2

1) Appearance:
white solid

2) Molecular weight (FAB-MS method):
m/z 1549 (M+H)⁺

3) Molecular formula:

C₇₁H₁₁₆N₁₄O₂₄

4) High resolution mass spectroscopy (for M+H)⁺:
Found: 1549.8384
Calculated for C₇₁H₁₁₇N₁₄O₂₄: 1549.8365

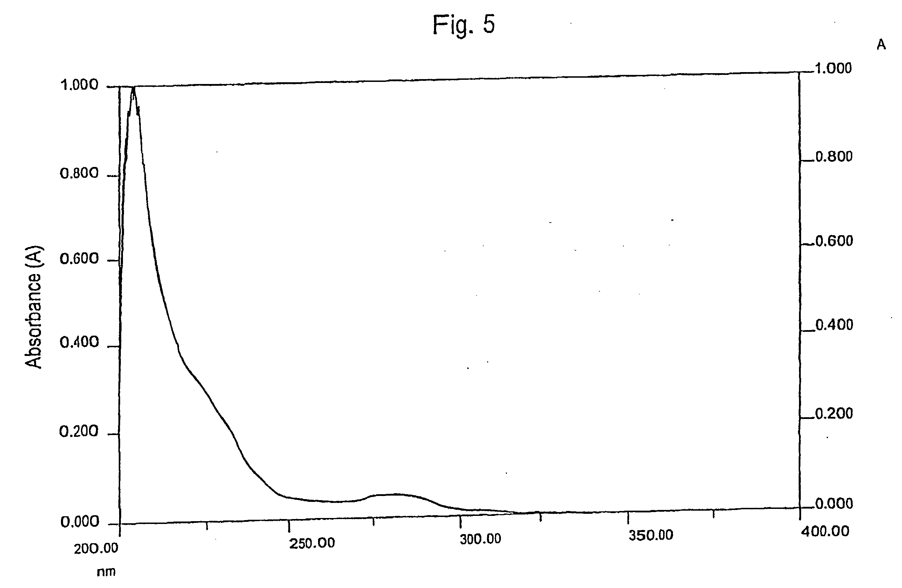

5) UV spectrum (Fig. 5): in methanol:
λ(ε)max (in MeOH): 225±5 (10200 sh), 275±5 (1900), 278±5 (2000)
λ(ε)max (in N/10 NaOH-MeOH): 240±5 (7700), 293±5 (2000)

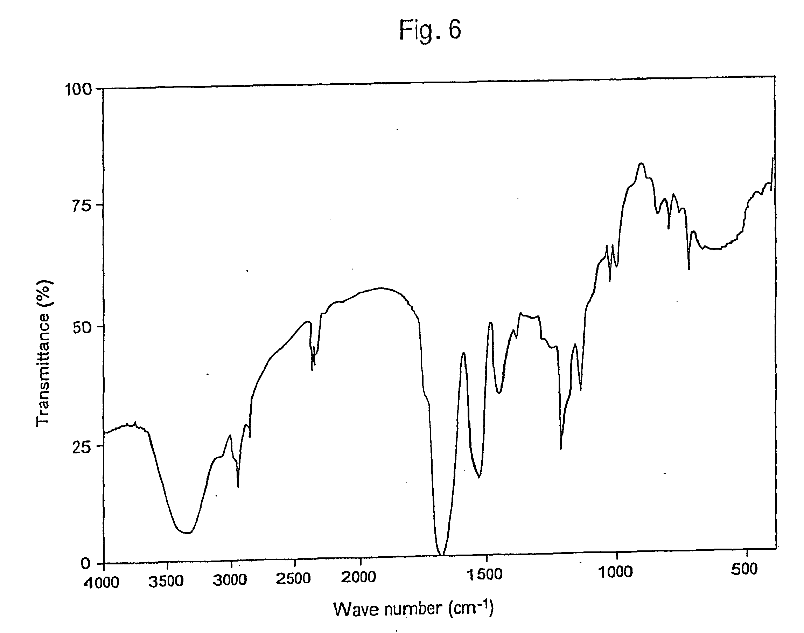

6) IR spectrum (KBr) (Fig. 6):
Main absorption wave numbers (cm⁻¹) are as follows:
   3323, 2928, 2856, 1740, 1670, 1531, 1450, 1203, 1137, 837

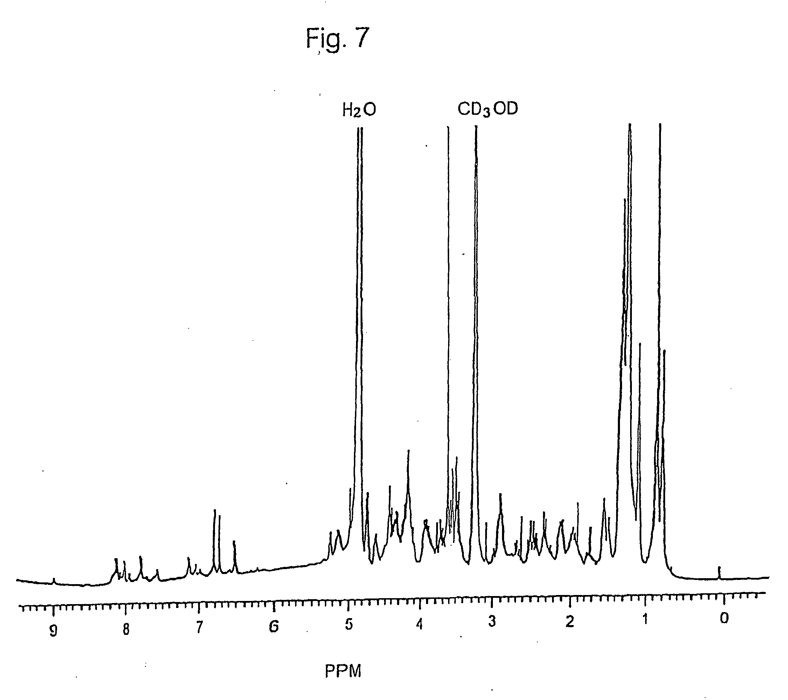

7) ¹H-NMR spectrum (Fig. 7):
400 MHz, in CD₃OD

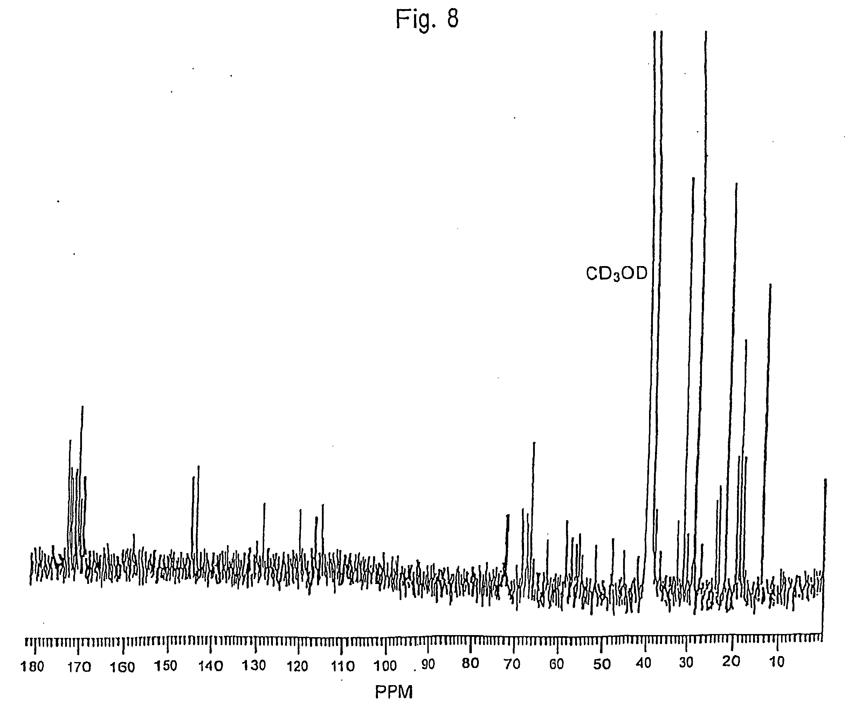

8) ¹³C-NMR spectrum (Fig. 8):
100 MHz, in CD₃OD

9) Solubility:
Soluble: water, methanol, dimethylsulfoxide

10) Color reaction:

| | |
|---|---|
| Positive | ninhydrin, anisaldehyde-sulfuric acid, Iodine vapor, vanillin-sulfuric acid, Rydon-Smith reagent, molybdophosphoric acid |
| Negative | Sakaguchi reagent, bromocresol green, 2,4-dinitrophenylhydrazine-sulfuric acid |

11) Thin-layer chromatography (TLC):

| Carrier | Solvent | Rf |
|---|---|---|
| Silica gel F254*¹ | *n*-BuOH: acetone:AcOH:H₂O (4:5:1:1) | 0.29 |
| | MeOH: H₂O (95:5) | 0.15 |

| | | |
|---|---|---|
| *¹ E. Merck AG., Germany | | |

12) High Performance Liquid Chromatography:
Carrier: Capcell Pak C18 gel S120A, 4.6x250mm (manufactured by Shiseido, Co., LTD.)
Mobile phase: Acetonitrile: 0.05% aqueous trifluoroacetic acid = 1:1
Flow rate: 1ml/min.
Rt = 9.9±0.5

13) Amino acid analysis:
Aerothricin 2 was heated at 120°C in 6N HCl for 24 h, followed by subjecting to amino acid analysis to detect threonine, 3 units of allo-threonine, glycine, alanine, valine, 3-hydroxytyrosml (DOPA), ornithine, 3-hydroxyproline, 4-hydroxyproline, 3-hydroxyglutamine.

### Aerothricin 3

1) Appearance:
white solid

2) Molecular weight (FAB-MS method):
m/z 1533 (M+H)⁺

3) Molecular formula:

C₇₁H₁₁₆N₁₄O₂₃

4) UV spectrum: in methanol
λ(ε)max (in MeOH): 225±5 (11000 sh), 275±5 (2000), 280±5 (1900)
λ(ε)max (in N/10 NaOH-MeOH): 243±5 (7800), 295±5 (1800)

5) IR spectrum (KBr):
Main absorption wave numbers (cm⁻¹) are as follows:
   3334, 2928, 2852, 1742, 1662, 1520, 1449, 1202, 1136, 836

6) Solubility:
Soluble: water, methanol, dimethylsulfoxide

7) Color reaction:

| | |
|---|---|
| Positive | ninhydrin, anisaldehyde-sulfuric acid, Iodine vapor, vanillin-sulfuric acid, Rydon-Smith reagent, molybdophosphoric acid |
| Negative | Sakaguchi reagent, bromocresol green, 2,4-dinitrophenylhydrazine-sulfuric acid |

8) Thin-layer chromatography (TLC):

| Carrier | Solvent | Rf |
|---|---|---|
| silica gel F254^{*1} | *n*-BuOH: acetone:AcOH:H₂O (4:5:1:1) | 0.26 |
| | MeOH: H₂O (95:5) | 0.09 |

| | | |
|---|---|---|
| ^{*1} E. Merck AG., Germany | | |

9) High Performance Liquid Chromatography:
Carrier: Capcell Pak C18 gel S120A, 4.6x250mm (manufactured by Shiseido, Co., LTD.)
Mobile phase: Acetonitrile : 0.05% aqueous trifluoroacetic acid = 1:1
Flow rate: 1ml/min.
Rt = 9.1±0.5

10) Amino acid analysis:
Aerothricin 3 was heated at 120°C in 6N HCl for 24 h, followed by subjecting to amino acid analysis to detect threonine, 3 units of allo-threonine, glycine, alanine, valine, tyrosine, ornithine, 3-hydroxyproline, 4-hydroxyproline, 3-hydroxyglutamine.

### Example 5

### Preparation of compound (IX)

1) Flask fermentation
   A 2 ml portion of the frozen culture of Deuteromycotina NR 7379 (FERM BP-6391) in 10% (v/v) glycerol solution was defrosted and inoculated into a 500-ml Erlenmeyer flask containing 100 ml of a medium consisting of 1% glucose, 1% oat flour, 4% tomato paste, 0.5% corn steep liquor (Ando kasei), 0.001% FeSO₄·7H₂O, 0.001% MnSO₄·4H₂O, 0.0001% CaCl₂, 0.0002% ZnSO₄·7H₂O, 0.00002% (NH₄)₆MoO₂·4H₂O, and 0.00006% H₃BO₃. The pH of the medium was adjusted to 6.8 before sterilization. The seed culture was incubated on a rotary shaker at 27°C for 4 days at 220 rpm. 2 ml of the seed culture was inoculated into 500-ml Erlenmeyer flasks containing 100 ml of the medium consisting of 8.5% glycerol, 1% pectin from citrus, 2% peanuts powder, 0.4% casein from milk vitamin-free, 0.4% tomato paste, 0.4% glycine, and 0.2% KH₂PO₄. The pH of the medium was adjusted to 7.0 before sterilization. The fermentation was conducted at 27°C with agitation of 220 rpm. After 14 days cultivation, the production reached maximum and the whole culture was subjected to the isolation work.
   The cultured whole broth (1.9 L) obtained was added n-butanol (2 L) and the mixture was stirred. The extracts thus obtained were concentrated to dryness under reduced pressure. And the residue was added hexane (500 ml) and methanol (500 ml) and the mixture thus obtained was stirred, followed by removal of the hexane layer. After removal of the methanol under reduced pressure, the residue thus obtained was washed with a mixture of hexane and ethyl acetate (1:1; 200 ml, twice), and dried under reduced pressure.
   The residue (3. 9 g) was added water (20 ml) and the mixture was stirred, followed by centrifugation to obtain the water solution. The solution thus obtained were then subjected to a column chromatography on reversed phase silica gel C18 (200 L). The column was first eluted with 0.1% aqueous trifluoroacetic acid and then eluted stepwise using a mixture of methanol-0.1% aqueous trifluoroacetic acid (1:9, 3:7, 5:5, 6:4, 7:3, and 8:2). The compound (IX) eluted with methanol-0.1% aqueous trifluoroacetic acid (7:3) were combined and the solution was neutralized with 1 N aqueous sodium hydroxide, followed by concentration to dryness *in vacuo*. The residue thus obtained was added water (10 ml) and *n*-butanol (10 ml) and the mixture was stirred. The extract thus obtained was concentrated under reduced pressure to obtain compound (IX) (96.9 mg) as white powder. The further purification to obtain compound (IX) for spectroscopy was achieved by HPLC under the following conditions: column: Capcell Pak C18 UG80 (i.d. 20 x 250 mm, Shiseido Co., LTD.); mobile phase: 0.05% trifluoroacetic acid/acetonitrile-0.05% trifluoroacetic acid/water (38:62); flow rate: 22.86 ml/min.; detection: UV 210 nm. The appropriate eluates obtained under the above conditions were concentrated to dryness *in vacuo* to obtain white powdery compound (IX) trifluoroacetic acid salt.
c) Jar fermentation
   A 2 ml portion of the frozen culture of Deuteromycotina NR 7379 (PERM BP-6391) in 10% (v/v) glycerol solution was defrosted and inoculated into a 500-ml Erlenmeyer flask containing 100 ml of the same seed medium as described above. The flask was shaken at 220 rpm for 4 days at 27°C. Two ml of the first seed culture was transferred into 500-ml Erlenmeyer flasks containing 100 ml of the same seed medium and incubated on a rotary shaker under the same conditions for 3 days. 600 ml of the second seed culture was inoculated into 50-liter jar fermentor containing 30 liters of the same production medium as described above and 0.4% disfoam (Nissan Disfoam CA-123). The fermentation was carried out at 27°C, with aeration of 30 liters/min. and agitation of 400 rpm. The production reached maximum at around 278 h of fermentation and the whole culture was subjected to the isolation procedure of compound (IX).

### Compound (IX)

1) Appearance:
white solid

2) Molecular weight (FAB-MS method):
m/z 1317 (M+H)⁺

3) Molecular formula:

C₅₉H₁₀₄N₁₂O₂₁

4) High resolution mass spectroscopy (for M+H)⁺:
Found: 1317.7555
Calculated for C₅₉H₁₀₅N₁₂O₂₁: 1317.7517

5) UV spectrum: in methanol:
λ(ε)max (in MeOH): End absorption

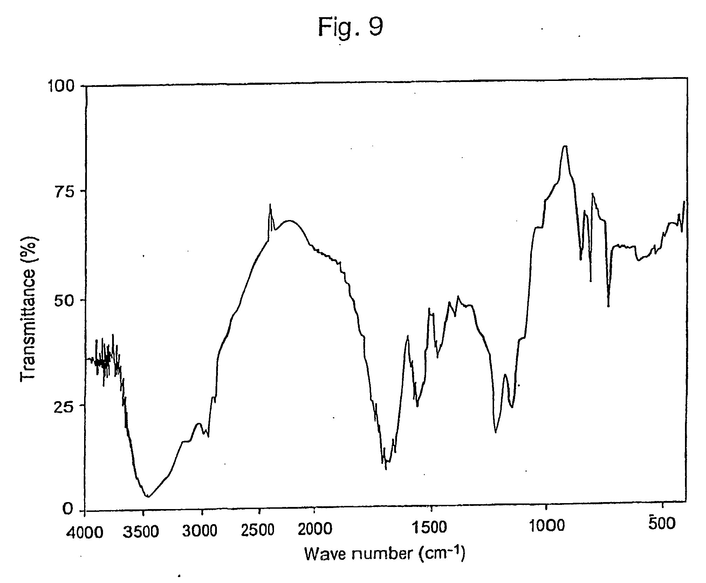

6) IR spectrum (KBr) (Fig. 9):
Main absorption wave numbers (cm⁻¹) are as follows:
   3450, 2928, 1665, 1520, 1450, 1225, 1135

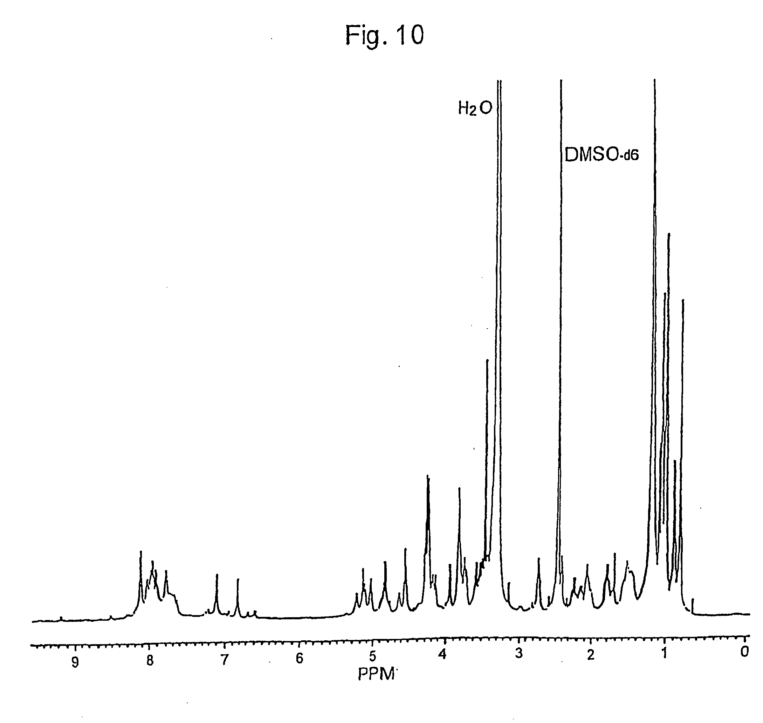

7) ¹H-NMR spectrum (Fig. 10):
500 MHz, in DMSO-d₆

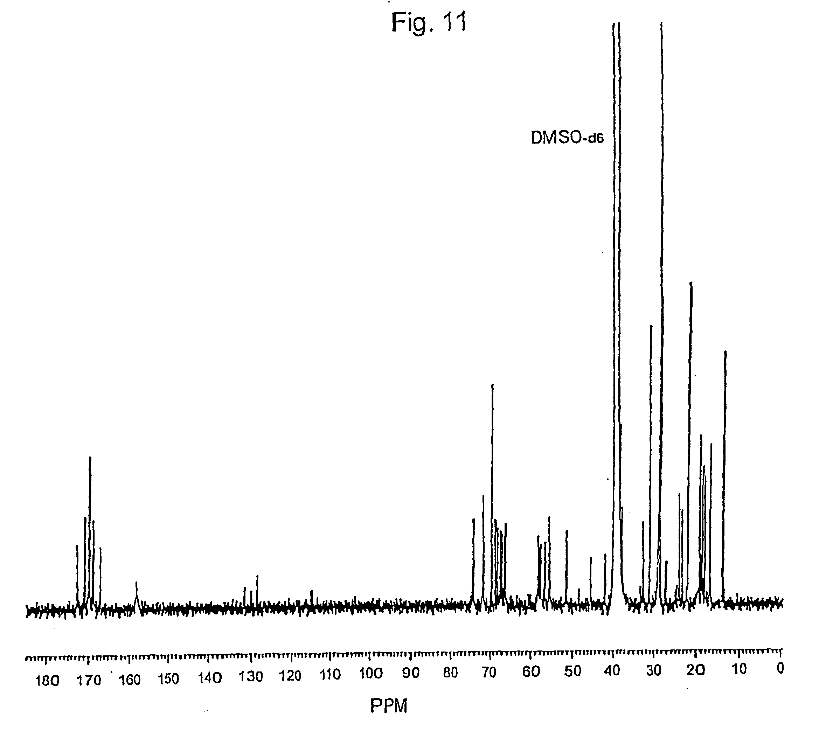

8) ¹³C-NMR spectrum (Fig. 11):
125 MHz, in DMSO-d₆

9) Solubility:
Soluble: water, methanol, dimethylsulfoxide

10) Color reaction:

| | |
|---|---|
| Positive | ninhydrin, anisaldehyde-sulfuric acid, iodine vapor, vanillin-sulfuric acid, Rydon-Smith reagent, molybdophosphoric acid |
| Negative | Sakaguchi reagent, Bromocresol green, 2,4-dinitrophenylhydrazine-sulfuric acid |

11) High Performance Liquid Chromatography:
Carrier: Capcell Pak C18 UG80A, 4.6x250mm (manufactured by Shiseido, Co., LTD.)
Mobile phase: 0.05% trifluoroacetic acid/acetonitrile : 0.05% trifluoroacetic acid/water = 38:62
Flow rate: 1 ml/min.
Rt = 7.7±0.5

### Example 6

### Preparation of N-Boc derivative (N(orn)-Boc-IX) of the ornitine residue of the compound (IX): The compound of Formula (XII: R⁶ = Boc)

To a solution of the compound (IX) obtained in the Example 5 (10.4 mg, 0.0073 mmol) in dioxane-H2O (0.43 ml-0.5 ml), were added triethylamine (3 µl) and 0.1 M solution of *tert*-butyl N-succinimidyl carbonate (0.0073µl, 0.0073 mmol) in dioxane at room temperature. After being stirred for 1.5 h, the mixture was acidified with acetic acid and was evaporated under reduced pressure. Purification of the residue by HPLC gave N(*orn*)-Boc-IX as a colorless amorphous (4.8 mg, 45% yield);

HPLC (Rt) 18.0 min. (column: Soken-ODS, 20x250 mm , flow rate: 9ml/min., eluent: H₂O : CH₃CN = gradient 1% acetic acid); FAB-MS [M+Na]⁺ 1440.

### Example 7

### Preparation of N-Boc derivative (N(val)-Boc-IX) of the valine residue of the compound (IX): The compound of Formula (X: R⁷ = Boc)

A mixture of the compound (IX) obtained in the Example 5 (15.0 mg, 0.0105 mol), di-*tert*-butyl dicarbonate (0.073M in methanol solution, 0.20 ml, 0.015 mmol) and triethylamine (7.8µl) in MeOH (3 ml) was stirred at 0°C for 24 h. The mixture was washed with *n*-hexane was evaporated under reduced pressure. Purification of the residue by reverse phase HPLC gave the (N(*val*)-Boc-IX) as a colorless amorphous (1.0 mg, 6% yield);

HPLC (Rt) 16.0 min. (column: Soken-ODS, 20x250 mm , flow rate: 9 ml/min., eluent: H₂O : CH₃CN = gradient 1% acetic acid); FAB-MS [M+H]⁺ 1418.

### Example 8

### Preparation of Aerothricin 33

To a stirred solution of (R)-3-(9-fluorenylmethoxycarbonylamino)-7-(4-pentyloxyphenyl)heptanoic acid (25.5 mg, 0.048 mmol) in DMF (0.5 ml) were added BOP reagent (21.3 mg, 0.048 mmol), HOBT hydrate (7.5 mg, 0.049 mmol) and N,N-diisopropylethylamine (0.0095 ml, 0.055 mmol). After the mixture was stirred at room temperature for 1 h, a solution of Compound B [= the linear peptide of Formula (III) wherein R² and R³ are hydrogen, R⁵ is carbamoyl group and R⁷ is *tert*-butoxycarbonyl which was prepared from Aerothricin 1 or 3 according to the procedure described in WO 96/30399] (50.7 mg, 0.036 mmol) and N,N-diisopropylethmine (0.0095 ml, 0.055 mmol) in DMF (0.6 ml) was added to the reaction mixture. After the mixture was stirred for 2.5 h at room temperature, piperidine (0.20 ml) was added, and the mixture was stirred for additional 2 h at room temperature. The solvent was evaporated *in vacuo*. The residue was purified by preparative reverse phase HPLC (column C, flow rate: 9 ml/min.; gradient: eluent: 1% AcOH-H₂O:1% AcOH-CH₃CN = 80:20 → 2:98). The appropriate fractions were combined, frozen and lyophilized to give 49.5 mg of the linear peptide C, a precursor for cyclization, as a white amorphous solid.

To a stirred solution of the linear peptide C (49.5 mg, 0.029 mmol) obtained above in DMF (27 ml) was added HOBT hydrate (11.3 mg, 0.074 mmol), N,N-diisopropyletylamine (0.018 ml, 0.105 mmol) and a solution of BOP reagent (33.1 mg, 0.075 mmol) in DMF (4 ml). After the mixture was stirred for 3 h at room temperature, the solvent was evaporated *in vacuo*.

The residue obtained above was dissolved in TFA (6 ml), and stirred at 0° C for 30 min. TFA was then evaporated *in vacuo*. The residue was purified by preparative reverse phase HPLC, the detailed condition of which is shown below. The appropriate fractions were combined, frozen and lyophilized to give 19.4 mg of Aerothricin 33 as a white amorphous solid.

HPLC(Rt): 12.4 min. (column C, flow rate: 9 ml/min.; eluent: 0.05% trifluoroacetic acid-water: 0.05% trifluoroacetic acid-acetonitrile = 61:39); FAB-MS (m/z): 1568[MH⁺].

The following Aerothricins 34-38, 40-53, 64-73 and 89-95, 97-99 and 123 were prepared according to the method similar to that descried in this Example 8 using the corresponding building block represented as Formula (IV).

| | FAB-MS | HPLC | Analytical condition |
|---|---|---|---|
| Compound name | m/z: [MH⁺] | retention time (min.) | (column) (flow rate; ratio of eluent*) |
| Aerothricin 34 | 1568 | 14.1 | (C)(9 ml/min.; 60/40) |
| Aerothricin 35 | 1568 | 13.2 | (C)(9 ml/min.; 57/43) |
| Aerothricin 36 | 1610 | 21.9 | (C)(9 ml/min.; 55/45) |
| Aerothricin 37 | 1638 | 44.1 | (C)(9 ml/min.; 54/46) |
| Aerothricin 38 | 1610 | 28.1 | (C)(9 ml/min.; 58142) |
| Aerothricin 40 | 1602 | 16.8 | (F)(10 ml/min.; 57/43) |
| Aerothricin 41 | 1616 | 20.6 | (C)(9 ml/min.; 60/40) |
| Aerothricin 42 | 1630 | 16.8 | (F)(10 ml/min.; 62/38) |
| Aerothricin 43 | 1644 | 29.2 | (C)(9 ml/min.; 57/43) |
| Aerothricin 44 | 1658 | 35.5 | (F)(10 ml/min.; 50/50) |
| Aerothricin 45 | 1630 | 24.7 | (C)(9 ml/min.; 59/41) |
| Aerothricin 46 | 1664 | 18.7 | (C)(9 ml/min.; 59/41) |
| Aerothricin 47 | 1594 | 22.9 | (C)(9 ml/min.; 54/46) |
| Aerothricin 48 | 1576 | 24.4 | (F)(10 ml/min.; 58/42) |
| Aerothricin 49 | 1590 | 24.2 | (C)(9 ml/min.; 65/35) |
| Aerothricin 50 | 1604 | 48.9 | (F)(10 ml/min.; 55/45) |
| Aerothricin 51 | 1618 | 40.4 | (F)(9 ml/min.; 60/40) |
| Aerothricin 52 | 1632 | 32.5 | (F)(10 ml/min.; 50/50) |
| Aerothricin 53 | 1646 | 27.0 | (C)(9 ml/min.; 54/46) |
| Aerothricin 64 | 1547 | 15.5 | (B)(4 ml/min.; 65/35) |
| Aerothricin 65 | 1575 | 15.5 | (C)(9 ml/min.; 55/45) |
| Aerothricin 66 | 1603 | 16.6 | (C)(9 ml/min.; 52/48) |
| Aerothricin 67 | 1587 | 19.9 | (C)(9 ml/min.; 59/41) |
| Aerothricin 68 | 1587 | 19.6 | (C)(9 ml/min.; 59/41) |
| Aerothricin 69 | 1589 | 21.8 | (C)(9 ml/min.; 58/42) |
| Aerothricin 70 | 1617 | 21.6 | (C)(9 ml/min.; 53/47) |
| Aerothricin 71 | 1746 | 30.0 | (C)(9 ml/min.; 64/36) |
| Aerothricin 72 | 1673 | 22.6 | (C)(9 ml/min.; 57/43) |
| Aerothricin 73 | 1721 | 20.2 | (C)(9 ml/min.; 55/45) |
| Aerothricin 89 | 1630 | 22.1 | (F)(10 ml/min.; 55/45) |
| Aerothricin 90 | 1658 | 24.9 | (F)(10 ml/min.; 50/50) |
| Aerothricin 91 | 1670 | 26.7 | (F)(10 ml/min.; 50/50) |
| Aerothricin 92 | 1642 | 26.0 | (F)(10 ml/min.; 55/45) |
| Aerothricin 93 | 1650 | 21.4 | (F)(10 ml/min.; 57/43) |
| Aerothricin 94 | 1658 | 30.8 | (F)(10 ml/min.; 52/48) |
| Aerothricin 95 | 1574 | 28.3 | (C)(9 ml/min.; 57/43) |
| Aerothricin 97 | 1740 | 44.7 | (F)(10 ml/min.; 57/43) |
| Aerothricin 98 | 1656 | 30.0 | (F)(10 ml/min.; 62/38) |
| Aerothricin 99 | 1644 | 16.9 | (F)(10 ml/min.;53/47) |
| Aerothricin 123 | 1630 | 20.7 | (F)(10 ml/min.;56/44) |

| | | | |
|---|---|---|---|
| * ratio of 0.05% trifluoroacetic acid-water : 0.05% trifluoroacetic acid-acetonitrile | | | |

### Example 9

### Preparation of Aerothricin 16

(a). To a stirred solution of Compound A (described in Reference Example 3) (1 g, 0.61 mmol) in pyridine (2.5 ml) was added tetranitromethane (0.365 ml, 3.05 mmol). After being stirred for 4 h at room temperature, the reaction mixture was concentrated *in vacuo*. The dark-brown residue was purified by reverse phase HPLC (Lobar RP18, 10 ml/min., 0.05% trifluoroacetic acid-water: 0.05% trifluoroacetic acid-acetonitrile = 50:50 → 33:66 0.05% TFA). The appropriate fractions were combined, frozen and lyophilized to give 711 mg of the crude nitro derivative of Compound A as a pale yellow amorphous solid.
(b). A mixture of the crude product obtained above (12 mg, 0.0071 mmol) and TFA (0.5 ml) was stirred at 0°C for 30 min. TFA was evaporated under a stream of dry nitrogen. The yellow residue was purified by preparative reverse phase HPLC. The appropriate fractions were combined, frozen and lyophilized to give 8 mg of Aerothricin 16-TFA salt as a pale yellow amorphous solid.
   HPLC(Rt): 15.5 min. (column B, flow rate: 4 ml/min., eluent: 0.05% trifluoroacetic acid-water: 0.05% trifluoroacetic acid-acetonitrile = 55:45); FAB-MS (m/z): 1578[MH⁺].

The following Aerothricins 39, 54, 55 and 77 were prepared according to the method similar to that described in Example 9, using Aerothricins obtained in Example 8 as the starting material.

| | FAB-MS | HPLC | Analytical condition |
|---|---|---|---|
| Compound name | m/z: [MH⁺] | retention time (min.) | (column) (flow rate; ratio of eluent*) |
| Aerothricin 39 | 1577 | 13.2 | (C)(9 ml/min.; 55/45) |
| Aerothricin 54 | 1661 | 14.2 | (C)(9 ml/min.; 57/43) |
| Aerothricin 55 | 1689 | 27.8 | (C)(9 ml/min.; 55/45) |
| Aerothricin 77 | 1648 | 25.0 | (C)(9 ml/min.; 53/47) |

| | | | |
|---|---|---|---|
| * ratio of 0.05% trifluoroacetic acid-water : 0.05% trifluoroacetic acid-acetonitrile | | | |

### Example 10

### Preparation of Aerothricin 17

(a). To the solution of the crude nitro derivative of Compound A, obtained in Example 9(a), (55 mg, 0.033 mmol) in MeOH (5 ml) was added 10% palladium on charcoal (20 mg), and the reaction vessel was filled with hydrogen. After being stirred for 13.5 h at room temperature, the mixture was filtered through membrane filter (pore size: 0.2 µm) and the solvent was evaporated to give 52 mg of the crude amino derivative of Aerothricin 3 as brown amorphous, which was used in the next step without further purification.
(b). A mixture of the crude amino derivative of Compound A (described in Reference Example 3), obtained above, (3.4 mg, 0.0021 mmol) and TFA (0.2 ml) was stirred at 0°C for 30 min. TFA was evaporated under a stream of dry nitrogen. The brown residue was purified by preparative reverse phase HPLC. The appropriate fractions were combined, frozen and lyophilized to give 1.3 mg of Aerothricin 17 as a colorless amorphous solid.
   HPLC(Rt): 12.8 min. (column A, flow rate: 1 min./ml, eluent: 0.05% trifluoroacetic acid-water: 0.05% trifluoroacetic acid-acetonitrile = 59:41); FAB-MS (m/z): 1548[MH⁺].

The following Aerothricins 29, 56 and 78 were prepared according to the method similar to that described in Example 10, using Aerothricins obtained in Example 9 as the starting material.

| | FAB-MS | HPLC | Analytical condition |
|---|---|---|---|
| Compound name | m/z: [MH⁺] | retention time (min.) | (column) (flow rate; ratio of eluent*) |
| Aerothricin 29 | 1606 | 31.0 | (C)(9 ml/min.; 60/40) |
| Aerothricin 56 | 1659 | 15.1 | (C)(9 ml/min.; 57/43) |
| Aerothricin 78 | 1618 | 16.8 | (C)(9 ml/min.; 57/43) |

| | | | |
|---|---|---|---|
| * ratio of 0.05% trifluoroacetic acid-water : 0.05% trifluoroacetic acid-acetonitrile | | | |

### Example 11

### Preparation of Aerothricin 18

(a). To a solution of the crude amino derivative of Compound A, obtained in Example 10(a), (1.7 mg, 0.001 mmol) in methanol (0.05 ml) and pyridine (0.025 ml) was added Boc-Gly-OH (18 mg, 0.10 mmol), WSCI (30 mg, 0.15 mmol) and HOBT hydrate (24 mg, 0.15 mmol) successively. After the mixture was stirred for 15 h at room temperature, the solvent was removed by a stream of dry nitrogen.
(b). The crude residue obtained above was dissolved in TFA (0.1 ml) and stirred at 0°C for 30 min. TFA was removed with a stream of dry nitrogen. The residue was purified by preparative reverse phase HPLC. The appropriate fractions were combined, frozen and lyophilized to give 0.54 mg of Aerothricin 18 as a colorless amorphous solid.
   HPLC(Rt): 8.9 min. (column B, flow rate: 4 ml/min., eluent: 0.05% trifluoroacetic acid-water: 0.05% trifluoroacetic acid-acetonitrile = 57:43); FAB-MS (m/z): 1605[MH⁺].

The following Aerothricins 19-23, 30, 57-62, 79, and 81 were prepared according to the method similar to that described in Example 11 using the corresponding acylating agent and Aerothricins obtained in Example 10 as the starting material.

| | FAB-MS | HPLC | Analytical condition |
|---|---|---|---|
| Compound name | m/z: [MH⁺] | retention time (min.) | (column) (flow rate; ratio of eluent*) |
| Aerothricin 19 | 1590 | 17.5 | (A)(1 ml/min.; 57/43) |
| Aerothricin 20 | 1619 | 6.0 | (B)(4 ml/min.; 55/45) |
| Aerothricin 21 | 1663 | 18.0 | (C)(9 ml/min.; 60/40) |
| Aerothricin 22 | 1605 | 12.5 | (A)(1 ml/min.; 55/45) |
| Aerothricin 23 | 1620 | 23.9 | (C)(9 ml/min.; 55/45) |
| Aerothricin 30 | 1676 | 24.6 | (C)(9 ml/min.; 61/39) |
| Aerothricin 57 | 1701 | 21.2 | (C)(9 ml/mm.; 56/44) |
| Aerothricin 58 | 1730 | 23.4 | (C)(9 ml/min.; 55/45) |
| Aerothricin 59 | 1716 | 13.7 | (C)(9 ml/min.; 58/42) |
| Aerothricin 60 | 1730 | 16.3 | (C)(9 ml/min.; 55/45) |
| Aerothricin 61 | 1730 | 39.1 | (C)(9 ml/min.; 47/53) |
| Aerothricin 62 | 1730 | 15.8 | (C)(9 ml/min.; 55/45) |
| Aerothricin 79 | 1689 | 36.1 | (C)(9 ml/min.; 57/43) |
| Aerothricin 81 | 1675 | 24.4 | (C)(9 ml/min.; 60/40) |

| | | | |
|---|---|---|---|
| * ratio of 0.05% trifluoroacetic acid-water : 0.05% trifluoroacetic acid-acetonitrile | | | |

### Example 12

### Preparation of Aerothricin 12

To a solution of Aerothricin 5 (7.5 mg, 0.0048 mmol), 37% formalin (150 µl) and acetic acid (50 µl) in MeOH (1.0 ml) was added sodium cyanoborohydride (7.5 mg, 0.119 mmol) in MeOH (100 µl) at room temperature and the mixture was stirred for 7 h at room temperature. After the solvent was evaporated *in vacuo*, the residue was dissolved in *n*-butanol and washed with diluted hydrochloric acid and water successively. The organic layer was evaporated *in vacuo*. The residue was purified by preparative reverse phase HPLC, the detailed condition of which is shown below. The appropriate fractions were combined, frozen and lyophilized to give 5.4 mg of Aerothricin 12 as a colorless amorphous solid.

HPLC(Rt): 7.1 min. (column B, flow rate: 4 ml/min., eluent: 0.05% trifluoroacetic acid-water: 0.05% trifluoroacetic acid-acetonitrile = 50:50); FAB-MS (m/z): 1575[MH⁺].

The following Aerothricins 13, 25, 30 and 75 were prepared according to the method similar to that described in Example 12.

| | FAB-MS | HPLC | Analytical condition |
|---|---|---|---|
| Compound name | m/z: [MH⁺] | retention time (min.) | (column) (flow rate; ratio of eluent*) |
| Aerothricin 13 | 1561 | 13.7 | (B)(4 ml/min.; 55/45) |
| Aerothricin 25 | 1607 | 23.5 | (C)(4 ml/min.; 55/45) |
| Aerothricin 30 | 1676 | 24.6 | (C)(9 ml/min.; 61/39) |
| Aerothricin 75 | 1631 | 24.2 | (C)(9 ml/min.; 55/45) |

| | | | |
|---|---|---|---|
| * ratio of 0.05% trifluoroacetic acid-water : 0.05% trifluoroacetic acid-acetonitrile | | | |

### Example 13

### Prepration of Aerothricin 111

(a). To a solution of Aerothricin 3 (500 mg, 0.326 mmol), (2-oxoethyl)-carbamic acid *tert*-butyl ester* (1.66g, 10.4 mmol) and acetic acid (5 ml) in MeOH (45 ml) was added sodium cyanoborohydride (410 mg, 6.52 mmol) in MeOH (5 ml) at room temperature. The mixture was stirred for 18 h at room temperature. After the solvent was evaporated *in vacuo*, the residue was dissolved in n-butanol and washed with diluted hydrochloric acid and water successively. The organic layer was evaporated *in vacuo*.
   The crude residue was used for the next step without further purification.
   *CAS No. 89711-08-0
(b). A solution of the crude residue obtained above in TFA (20 ml) was stirred at 0°C for 30 min. TFA was evaporated *in vacuo*. The residue was purified by preparative reverse HPLC, the detailed condition of which is shown below. The appropriate fraction were combined, frozen and lyophilized to give 253 mg of Aerothricin 111 as a colorless amorphous solid.
   HPLC(Rt) 18.6 min. (column F, flow rate: 10ml/min., eluent: 0.05% trifluoroacetic acid-water : 0.05% trifluoroacetic acid-acetonitrile = 57:43); FAB-MS (m/z): 1619
   [M+H]⁺.

The following Aerothricins 100, 112, 114 and 115 were prepared according to the method similar to that described in Example 13.

| | FAB-MS | HPLC | Analytical condition |
|---|---|---|---|
| Compound name | m/z: [MH⁺] | retention time (min.) | (column) (flow rate; ratio of eluent*) |
| Aerothricin 100 | 1730 | 14.8 | (F)(10 ml/min.; 56/44) |
| Aerothricin 112 | 1647 | 11.8 | (F)(10 ml/min.; 57/43) |
| Aerothricin 114 | 1759 | 23.1 | (C)(10 ml/min.; 60/40) |
| Aerothricin 115 | 1633 | 19.6 | (F)(10 ml/min.; 59/41) |

| | | | |
|---|---|---|---|
| * ratio of 0.05% trifluoroacetic acid-water : 0.05% trifluoroacetic acid-acetonitrile | | | |

### Example 14

### Preparation of Aerothricin 120

To a mixture of Aerothricin 3 (500 mg, 0.326 mmol) and triethylamine (682 µl, 4.89 mmol) in MeOH (10 ml) was added acrylonitrile (214 µl, 3.27 mmol) at room temperature. The mixture was stirred for 20 h at room temperature. After the solvent was evaporated *in vacuo*, the residue was dissolved in *n*-butanol and washed with diluted hydrochloric acid and water successively. The organic layer was evaporated *in vacuo*. The crude residue was purified by preparative reverse HPLC, the detailed condition of which is shown below. The appropriate fraction were combined, frozen and lyophilized to give 207 mg of Aerothricin 120 as a colorless amorphous solid.

HPLC(Rt) 27.5 min. (column F, flow rate: 10 ml/min., eluent: 0.05% trifluoroacetic acid-water: 0.05% trifluoroacetic acid-acetonitrile = 53:47); FAB-MS (m/z): 1586
[M+H]⁺.

### Example 15

### Prepration of Aerothricin 113

To a mixture of Aerothricin 120 (100 mg, 0.063 mmol) in MeOH (5 ml) was added 10% palladium on charcoal (20 mg), and the reaction vessel was filled with hydrogen. After being stirred for 20 h at room temperature, the mixture was filtered through membrane filter (pore size: 0.2 µm) and the solvent was evaporated *in vacuo*. The crude residue was purified by preparative reverse HPLC, the detailed condition of which is shown below. The appropriate fraction were combined, frozen and lyophilized to give 87.2 mg of Aerothricin 113 as a colorless amorphous solid.

HPLC(Rt) 23.0 min. (column F, flow rate: 10ml/min., mobile phase: 0.05% trifluoroacetic acid-water : 0.05% trifluoroacetic acid-acetonitrile = 57:43) ; FAB-MS (m/z): 1590 [M+H]⁺.

Aerothricin 129 was prepared according to the method similar to that described in Example 14 - 15 followed by removal Boc group of the ornitine residue with trifluoroacetic acid. The starting material, in this case, was the N^{δ}-Boc derivative of the (D)-ornitin moiety of Aerothricin 106 obtained in the process similar to Example 16.

| | FAB-MS | HPLC | Analytical condition |
|---|---|---|---|
| Compound name | m/z: [MH⁺] | retention time (min.) | (column) (flow rate; ratio of eluent*) |
| Aerothricin 129 | 1705 | 33.8 | (F)(10 ml/min.; 62/38) |

| | | | |
|---|---|---|---|
| * ratio of 0.05% trifluoroacetic acid-water : 0.05% trifluoroacetic acid-acetonitrile | | | |

### Example 16

### Preparation of Aerothricin 14

To a solution of N-Boc-Sarcosine (123 mg, 0.65 mmol), WSC-HCl (240 mg, 1.25 mmol) and DMAP (150 mg, 1.23 mmol) in CH₃CN (10 ml) was added a solution of Aerothricin 3 (100 mg, 0.065 mmol) in CH₃OH (3 ml). The mixture was stirred at room temperature for 15 h and then concentrated in vacuo. The residue was dissolved in *n*-BuOH (10 ml) and washed with H₂O (5 ml x 2, adjusted pH 3∼4 with 1 N HCl). The *n*-BuOH layer was concentrated *in vacuo* and the residue was dissolved in TFA (5 ml) at 0°C. After the solution was stirred at room temperature for 1 h, TFA was evaporated *in vacuo*. The residue was purified by preparative reverse phase HPLC to give 40.8 mg (39% yield) of Aerothricin 14 as a white amorphous powder.

HPLC(Rt): 23.1 min. (column C, flow rate: 9 ml/min., 0.05% trifluoroacetic acid-water : 0.05% trifluoroacetic acid-acetonitrile = 60:40); FAB-MS (m/z): 1605[MH⁺].

The following Aerothricins 15, 21, 26-29 and 101-107, 109, 110, 118, 130 and 131 were prepared according to the method similar to that described in Example 16 using the corresponding acid as a building block.

| | FAB-MS | HPLC | Analytical condition |
|---|---|---|---|
| Compound name | m/z: [MH⁺] | retention time (min.) | (column) (flow rate; ratio of eluent*) |
| Aerothricin 15 | 1631 | 24.0 | (C)(9 ml/min.; 57/43) |
| Aerothricin 21 | 1663 | 18.0 | (C)(9 ml/min.; 60/40) |
| Aerothricin 26 | 1650 | 19.9 | (C)(9 ml/min.; 50/50) |
| Aerothricin 27 | 1676 | 22.5 | (C)(9 ml/min.; 55/45) |
| Aerothricin 28 | 1636 | 20.9 | (C)(9 ml/min.; 50/50) |
| Aerothricin 29 | 1606 | 31.0 | (C)(9 ml/min.; 60/40) |
| Aerothricin 101 | 1647 | 16.5 | (F)(10 ml/min.; 56/44) |
| Aerothricin 102 | 1661 | 16.3 | (F)(10 ml/min.; 56/44) |
| Aerothricin 103 | 1689 | 13.4 | (F)(10 ml/min.; 54/46) |
| Aerothricin 104 | 1633 | 22.6 | (F)(10 ml/min.; 58/42) |
| Aerothricin 105 | 1619 | 29.2 | (F)(10 ml/min.; 52/38) |
| Aerothricin 106 | 1647 | 17.3 | (F)(10 ml/min.; 56/44) |
| Aerothricin 107 | 1661 | 36.5 | (F)(10 ml/min.; 60/40) |
| Aerothricin 109 | 1633 | 26.1 | (F)(10 ml/min.; 58/42) |
| Aerothricin 110 | 1619 | 28.8 | (F)(9 ml/min.; 58/42) |
| Aerothricin 118 | 1685 | 15.2 | (F)(10 ml/min.; 51/49) |
| Aerothricin 130 | 1847 | 16.0 | (F)(10 ml/min.; 63/37) |
| Aerothricin 131 | 1818 | 21.1 | (F)(10 ml/min.; 63/37) |

| | | | |
|---|---|---|---|
| * ratio of 0.05% trifluoroacetic acid-water : 0.05% trifluoroacetic acid-acetonitrile | | | |

### Example 17

### Preparation of Aerothricin 74

A mixture of Aerothricin 66 (20 mg, 0.012 mmol), 3,5-dimethylpyrazole-1-carboxamidine nitrate (13 mg, 0.064 mmol) and triethylamine (18 ml, 0.13 mmol) in MeOH (1 ml) was stirred at room temperature for 15 h. After solvent was evaporated, the crude residue was purified by preparative reverse phase HPLC, the detailed condition of which is shown below. The appropriate fractions were combined, frozen and lyophilized to give 10.2 mg of Aerothricin 74 as a colorless amorphous solid.

HPLC(Rt) 21.2 min. (column C, How rate: 9 ml/min., eluent 0.05% trifluoroacetic acid-water : 0.05% trifluoroacetic acid-acetonitrile = 54:46); FAB-MS (m/z): 1645[MH⁺].

The following Aerothricins 4 and 116 were prepared according to the method similar to that described in Example 17 using Aerothricin 3 and 111 as a starting material, respectively.

| | FAB-MS | HPLC | Analytical condition |
|---|---|---|---|
| Compound name | m/z: [MH⁺] | retention time (min.) | (column) (flow rate; ratio of eluent*) |
| Aerothricin 4 | 1576 | 7.6 | (D)(1 ml/min.; 50/50) |
| Aerothricin 116 | 1703 | 14.9 | (F)(10 ml/min.; 57/43) |

| | | | |
|---|---|---|---|
| * ratio of 0.05% trifluoroacetic acid-water : 0.05% trifluoroacetic acid-acetonitrile | | | |

### Example 18

### Preparation of Aerothricin 5

(a). To a solution of Compound A, obtained in Reference Example 3, (10 mg, 0.0061 mmol) and potassium carbonate (10 mg, 0.072 mmol) in DMF (1 ml) was added methyl iodide (8 µl, 0.129 mmol) at room temperature and the mixture was stirred for 43 h at room temperature. After the mixture was filtered by Celite-pad and the filtrate was evaporated *in vacuo*. The residue was dissolved in *n*-butanol and washed with diluted hydrochloric acid and water successively. The organic layer was evaporated *in vacuo*. The crude residue was used for the next step without further purification.
(b). A solution of the crude residue obtained above in TFA (1.0 ml) was stirred at 0°C for 30 min. TFA was evaporated *in vacuo*. The residue was purified by preparative reverse phase HPLC, the detailed condition of which is shown below. The appropriate fractions were combined, frozen and lyophilized to give 3.8 mg of Aerothricin 5 as a colorless amorphous solid.
   HPLC(Rt): 14.5 min. (column B, flow rate: 4 ml/min., eluent: 0.05% trifluoroacetic acid-water : 0.05% trifluoroacetic acid-acetonitrile = 55:45); FAB-MS (m/z): 1547[MH⁺].

The following Aerothricins 6-10 and 76 were prepared according to the method similar to that described in Example 18 using the corresponding alkylating agent.

| | FAB-MS | HPLC | Analytical condition |
|---|---|---|---|
| Compound name | m/z: [MH⁺] | retention time (min.) | (column) (flow rate; ratio of eluent*) |
| Aerothricin 6 | 1561 | 16.0 | (A)(1 ml/min.; 55/45) |
| Aerothricin 7 | 1573 | 8.4 | (A) (1 ml/min.; 50/50) |
| Aerothricin 8 | 1589 | 26.1 | (B)(4.7 ml/min.; 58/42). |
| Aerothricin 9 | 1591 | 38.5 | (B)(4 ml/min.; 60/40) |
| Aerothricin 10 | 1590 | 6.7 | (A)(1 ml/min.; 53/47) |
| Aerothricin 76 | 1617 | 26.0 | (C)(9 ml/min.; 53/47) |

| | | | |
|---|---|---|---|
| * ratio of 0.05% trifluoroacetic acid-water : 0.05% trifluoroacetic acid-acetonitrile | | | |

### Example 19

### Preparation of Aerothricin 24

(a). A cold mixture of Compound A, obtained in Reference Example 3, (100 mg), sodium iodide (29.5 mg, 0.197 mmol) and sodium hypochlorite solution (250 µl) in methanol (2 ml) was stirred at 0°C for 2 h. The reaction mixture was quenched with saturated aqueous sodium thiosulfate, acidified with 1 N HCl and extracted with n-butanol. The combined organic extracts were evaporated *in vacuo*. At this point, the starting material was still remained. To complete the iodination reaction, the same experimental procedure was repeated. After the same work up, the residue was purified by preparative reverse phase HPLC to give the iodino derivative of the Compound A as colorless solid (54 mg, 50% yield).
(b). A mixture of the iodido derivative of Compound A obtained above (23.8 mg), methyl acrylate (16 µl), triethylamine (40 µl) and palladium acetate (2.1 mg) in acetonitrile (250 µl) and N,N-dimethylformamide (750 µl) was heated at 70°C for 28 h. The resulting mixture was passed through C-18 short column and the residue was treated with trifluoroacetic acid (1 ml) at 0°C for 1 h. The resulting mixture was evaporated *in vacuo*. Purification of the residue by preparative reverse phase HPLC gave Aerothricin 24 as colorless solid (8.8 mg, 40% yield).
   HPLC(Rt): 86.3 min. (column F, flow rate: 9 ml/min., eluent: 0.05% trifluoroacetic acid-water: 0.05% trifluoroacetic acid-acetonitrile = 58:42); FAB-MS (m/z): 1617[MH⁺].

### Example 20

### Preparation of Aerothricin 96

A mixture of the iodido derivative of the Compound A (30 mg), obtained in Example 19(a), potassium acetate (6.9 mg) and tetrakis(triphenylphoshine)palladium (4.6 mg) in degassed dimethysulfoxide (2 ml) was heated at 60°C for 20 h under carbon monooxide atmosphere. The resulting mixture was passed through C-18 reverse phase short column and the residue was treated with trifluoroacetic acid at 0°C for 1 h. The resulting mixture was evaporated under reduced pressure. Purification of the residue by preparative reverse phase HPLC gave Aerothricin 96 as colorless solid (2.3 mg, 8% yield).

HPLC(Rt): 23.2 min. (column F, flow rate: 10 ml/min., eluent: 0.05% trifluoroacetic acid-water: 0.05% trifluoroacetic acid-acetonitrile = 52.2:47.8); FAB-MS (m/z): 1677[MH⁺].

### Example 21

### Preparation of Aerothricin 32

(a). A mixture of the Compound A, obtained in Reference Example 3, (20 mg) and (methoxycarbonylsulfamoyl)triethylammoniunn hydroxide (26.5 mg, 0.108 mmol) in acetonitrile (3 ml) was stirred at room temperature for 8 h. The reaction mixture was acidified with 1 N HCl and was evaporated *in vacuo*. The residue was extracted with *n*-butanol and the extracts were evaporated *in vacuo*.
(b). The crude product was treated with trifluoroacetic acid at 0°C for 1 h. TFA was evaporated *in vacuo*. Purification of the residue by preparative reverse phase HPLC gave Aerothricin 32 as colorless solid (2.0 mg, 10% yield).
   HPLC(Rt): 42.9 min. (column B, flow rate: 4 ml/min., eluent: 0.05% trifluoroacetic acid-water : 0.05% trifluoroacetic acid-acetonitrile = 55:45); FAB-MS (m/z): 1516[MH⁺].

### Example 22

### Preparation of Aerothricin 31

(a). To a cold solution of the Compound A, obtained in Reference Example 3, (25.7 mg) in tetrahydrofurane (5 ml) was added borane-dimethylsulfide complex (25 ml) at -10 °C. After being stirred at -10°C for 5 h, the reaction mixture was quenched with 2 N HCl and was extracted with *n*-butanol. The combined extracts were evaporated *in vacuo*.
(b). The crude product was treated with trifluoroacetic acid at 0°C for 1 h. THF was evaporated under reduced pressure. Purification of the residue by preparative reverse phase HPLC gave Aerothricin 31 as colorless solid (3.7 mg, 15% yield).
   HPLC(Rt): 25.1 min. (column B, flow rate: 4 ml/min., eluent: 0.05% trifluoroacetic acid-water: 0.05% trifluoroacetic acid-acetonitrile = 62:38); FAB-MS (m/z): 1519[MH⁺].

### Example 23

### Preparation of Aerothricin 121

To a solution of Aerothricin 3 (50 mg) in DMF(1 ml) and triethylamine (0.025 ml) was added methyl iodide (0.010 ml). After being stirred for 16 h at room temperature, to the mixture was further added triethylamine (0.025 ml) and methyl iodide (0.05 ml) and stirred for 24 h at room temperature. LCMS analysis of the mixture indicated > 90% conversion to the desired compound. The solvent was purged with a stream of nitrogen and the residue was purified by preparative reverse phase HPLC, the detailed condition of which is shown below. The appropriate fractions were combined, frozen and lyophilized to give 23 mg of Aerothricin 121, as a colorless amorphous solid.

HPLC(Rt): 20.5 min. (column B, flow rate: 4 ml/min., eluent: 0.05% trifluoroacetic acid-water : 0.05% trifluoroacetic acid-acetonitrile = 52:48); FAB-MS (m/z): 1576[M⁺].

### Example 24

### Preparation of Aerothricin 122

To a solution of Aerothricin 3 (50 mg) in pyridine(1 ml) was added sulfur trioxide N,N-dimethylformamide complex(23 mg). After being stirred for 2 h at room temperature, the solvent was purged with a stream of dry nitrogen.

A solution of the crude residue obtained above in TFA (1 ml) was stirred at 0°C for 30 min. TFA was purged with a stream of dry nitrogen and the residue was purified by preparative reverse phase HPLC, the detailed condition of which is shown below. The pure fractions were combined, frozen and lyophilized to give 5 mg of Aerothricin 122, as a colorless amorphous solid.

HPLC(Rt): 24.6 min. (column F, flow rate: 10 ml/min., eluent: 0.05% trifluoroacetic acid-water : 0.05% trifluoroacetic acid-acetonitrile = 52:48); FAB-MS (m/z): 1613[MH⁺].

### Example 25

### Preparation of Aerothricin 63

(a). To a stirred solution of N^{α}-Fmoc-N^{β}-Boc-(S)-2,3-diaminopropionic acid (343 mg, 0.80 mmol) in DMF (10 ml) were added BOP reagent (355 mg, 0.80 mmol), HOBT hydrate (124 mg, 0.81 mmol) and N,N-diisopropylethylamine (0.174 ml, 1.00 mmol). After the mixture was stirred for 1.5 h at room temperature, a solution of Aerothricin 3 (1.10 g, 0.67 mmol) and N,N-diisopropylethylamine (0.174 ml, 1.00mmol) in DMF (9.5 ml) was added to the mixture. After being stirred for additional 1 h at room temperature, the mixture was concentrated *in vacuo*.
(b). To a stirred solution of the residue obtained above in DMF (20 ml) was added piperidine-4-carboxylic acid polyamine resin (200-400 mesh),HL (1.50 mmol/g, 2.66 g), and the reaction mixture was irradiated with ultrasonic sound for 6 h. The resin was removed by filtration through a Celite pad, washed with MeOH and the combined filtrate and washing were frozen and lyophilized to give 1.08 g of the crude derivative of Aerothricin 3 as a white amorphous solid, which was used for the next step without further purification.
(c). To a stirred solution of the crude derivative of Aerothricin 3, obtained above, (25.6 mg, 0.015 mmol) in MeOH (1 ml) were added (2-oxo-ethyl)carbamic acid *tert*-butyl ester (crude, 207 mg), AcOH (0.1 ml) and NaBH₃CN (19.1 mg). After the mixture was stirred for 2 h at room temperature, the reaction mixture was concentrated *in vacuo*. The residue was diluted with *n*-BuOH (4 ml) and washed with H₂O (1 ml x 2, adjusted pH 3-4 with 0.1 N HCl). The *n*-BuOH layer was concentrated *in vacuo*. The crude residue was used for the next step without further purification.
(d). A solution of the crude residue obtained above in TFA (2 ml) was stirred at 0°C for 2 h. TFA was evaporated *in vacuo* and the residue was purified by preparative reverse phase HPLC, the detailed condition of which is shown below. The pure fractions were combined, frozen and lyophilized to give 8.8 mg of Aerothricin 63 as a white amorphous solid.
   HPLC(Rt): 24.8 min. (column F, flow rate: 9 ml/min., eluent: 0.05% trifluoroacetic acid-water : 0.05% trifluoroacetic acid-acetonitrile = 54:36); FAB-MS(m/z): 1706 [MH⁺].

### Example 26

### Preparation of Aerothricin 127

Aerothricin 127 was prepared by the same method as that described for Aerothricin 63 by use of N^{α}-Fmoc-N^{δ}-Boc-(D)-ornitin. Aerothricin 127 was obtained as a white amorphous solid.

HPLC(Rt): 23.9 min. (column F, flow rate: 9 ml/min., eluent: 0.05% trifluoroacetic acid-water: 0.05% trifluoroacetic acid-acetonitrile = 54:36); FAB-MS(m/z): 1734 [MH⁺].

### Example 27

### Preparation of Aerothricin 124

(a). To a stirred solution of Boc-D-Orn(Boc)-OH (46 mg, 0.138 mmol) in DMF (2 ml) were added BOP reagent (62 mg, 0.14 mmol), HOBT hydrate (22 mg, 0.144 mmol) and N,N-diisopropylethylamine (24 µl, 0.138 mmol ). After being stirred for 30 min. at room temperature, a solution of Aerothricin 120 (100 mg, 0.063 mmol) and N-diisopropylethylamine (24 µl, 0.138 mmol ) in DMF (2 ml) was added to the reaction mixture. After being stirred for 18 h at room temperature, the solvent was evaporated *in vacuo*.
   The residue was dissolved in TFA (4 ml), and the solution was stirred at 0°C for 30 min. After removal of TFA with a stream of dry nitrogen, the residue was purified by preparative reverse phase HPLC, the detailed condition of which is shown below. The pure fractions were combined, frozen and lyophilized to give 48.6 mg of the nitrile derivative as a white amorphous solid.
   HPLC(Rt): 20.2 min. (column F, flow rate: 10 ml/min., eluent: 0.05% trifluoroacetic acid-water : 0.05% trifluoroacetic acid-acetonitrile = 57:43); FAB-MS (m/z): 1700 [M+H]⁺.
(b). To a mixture of the nitrile derivative obtained above (48.6 mg, 0.0286 mmol) in dioxane (1 ml) and water (1 ml) was added 10% palladium on charcoal (10 mg), and the mixture was stirred under hydrogen atmosphere for 14 h at room temperature. Then the mixture was filtered through membrane filter (pore size: 0.2 µm) and the solvent was evaporated *in vacuo*. The crude residue was purified by preparative reverse phase HPLC, the detailed condition of which is shown below. The pure fractions were combined, frozen and lyophilized to give 26.5 mg of Aerothricin 124 as a colorless amorphous solid.
   HPLC(Rt): 18.2 min. (column F, flow rate: 10 ml/min., eluent: 0.05% trifluoroacetic acid-water : 0.05% trifluoroacetic acid-acetonitrile = 60:40); FAB-MS (m/z): 1704 [M+H]⁺.

The following Aerothricins 132, 134-136 were prepared according to the method similar to that described in Example 27.

| | FAB-MS | HPLC | Analytical condition |
|---|---|---|---|
| Compound name | m/z: [MH⁺] | retention time (min.) | (column) (flow rate; ratio of eluent*) |
| Aerothricin 132 | 1706 | 18.9 | (F) (10 ml/min.; 60/40) |
| Aerothricin 134 | 1790 | 25.7 | (F) (10 ml/min.; 63/37) |
| Aerothricin 135 | 1790 | 25.5 | (F) (10 ml/min.; 63/37) |
| Aerothricin 136 | 1761 | 25.8 | (F) (10 ml/min.; 63/37) |

| | | | |
|---|---|---|---|
| * ratio of 0.05% trifluoroacetic acid-water : 0.05% trifluoroacetic acid-acetonitrile | | | |

### Example 28

### Preparation of Aerothricin 125

(a). To a solution of Aerothricin 3 mono TFA salt (natural product: 50 mg) in DMF(1 ml) and triethylamine(0.126 ml) was added 2-bromo-5-nitropyridine(185 mg). After being stirred for 25 h at room temperature, the solvent was purged with a stream of dry nitrogen. The residue was purified by preparative reverse phase HPLC. The appropriate fractions were combined, frozen and lyophilized to give 25 mg of 5-nitropyrid-2-yl derivative of Aerothricin 3 as a slight yellow amorphous solid.
   HPLC(Rt): 29.9 min. (column F, flow rate: 10 ml/min., eluent: 0.05% trifluoroacetic acid-water : 0.05% trifluoroacetic acid-acetonitrile = 47:53); FAB-MS (m/z): 1655 [M+H]⁺.
(b). 5-Nitropyrid-2-yl derivative of Aerothricin 3 obtained above (10 mg) was dissolved in dioxane-H₂O (1 ml-5 ml). 5% Palladium on charcoal (20 mg) was added and the reaction vessel was filled with hydrogen. After being stirred for 3 h at room temperature, filtration through membrane filter (pore size: 0.2 µm) and evaporation of solvent gave 14 mg of crude product, which was purified by preparative reverse phase HPLC, the detailed condition of which is shown below. The pure fractions were combined, frozen and lyophilized to give 2.5 mg of Aerothricin 125 as a colorless amorphous solid.
   HPLC(Rt): 18.7 min. (column F, flow rate: 10 ml/min., eluent: 0.05% trifluoroacetic acid-water: 0.05% trifluoroacetic acid-acetonitrile = 52:48); FAB-MS (m/z): 1625 [M+H]⁺.

### Example 29-1

### Preparation of Aerothricin 128

(a). To a stirred solution of Fmoc-D-Orn(Boc)-OH (389 mg, 0.86 mmol) in DMF (10 ml) were added BOP reagent (378 mg, 0.85 mmol), HOBT hydrate (131 mg, 0.86 mmol) and N,N-diisopropylethylamine (171 µl, 0.98 mmol). After the mixture was stirred at a room temperature for 40 min., a solution of Aerothricin 3 (1.08 g, 0.66 mmol) and N,N-diisopropylethylamine (171 µl, 0.98 mmol) in DMF (10 ml) was added to the mixture. After being stirred for 2.5 h at a room temperature, piperidine (4 ml) was added, and the mixture was stirred for additional 1 h at a room temperature. The mixture was concentrated *in vacuo*. The residue was diluted with n-BuOH (50 ml) and washed with H₂O (25 ml x 2, adjusted pH 3 with 1 N HCl). The *n*-BuOH layer was concentrated *in vacuo*.
(b). To a stirred solution of Boc-D-Orn(Boc)-OH (9.6 mg, 0.029 mmol) in DMF (1ml) were added BOP reagent (13.3 mg, 0.030 mmol), HOBT hydrate (4.6 mg, 0.030 mmol) and N,N-diisopropylethylamine (4.8 µl, 0.028 mmol). After the mixture was stirred at room temperature for 30 min., a solution of the crude residue (31.9 mg) obtained above and N,N-diisopropylethylamine (4.8 µl, 0.028 mmol) in DMF (1 ml) was added to the mixture. After being stirred for 4 h at a room temperature, the reaction mixture was concentrated *in vacuo*.
(c). The crude residue obtained above was dissolved in TFA (1.5 ml) and stirred at 0°C for 1 h. The reaction mixture was concentrated *in vacuo*, and the residue was purified by preparative reverse HPLC. The appropriate fraction were combined, frozen and lyophilized to give 16.6 mg of Aerothricin 128 as a white amorphous solid:
   HPLC(Rt): 27.23 min. (column F, flow rate: 9 ml/min., eluent: 0.05% trifluoroacetic acid-water: 0.05% trifluoroacetic acid-acetonitrile = 55:35); FAB-MS (m/z): 1761 [MH⁺].

### Example 29-2

### Preparation of Aerothricin 133

Aerothricin 133 was prepared according to a method corresponding to that described in Example 29-1.

HPLC(Rt): 19.7 min. (column F, flow rate: 10 ml/min., eluent: 0.05% trifluoroacetic acid-water : 0.05% trifluoroacetic acid-acetonitrile = 60:40); FAB-MS (m/z): 1761 [MH⁺].

### Example 30

### Preparation of Aerothricin 106 from the Compound (IX)

(a). A mixture of Fmoc-Tyr(Bu^{t}) (21 mg, 0.0457 mmol), HOBt mono hydrate (6.6 mg, 0.0431 mmol), BOP reagent (18.8 mg, 0.0424 mmol) and diisopropylethylamine (DIEA , 20µl) in DMF (0.5 ml) was stirred at room temperature for 1 h and then was added to a mixture of N(*orn*)-Boc-IX (19.3 mg, 0.0131 mmol) obtained in Example 6 and DIEA (10 µl) in DMF (1 ml). After stirring at room temperature for 3 h, the resulting mixture was treated with piperidine (0.375 ml) for 1 h and then was concentrated *in vacuo*. The residue was washed with dichloromethane and diethylether to remove the reagents. Purification of the residue by HPLC gave the desired linear peptide A as a white solid (16.6 mg).
   HPLC (Rt) 19 min. (column: Soken-ODS / 20 x 250 mm, flow rate: 9 ml/min., eluent H₂O: CH₃CN = gradient, 1% AcOH).
(b). A mixture of Fmoc-D-Ala mono hydrate (1.2 mg, 0.034 mmol), HOBt mono hydrate (4.7 mg, 0.031 mmol), BOP reagent (13.6 mg, 0.031 mmol) and DIEA (8 µl) in DMF (0.5 ml) was stirred at room temperature for 1 h and then was added to a mixture of the linear peptide A obtained above (16.6 mg, 0.0098 mmol) and DIEA (6 µl) in DMF (1 ml). The reaction mixture was stirred at room temperature and the activated ester was added until the almost starting material was consumed. The resulting mixture was concentrated *in vacuo*. The residue was washed with dichloromethane and diethylether to remove the reagents. The crude product was treated with trifluoroacetic acid at 0°C for 1h. The mixture was concentrated under reduced pressure. Purification of the residue by HPLC gave the desired linear peptide B as a white solid (6.1 mg).
   HPLC(Rt) 19 min. (column: Soken-ODS / 20 x 250 mm, flow rate: 9 ml/min., eluent: H₂O : CH₃CN = gradient, 1% AcOH).
(c). A mixture of Boc-D-Orn(Bu^{t}) (5.7 mg, 0.017 mmol), HOBt mono hydrate (2.3 mg, 0.015 mmol), BOP reagent (5.4 mg, 0.012 mmol) and DIEA (6 µl,) in DMF (0.5 ml) was stirred at room temperature for 1 h and then was added to a mixture of the linear peptide B (6.1 mg, 0.0033 mmol) and DIEA (3 µl) in DMF (1 ml). After stirring at room temperature for 2 h, the resulting mixture was treated with piperidine (0.375 ml) for 1 h. and was concentrated in vacuo. Purification of the residue by HPLC gave linear peptide C as a white solid (4.1 mg).
   HPLC(Rt) 16.7 min. (column: Soken-ODS / 20 x 250 mm, flow rate: 9 ml/min., eluent H₂O : CH₃CN = gradient, 1% AcOH).
(d). The linear peptide C was acidified with 0.01 N hydrochloride and was extracted with *n*-butanol. The butanol extract was concentrated *in vacuo*. The extract was dissolved into DMF (2 ml). Then HOBt mono hydrate (0.1M in DMF, 60 µl), BOP reagent (0.1 M in DMF, 60 µl) and DIEA (2 µl) were added to the mixture. After stirring at room temperature for 1 h, the resulting mixture was concentrated *in vacuo*. The residue was treated with trifluoroacetic acid at 0°C for 1 h. The mixture was concentrated under reduced pressure. Purification of the residue by HPLC gave Aerothricin 106 as a white solid (2.2 mg, 9% from N(*orn*)-Boc-IX).

The analytical data is described in the table of Example 16.

### Example 31

### Preparation of Aerothricin 137

(a). To a solution of Aerothricin 3 monoTFA salt (622mg) in dichloromethane(16ml) and MeOH (4ml) was added N-Boc-aminoethanal(120mg) and N-ethyldiisopropylamine(0.072ml). After being stirred for 1hr at room temperature, to the reaction mixture was added sodium cyanoborohydride (48mg) and sulfuric acid (0.04ml). After the reaction mixture was stirred for 72hr at room temperature, the solvent was evaporated *in vacuo* and then 0.1N HCl was added. It was extracted with nBuOH and concentrated.
(b). The residue was dissolved in DMF(6ml), to which was added 2-(S)-[bis-(2-Boc-aminoethyl)amino]-5-Boc-aminopentanoic acid (294mg), HOAt (77mg), HBTU (215mg) and N-ethyldiisopropylamine (0.148ml). After being stirred for 48hr at room temperature, the solvent was evaporated in *vacuo* and the residue was dissolved in dichloromethane. To this slolution was added ether to give a white precipitate. It was washed with ether and used in the next step without further purification.
(c). To the compound obtained above was then added TFA (3ml) at 0°C. After being stirred for 30min. at 0°C, ether was added to the reaction mixture to give a white precipitate. It was washed with ether and purified by preparative reverse phase HPLC. The pure fractions were combined, frozen and lyophilized to give 95 mg of Aerothricin 137 as a colorless amorphous solid:
   HPLC(Rt): 14.6 min. (column F, flow rate: 10 ml/min., eluent 0.05% trifluoroacetic acid-water: 0.05% trifluoroacetic acid-acetonitrile = 61:39); FAB-MS (m/z): 1776
   [M+H]⁺.

### Example 32

201 mg of Aerothricin 106 and 599 mg of calcium carbonate (mean particle size: 40∼60µm) were mixed well with microspatula in a beaker. Then 200 µl of distilled water was added, and mixing was continued until the mixture became paste. The resulting pasty solid was freeze-dried at -5°C over night, and further dried at 30°C for 3 hr *in vacuo*. After large particle in the dry powder was broken into small particles, 8 mg of calcium stearate was added. The mixture was passed through 180 µm mesh three times.

### Example 33

8 mg of glutinous rice powder and 591 mg of calcium carbonate (mean particle size: 40-60 µm) were mixed well with microspatula in a beaker. Then 201 mg of aerothricin 106 was added and mixed well. To the mixture was added 200 µl of distilled water, and mixing was continued until the mixture became paste. The resulting pasty solid was freeze-dried at-5°C over night, and further dried at 30°C for 3 hr *in vacuo*. After large particle in the dry powder was broken into small particles, 8 mg of calcium stearate was added. The mixture was passed through 180 µm mesh three times.

### Example 34

201 mg of Aerothricin 106 and 599 mg of rice powder (mean particle size: 45-90 µm) were mixed well with microspatula in a beaker. Then 400 µl of distilled water was added, and mixing was continued until the mixture became paste. The resulting pasty solid was freeze-dried at -5°C over night, and further dried at 30°C for 3 hr *in vacuo*. After large particle in the dry powder was broken into small particles, 8 mg of calcium stearate was added. The mixture was passed through 180 µm mesh three times.

### Example 35

201 mg of Aerothricin 106 and 599 mg of corn starch (mean particle size: 20∼180 µm) were mixed well with microspatula in a beaker. Then 500 µl of distilled water was added, and mixing was continued until the mixture became paste. The resulting pasty solid was freeze-dried at -5°C over night, and further dried at 30°C for 3 hr *in vacuo*. After large particle in the dry powder was broken into small particles, 8 mg of calcium stearate was added. The mixture was passed through 180 µm mesh three times.

### Example 36

201 mg of Aerothricin 133 and 599 mg of calcium carbonate (mean particle size: 40-60 µm) were mixed well with microspatula in a beaker. Then 200 µl of distilled water was added, and mixing was continued until the mixture became paste. The resulting pasty solid was freeze-dried at -5°C over night, and further dried at 30°C for 3 hr *in vacuo*. After large particle in the dry powder was broken into small particles, 8 mg of calcium stearate was added. The mixture was passed through 180 µm mesh three times.

### Example 37

201 mg of Aerothricin 132 and 599 mg of calcium carbonate (mean particle size: 40-60 µm) were mixed well with microspatula in a beaker. Then 200 µl of distilled water was added, and mixing was continued until the mixture became paste. The resulting pasty solid was freeze-dried at -5°C over night, and further dried at 30°C for 3 hr *in vacuo*. After large particle in the dry powder was broken into small particles, 8 mg of calcium stearate was added. The mixture was passed through 180 µm mesh three times.

### Example 38

201 mg of Aerothricin 133 and 599 mg of calcium carbonate (mean particle size: 40∼60 µm) were mixed well with microspatula in a beaker. Then a solution of 2.4 mg of gelatine in 500 µl of distilled water was added, and mixing was continued until the mixture became paste. The resulting pasty solid was freed-dried at ∼5°C over night, and further dried at 30°C for 3 hr *in vacuo*. After large particle in the dry powder was broken into small particles, 8 mg of calcium stearate was added. The mixture was passed through 180 µm mesh three times.

### Example 39

201 mg of MK0991 and 599 mg of calcium carbonate (mean particle size: 40-60 µm) were mixed well with microspatula in a beaker. Then 200 µl of distilled water was added, and mixing was continued until the mixture became paste. The resulting pasty solid was freeze-dried at -5°C over night, and further dried at 30°C for 3 hr *in vacuo*. After large particle in the dry powder was broken into small particles, 8 mg of calcium stearate was added. The mixture was passed through 180 µm mesh three times.

## Claims

1. A nasally administrable composition comprising
(i) an antifungal cyclic peptide,
(ii) a physiologically acceptable powdery or crystalline carrier containing either a polyvalence metal or an organic carrier, and
(iii) an absorption enhancer,
wherein an antifungally effective amount of said antifungal cyclic peptide is dispersed homogeneously in and adsorbed homogeneously onto said physiologically acceptable powdery or crystalline polyvalence metal carrier or organic carrier, whose mean particle size is in the range of 20 to 500 µm.

2. A nasally administrable composition of claim 1, wherein said absorption enhancer is a pharmaceutically acceptable natural or unnatural polymer material selected from a group consisting of cellulose, starch, other natural polymer, synthetic polymer and their derivatives.

3. A nasally administrable composition of claim 2, wherein said cellulose and its derivatives is selected from a group consisting of crystalline cellulose, methyl cellulose, hydroxypropyl cellulose, hydroxypropylmethyl cellulose, ethyl cellulose, cellulose acetate and carboxymethyl cellulose.

4. A nasally administrable composition of claim 2, wherein said starch and its derivatives is selected from a group consisting of corn starch, potato starch, rice starch, glutinous rice starch, wheat starch, pregelatinized starch, dextrin, sodium carboxymethyl starch, hydroxypropyl starch and pullulan.

5. A nasally administrable composition of claim 2, wherein said other natural polymer is selected from a group consisting of agar, sodium alginate, chitin, chitosan, egg yolk lecithin, gum arabic, tragacanth, gelatine, collagen, casein, albumin, fibrinogen and fibrin.

6. A nasally administrable composition of claim 2, wherein said synthetic polymer is selected from a group consisting of sodium polyacrylate and polyvinyl pyrrolidone.

7. A nasally administrable composition of claim 1, wherein said absorption enhancer is selected from a group consisting of rice, glutinous rice, starch, gelatine, dextrin, hydroxypropyl cellulose, hydroxypropylmethyl cellulose, polyvinyl pyrrolidone, egg yolk lecithin, gum arabic, tragacanth and a mixture thereof.

8. A nasally administrable composition of claim 1, wherein said absorption enhancer is glutinous rice.

9. A nasally administrable composition of any of claims 1 to 8, wherein said physiologically acceptable powdery or crystalline carrier is an organic carrier.

10. A nasally administrable composition of claim 9, wherein said organic carrier is fine grain powder.

11. A nasally administrable composition of claim 10, wherein said fine grain powder is selected from the group consisting of pulverized rice, wheat, buck wheat, barley, soybean, corn, millet and foxtail millet.

12. A nasally administrable composition of any of claims 1 to 8, wherein said physiologically acceptable powdery or crystalline carrier is a polyvalence metal carrier.

13. A nasally administrable composition of claim 12, wherein said polyvalence metal carrier is a divalence metal compound selected from a group consisting of aluminum compound, calcium compound, magnesium compound, silicon compound, iron compound and zinc compound.

14. A nasally administrable composition of claim 13, wherein said aluminum compound is selected from a group consisting of dry aluminum hydroxy gel, aluminum hydroxychloride, synthetic aluminum silicate, light aluminum oxide, colloidal aluminum silicate hydrate, aluminum magnesium hydroxide, aluminum hydroxide, aluminum hydroxide gel, aluminum sulfate, dihydroxyaluminum aminoacetate, aluminum stearate, natural aluminum silicate, aluminum monostearate and potassium aluminum sulfate.

15. A nasally administrable composition of claim 13, wherein said aluminum compound is aluminum hydroxide.

16. A nasally administrable composition of claim 13, wherein said calcium compound is selected from a group consisting of apatite, hydroxyapatite, calcium carbonate, calcium disodium EDTA, calcium chloride, calcium citrate, calcium glycerophosphate, calcium gluconate, calcium silicate, calcium oxide, calcium hydroxide, calcium stearate, calcium phosphate tribasic, calcium lactate, calcium pantothenate, calcium oleate, calcium palmitate, calcium D-pantothenate, calcium alginate, calcium phosphate anhydride, calcium hydrogenphosphate, calcium primary phosphate, calcium acetate, calcium saccharate, calcium sulfate, calcium secondary phosphate, calcium para-aminosalicylate and bio calcilutite compounds.

17. A nasally administrable composition of claim 13, wherein said calcium compound is hydroxyapatite, calcium carbonate or calcium lactate.

18. A nasally administrable composition of claim 13, wherein said magnesium compound is selected from a group consisting of magnesium L-aspartate, magnesium chloride, magnesium gluconate, magnesium aluminate silicate, magnesium silicate, magnesium oxide, magnesium hydroxide, magnesium stearate, magnesium carbonate, magnesium aluminate metasilicate, magnesium sulfate, sodium magnesium silicate and synthetic sodium magnesium silicate.

19. A nasally administrable composition of claim 13, wherein said magnesium compound is magnesium stearate.

20. A nasally administrable composition of claim 13, wherein said silicon compound is selected from silicon oxide hydrate, light silicic anhydride, synthetic hydrotalcite, diatomaceous earth or silicon dioxide.

21. A nasally administrable composition of claim 13, wherein said iron compound is ferrous sulfate.

22. A nasally administrable composition of claim 13, wherein said zinc compound is selected from zinc chloride, zinc stearate, zinc oxide or zinc sulfate.

23. A nasally administrable composition of any one of claims 1 to 8 and 12 to 22, wherein said physiologically acceptable powdery or crystalline carrier containing a polyvalence metal has a mean particle size of 20 to 250 µm, preferably of 20 to 100 µm.

24. A nasally administrable composition of claim 23, wherein the mean particle size of said physiologically acceptable powdery or crystalline carrier containing a polyvalence metal ranges from 20 µm to 60 µm.

25. A nasally administrable composition of any of claims 9to 11, wherein the mean particle size of said physiologically acceptable powdery or crystalline carrier containing an organic carrier ranges from 20 µm to 300 µm.

26. A nasally administrable composition of any of claims 1 - 25, wherein the antifungal cyclic peptide is any one of aerothricins represented by the Formula (I), 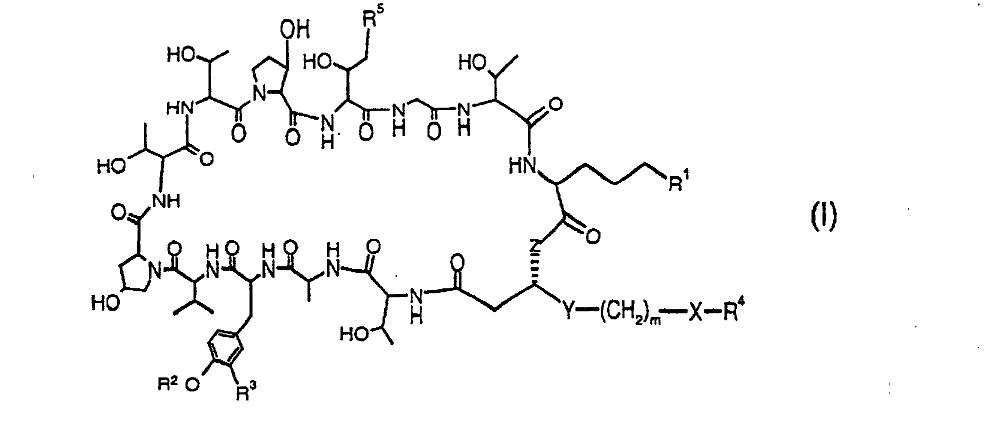 wherein
R¹ is guanidino, tri-C₁₋₆ alkylammonio, -N(R¹⁰)-R¹¹, -N(R¹⁵)-CO-R¹⁴, -N(R¹⁵)-CO-CH[N(R¹⁰)R¹¹]-R¹³,-NHCOCH(R¹³)-NHCOCH(NH₂)-R¹³, 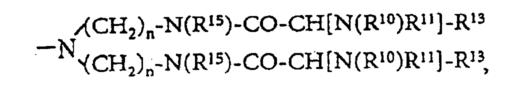 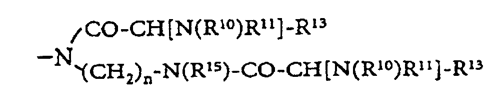 or 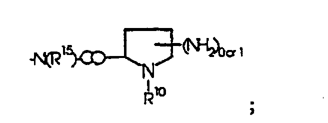 R¹⁰ and R¹¹ are each independently selected from hydrogen; heteroaryl substituted with one or two amino; C₁₋₆ alkyl optionally substituted with one or more, preferably one or two, amino, amino- C₁₋₆ alkyl, cyano, guanidino, nitrogen containing heterocycle(s) or phenyl group(s) containing an amino, amidino or guanidino group;
R¹³ is a residue derived from natural or unnatural amino acids;
R¹⁴ is C₁₋₆ alkyl substituted with one or more, preferably one or two, amino, guanidino, nitrogen containing heterocycle(s) or phenyl group(s) containing an amino, amidino or guanidino group;
R¹⁵ is hydrogen, C₁₋₆ alkyl optionally substituted with one or more, preferably one or two, amino, guanidino, nitrogen containing heterocycle(s) or phenyl group(s) containing an amino, amidino or guanidino group;
R² is hydrogen, hydroxysulfonyl, C₁₋₆ alkyl or C₂₋₆ alkenyl, wherein C₁₋₆ alkyl and C₂₋₆ alkenyl may be optionally substituted with acyl, carbamoyl, amino, mono- C₁₋₆ alkylamino or di- C₁₋₆ alkylamino;
R³ is hydrogen, hydroxy, nitro, amino, acylamino, (C₂₋₇ alkylcarbamoyl)amino, carboxyl, C₁₋₆ alkoxy, C₂₋₇ alkoxycarbonyl, C₁₋₆ alkyl, C₂₋₆ alkenyl or C₂₋₆ alkynyl, wherein C₁₋₆ alkyl, C₂₋₆ alkenyl and C₂₋₆ alkynyl may be optionally substituted with hydroxy, amino, mono- C₁₋₆ alkylamino, di- C₁₋₆ alkylamino, C₂₋₇ alkoxycarbonyl or carbamoyl;
R⁴ is alkyl, alkenyl, alkoxy or alkenyloxy which may be optionally substituted with C₁₋₆ alkyl, aryl, cycloalkyl or fluorine atom(s);
R⁵ is -CONH₂, -CN or -CH₂NH₂;
X is a single bond, or an aryl, biphenyl or terphenyl group optionally containing one or more hetero atom(s) and/or being substituted with halogen atom(s) or C₁₋₆ alkyl;
Y is a single bond, -CH₂-, -CH(lower alkyl)-, -CONH- or -CON(C₁₋₆ alkyl)-;
Z is -O-, -NH- or -N(C₁₋₆ alkyl)-;
m is an integer of 0 to 4; and
n is an integer of 2 to 5;
or pharmaceutically acceptable salts thereof.

27. A nasally administrable composition of claim 26, wherein the antifungal cyclic peptide is any one of aerothricins represented by the Formula (I),
wherein
R¹ is -N(R¹⁰)-R¹¹, -N(R¹⁵)-CO-R¹⁴, -N(R¹⁵)-CO-CH[N(R¹⁰)R¹¹]-R¹³,
-NHCOCH(R¹³)-NHCOCH(NH₂)-R¹³, 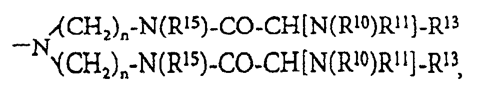 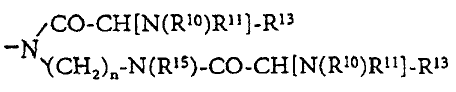 or 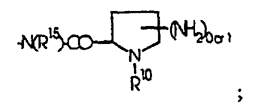 R¹⁰ and R¹¹ are each independently selected from hydrogen; C₁₋₆ alkyl optionally substituted with one or more, preferably one or two, amino, amino- C₁₋₆ alkyl, cyano, guanidino, or nitrogen containing heterocycle(s);
R¹³ is a residue derived from natural or unnatural amino acids;
R¹⁴ is C₁₋₆ alkyl substituted with one or more, preferably one or two, amino, guanidino, nitrogen containing heterocycle(s);
R¹⁵ is hydrogen, C₁₋₆ alkyl optionally substituted with one or more, preferably one or two, amino, guanidino, or nitrogen containing heterocycle(s);
R² is hydrogen or C₁₋₆ alkyl;
R³ is hydrogen, hydroxy, or amino;
R⁴ is alkyl;
R⁵ is -CONH₂, -CN or -CH₂NH₂;
X is a single bond;
Y is a single bond, -CH₂-, -CH(C₁₋₆ alkyl)-;
Z is -O-;
m is an integer of 0 to 4; and
n is an integer of 2 to 5;
or pharmaceutically acceptable salts thereof.

28. A nasally administrable composition of claim 26, wherein the antifungal cyclic peptide is selected from the group consisting of aerothricins 1-5, 14, 15, 17, 31, 32, 63, 96, 101-122, 124, 126-137.

29. A nasally administrable composition of claim 26, wherein the antifungal cyclic peptide is selected from the group consisting of aerothricins 132-137.

30. A nasally administrable composition of any one of claims 1 - 25, wherein the antifungal cyclic peptide is any one of echinocandin analogs.

31. A nasally administrable composition of claim 30, wherein said echinocandin analog is LY303366 or FK463.

32. A nasally administrable composition of claim 30 wherein said echinocandin analog is any one of pneumocandin analogs.

33. A nasally administrable composition of claim 32, wherein said pneumocandin analog is MK0991.

34. The use of the nasally administrable composition as defined in any one of claims 1 to 33 for the manufacture of a medicament for the treatment or prophylaxis of mycoses.

35. A process for the preparation of a nasally administrable composition as defined in any one of claims 1 to 33, which process comprises homogeneously dispersing a physiologically effective amount of an antifungal cyclic peptide in a physiologically acceptable powdery or crystalline carrier containing either a polyvalence metal or organic carrier, in the presence of an absorption enhancer, and adsorbing said active substance thereonto.

## Patentansprüche

1. Eine nasal verabreichbare Zusammensetzung, umfassend:
i) ein antifungales cyclisches Peptid,
ii) einen physiologisch verträglichen pulverförmigen oder kristallinen Träger, der entweder ein mehrwertiges Metall oder einen organischen Träger enthält, und
iii) einen Absorptionsverstärker,
wobei eine antifungal wirksame Menge des antifungalen cyclischen Peptids homogen in dem physiologisch verträglichen pulverförmigen oder kristallinen mehrwertigen Metallträger oder organischen Träger dispergiert wird und homogen daran adsorbiert wird, dessen mittlere Partikelgrösse in dem Bereich von 20 bis 500 µm liegt.

2. Eine nasal verabreichbare Zusammensetzung nach Anspruch 1, wobei der Absorptionsverstärker ein pharmazeutisch verträgliches natürliches oder nicht natürliches Polymermaterial ist, das ausgewählt ist aus der Gruppe, bestehend aus Cellulose, Stärke, einem anderen natürlichen Polymer, einem synthetischen Polymer und deren Derivaten.

3. Eine nasal verabreichbare Zusammensetzung nach Anspruch 2, wobei die Cellulose und deren Derivate ausgewählt sind aus einer Gruppe, bestehend aus kristalliner Cellulose, Methylcellulose, Hydroxypropylcellulose, Hydroxypropylmethylcellulose, Ethylcellulose, Celluloseacetat und Carboxymethylcellulose.

4. Eine nasal verabreichbare Zusammensetzung nach Anspruch 2, wobei die Stärke und deren Derivate ausgewählt sind aus einer Gruppe, bestehend aus Maisstärke, Kartoffelstärke, Reisstärke, Klebreisstärke, Weizenstärke, vorgelatinisierter Stärke, Dextrin, Natriumcarboxymethylstärke, Hydroxypropylstärke und Pullulan.

5. Eine nasal verabreichbare Zusammensetzung nach Anspruch 2, wobei das andere natürliche Polymer ausgewählt ist aus einer Gruppe, bestehend aus Agar, Natriumalginat, Chitin, Chitosan, Eigelblecithin, Gummi arabicum, Tragant, Gelatine, Collagen, Casein, Albumin, Fibrinogen und Fibrin.

6. Eine nasal verabreichbare Zusammensetzung nach Anspruch 2, wobei das synthetische Polymer ausgewählt ist aus einer Gruppe, bestehend aus Natriumpolyacrylat und Polyvinylpyrrolidon.

7. Eine nasal verabreichbare Zusammensetzung nach Anspruch 1, wobei der Absorptionsverstärker ausgewählt ist aus einer Gruppe, bestehend aus Reis, Klebreis, Stärke, Gelatine, Dextrin, Hydroxypropylcellulose, Hydroxypropylmethylcellulose, Polyvinylpyrrolidon, Eigelblecithin, Gummi arabicum, Tragant und einer Mischung von diesen.

8. Eine nasal verabreichbare Zusammensetzung nach Anspruch 1, wobei der Absorptionsverstärker Klebreis ist.

9. Eine nasal verabreichbare Zusammensetzung nach irgendeinem der Ansprüche 1 bis 8, wobei der physiologisch verträgliche pulverförmige oder kristalline Träger ein organischer Träger ist.

10. Eine nasal verabreichbare Zusammensetzung nach Anspruch 9, wobei der organische Träger ein feinkörniges Pulver ist.

11. Eine nasal verabreichbare Zusammensetzung nach Anspruch 10, wobei das feinkörnige Pulver ausgewählt ist aus der Gruppe, bestehend aus pulverisiertem Reis, Weizen, Buchweizen, Gerste, Sojabohnen, Mais, Hirse und kolbenhirse.

12. Eine nasal verabreichbare Zusammensetzung nach irgendeinem der Ansprüche 1 bis 8, wobei der physiologisch verträgliche pulverförmige oder kristalline Träger ein mehrwertiger Metallträger ist.

13. Eine nasal verabreichbare Zusammensetzung nach Anspruch 12, wobei der mehrwertige Metallträger eine zweiwertige Metallverbindung ist, die ausgewählt ist aus einer Gruppe, bestehend aus einer Aluminiumverbindung, Calciumverbindung, Magnesiumverbindung, Siliciumverbindung, Eisenverbindung und Zinkverbindung.

14. Eine nasal verabreichbare Zusammensetzung nach Anspruch 13, wobei die Aluminiumverbindung ausgewählt ist aus einer Gruppe, bestehend aus trockenem Aluminiumhydroxygel, Aluminiumhydroxychlorid, synthetischem Aluminiumsilicat, leichtem Aluminiumoxid, kolloidalem Aluminiumsilicathydrat, Aluminiummagnesiumhydroxid, Aluminiumhydroxid, Aluminiumhydroxidgel, Aluminiumsulfat, Dihydroxyaluminiumaminoacetat, Aluminiumstearat, natürlichem Aluminiumsilicat, Aluminiummonostearat und Kaliumaluminiumsulfat.

15. Eine nasal verabreichbare Zusammensetzung nach Anspruch 13, wobei die Aluminiumverbindung Aluminiumhydroxid ist.

16. Eine nasal verabreichbare Zusammensetzung nach Anspruch 13, wobei die Calciumverbindung ausgewählt ist aus einer Gruppe, bestehend aus Apatit, Hydroxyapatit, Calciumcarbonat, Calciumdinatrium-EDTA, Calciumchlorid, Calciumcitrat, Calciumglycerophosphat, Calciumgluconat, Calciumsilicat, Calciumoxid, Calciumhydroxid, Calciumstearat, dreiwertigem Calciumphosphat, Calciumlactat, Calciumpantothenat, Calciumoleat, Calciumpalmitat, Calcium-D-pantothenat, Calciumalginat, Calciumphosphatanhydrid, Calciumhydrogenphosphat, primärem Calciumphosphat, Calciumacetat, Calciumsaccharat, Calciumsulfat, sekundärem Calciumphosphat, Calciumparaaminosalicylat und Biocalcilutit-Verbindungen.

17. Eine nasal verabreichbare Zusammensetzung nach Anspruch 13, wobei die Calciumverbindung Hydroxyapatit, Calciumcarbonat oder Calciumlactat ist.

18. Eine nasal verabreichbare Zusammensetzung nach Anspruch 13, wobei die Magnesiumverbindung ausgewählt ist aus einer Gruppe, bestehend aus Magnesium-Laspartat, Magnesiumchlorid, Magnesiumgluconat, Magnesiumaluminatsilicat, Magnesiumsilicat, Magnesiumoxid, Magnesiumhydroxid, Magnesiumstearat, Magnesiumcarbonat, Magnesiumaluminatmetasilicat, Magnesiumsulfat, Natriummagnesiumsilicat und synthetischem Natriummagnesiumsilicat.

19. Eine nasal verabreichbare Zusammensetzung nach Anspruch 13, wobei die Magnesiumverbindung Magnesiumstearat ist.

20. Eine nasal verabreichbare Zusammensetzung nach Anspruch 13, wobei die Siliciumverbindung ausgewählt ist aus Siliciumoxidhydrat, leichtem Kieselsäureanhydrid, synthetischem Hydrotalcit, Diatomeenerde oder Siliciumdioxid.

21. Eine nasal verabreichbare Zusammensetzung nach Anspruch 13, wobei die Eisenverbindung Eisen(II)-sulfat ist.

22. Eine nasal verabreichbare Zusammensetzung nach Anspruch 13, wobei die Zinkverbindung ausgewählt ist aus Zinkchlorid, Zinkstearat, Zinkoxid oder Zinksulfat.

23. Eine nasal verabreichbare Zusammensetzung nach irgendeinem der Ansprüche 1 bis 8 und 12 bis 22, wobei der physiologisch verträgliche pulverförmige oder kristalline Träger, welcher ein mehrwertiges Metall enthält, eine mittlere Partikelgrösse von 20 bis 250 µm, vorzugsweise von 20 bis 100 µm aufweist.

24. Eine nasal verabreichbare Zusammensetzung nach Anspruch 23, wobei die mittlere Partikelgrösse des physiologisch verträglichen pulverförmigen oder kristallinen Trägers, welcher ein mehrwertiges Metall enthält, von 20 bis 60 µm reicht.

25. Eine nasal verabreichbare Zusammensetzung nach irgendeinem der Ansprüche 9 bis 11, wobei die mittlere Partikelgrösse des physiologisch verträglichen pulverförmigen oder kristallinen Trägers, welcher einen organischen Träger enthält, von 20 µm bis 300 µm reicht.

26. Eine nasal verabreichbare Zusammensetzung nach irgendeinem der Ansprüche 1 bis 25, wobei das antifungale cyclische Peptid irgendein Aerothricin ist, das durch die Formel (I) dargestellt wird, 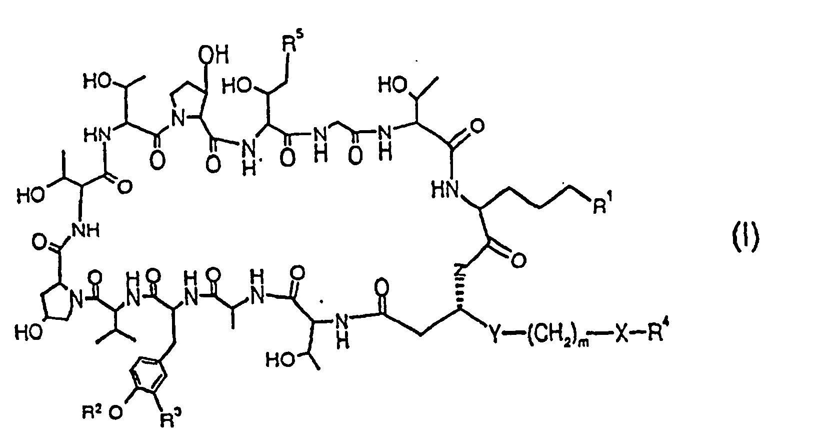 worin
R¹ Guanidino, Tri-C₁₋₆-alkylammonio, -N(R¹⁰)-R¹¹, -N(R¹⁵)-CO-R¹⁴, -N(R¹⁵)-CO-CH[N(R¹⁰)R¹¹]-R¹³, -NHCOCH(R¹³)-NHCOCH(NH₂)-R¹³, 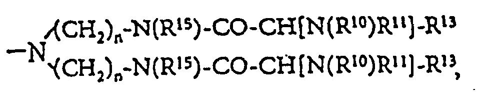 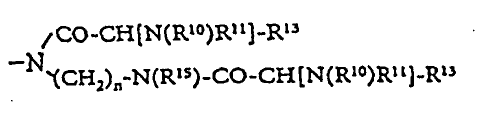 oder 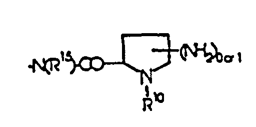 ist;
R¹⁰ und R¹¹ jeweils unabhängig ausgewählt sind aus Wasserstoff; Heteroaryl, welches mit ein oder zwei Aminogruppen substituiert ist; C₁₋₆-Alkyl, das gegebenenfalls substituiert ist mit einem oder mehreren, vorzugsweise einem oder zwei Amino, Amino-C₁₋₆-alkyl, Cyano, Guanidino, einem stickstoffhaltigen Heterocyclus(en) oder einer Phenylgruppe(n), welche eine Amino-, Amidino- oder Guanidinogruppe enthalten;
R¹³ ein Rest ist, der von natürlichen oder nicht natürlichen Aminosäuren abgeleitet ist;
R¹⁴ C₁₋₆-Alkyl ist, welches substituiert ist mit einem oder mehreren, vorzugsweise einem oder zwei Amino, Guanidino, einem stickstoffhaltigen Heterocyclus(en) oder einer Phenylgruppe(n), welche eine Amino-, Amidino- oder Guanidinogruppe enthalten;
R¹⁵ Wasserstoff, C₁₋₆-Alkyl, das gegebenenfalls substituiert ist mit einem oder mehreren, vorzugsweise einem oder zwei Amino, Guanidino, einem stickstoffhaltigen Heterocyclus(en) oder einer Phenylgruppe(n), welche eine Amino-, Amidino- oder Guanidinogruppe enthalten, ist;
R² Wasserstoff, Hydroxysulfonyl, C₁₋₆-Alkyl oder C₂₋₆-Alkenyl ist, wobei C₁₋₆-Alkyl und C₂₋₆-Alkenyl gegebenenfalls substituiert sein können mit Acyl, Carbamoyl, Amino, Mono-C₁₋₆-alkylamino oder Di-C₁₋₆-alkylamino;
R³ Wasserstoff, Hydroxy, Nitro, Amino, Acylamino, (C₂₋₇-Alkylcarbamoyl)amino, Carboxyl, C₁₋₆-Alkoxy, C₂₋₇-Alkoxycarbonyl, C₁₋₆-Alkyl, C₂₋₆-Alkenyl oder C₂₋₆-Alkinyl ist, wobei C₁₋₆-Alkyl, C₂₋₆-Alkenyl und C₂₋₆-Alkinyl gegebenenfalls substituiert sein können mit Hydroxy, Amino, Mono-C₁₋₆-alkylamino, Di-C₁₋₆-alkylamino, C₂₋₇-Alkoxycarbonyl oder Carbamoyl;
R⁴ Alkyl, Alkenyl, Alkoxy oder Alkenyloxy ist, welche gegebenenfalls substituiert sein können mit C₁₋₆-Alkyl, Aryl, Cycloalkyl oder einem Fluoratom(en);
R⁵ -CONH₂, -CN oder -CH₂NH₂ ist;
X eine Einfachbindung ist oder eine Aryl-, Biphenyl- oder Terphenylgruppe, welche gegebenenfalls ein oder mehrere Heteroatom(e) enthält und/oder mit einem Halogenatom(en) oder C₁₋₆-Alkyl substituiert ist;
Y eine Einfachbindung, -CH₂-, -CH(niederes Alkyl)-, -CONH- oder -CON(C₁₋₆-Alkyl)- ist;
Z -O-, -NH- oder -N(C₁₋₆-Alkyl) ist;
m ein ganze Zahl von 0 bis 4 ist; und
n eine ganze Zahl von 2 bis 5 ist;
oder pharmazeutische verträgliche Salze von diesen.

27. Eine nasal verabreichbare Zusammensetzung nach Anspruch 26, wobei das antifungale cyclische Peptid irgendeines der Aerothricine ist, welche durch die Formel (I) dargestellt werden,
worin
R¹-N(R¹⁰)-R¹¹, -N(R¹⁵)-CO-R¹⁴, -N(R¹⁵)-CO-CH[N(R¹⁰)R¹¹]-R¹³, -NHCOCH(R¹³)-NHCOCH(NH₂)-R¹³, 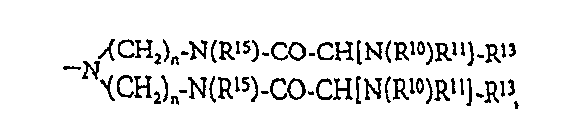 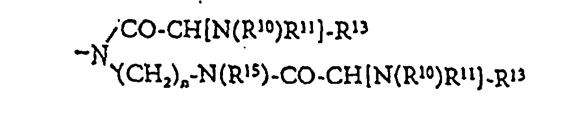 oder 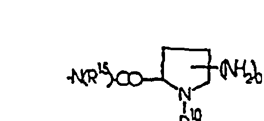 ist;
R¹⁰ und R¹¹ jeweils unabhängig ausgewählt sind aus Wasserstoff, C₁₋₆-Alkyl, welches gegebenenfalls substituiert ist mit einem oder mehreren, vorzugsweise einem oder zwei Amino, Amino-C₁₋₆-alky, Cyano, Guanidino oder einem stickstoffhaltigen Heterocyclus(en);
R¹³ ein Rest ist, der von natürlichen oder nicht natürlichen Aminosäuren abgeleitet ist;
R¹⁴ C₁₋₆-Alkyl ist, das substituiert ist mit einem oder mehreren, vorzugsweise einem oder zwei Amino, Guanidino, einem stickstoffhaltigen Heterocyclus(en);
R¹⁵ Wasserstoff, C₁₋₆-Alkyl ist, das gegebenenfalls substituiert ist mit einem oder mehreren, vorzugsweise einem oder zwei Amino, Guanidino oder einem stickstoffhaltigen Heterocyclus(en);
R² Wasserstoff oder C₁₋₆-Alkyl ist;
R³ Wasserstoff, Hydroxy oder Amino ist;
R⁴ Alkyl ist;
R⁵ -CONH₂, -CN oder -CH₂NH₂ ist;
X eine Einfachbindung ist;
Y eine Einfachbindung, -CH₂-, CH(C₁₋₆-Alkyl)- ist;
Z -O- ist,
m eine ganze Zahl von 0 bis 4 ist; und
n eine ganze Zahl von 2 bis 5 ist;
oder pharmazeutisch verträgliche Salze von diesen.

28. Eine nasal verabreichbare Zusammensetzung nach Anspruch 26, wobei das antifungale cyclische Peptid ausgewählt ist aus der Gruppe, bestehend aus den Aerothricinen 1-5, 14, 15, 17, 31, 32, 63, 96, 101-122, 124, 126-137.

29. Eine nasal verabreichbare Zusammensetzung nach Anspruch 26, wobei das antifungale cyclische Peptid ausgewählt ist aus der Gruppe, bestehend aus den Aerothricinen 132-137.

30. Eine nasal verabreichbare Zusammensetzung nach irgendeinem der Ansprüche 1 bis 25, wobei das antifungale cyclische Peptid irgendeines der Echinocandin-Analogen ist.

31. Eine nasal verabreichbare Zusammensetzung nach Anspruch 30, wobei das Echinocandin-Analoge LY303366 oder FK463 ist.

32. Eine nasal verabreichbare Zusammensetzung nach Anspruch 30, wobei das Echinocandin-Analoge irgendeines der Pneumocandin-Analogen ist.

33. Eine nasal verabreichbare Zusammensetzung nach Anspruch 32, wobei das Pneumocandin-Analoge MK0991 ist.

34. Die Verwendung der nasal verabreichbaren Zusammensetzung, wie in irgendeinem der Ansprüche 1 bis 33 definiert, zur Herstellung eines Medikaments zur Behandlung oder Prophylaxe von Mykosen.

35. Ein Verfahren zur Herstellung einer nasal verabreichbaren Zusammensetzung, wie in irgendeinem der Ansprüche 1 bis 33 definiert, wobei das Verfahren das homogene Dispergieren einer physiologisch wirksamen Menge eines antifungalen cyclischen Peptids in einem physiologisch verträglichen pulverförmigen oder kristallinen Träger, der entweder ein mehrwertiges Metall oder einen organischen Träger enthält, in Gegenwart eines Absorptionsverstärkers und das Adsorbieren der aktiven Substanz auf diesem umfasst.

## Revendications

1. Composition administrable par la voie nasale, comprenant :
(i) un peptide cyclique antifongique,
(ii) un véhicule pulvérulent ou cristallin physiologiquement acceptable contenant un métal polyvalent ou un véhicule organique, et
(iii) un promoteur d'absorption,
dans laquelle une quantité efficace au plan antifongique dudit peptide cyclique antifongique est dispersé de façon homogène dans ledit véhicule métallique ou organique polyvalent pulvérulent ou cristallin physiologiquement acceptable et adsorbé de manière homogène sur ce véhicule dont la taille particulaire moyenne se situe dans la plage de 20 à 500 µm.

2. Composition administrable par la voie nasale selon la revendication 1, dans laquelle ledit promoteur d'absorption est un matériau polymère naturel ou non, pharmaceutiquement acceptable, choisi dans le groupe constitué de la cellulose, de l'amidon, d'un autre polymère naturel, d'un polymère synthétique et de leurs dérivés.

3. Composition administrable par la voie nasale selon la revendication 2, dans laquelle on choisit ladite cellulose et ses dérivés dans le groupe constitué de la cellulose cristalline, de la méthylcellulose, de l'hydroxypropylcellulose, de l'hydroxypropylméthylcellulose, de l'éthylcellulose, de l'acétate de cellulose et de la carboxyméthylcellulose.

4. Composition administrable par la voie nasale selon la revendication 2, dans laquelle on choisit ledit amidon et ses dérivés dans le groupe constitué de l'amidon de maïs, de l'amidon de pomme de terre, de l'amidon de riz, de l'amidon de riz glutineux, de l'amidon de blé, de l'amidon prégélatinisé, de la dextrine, du carboxyméthylamidon sodique, de l'hydroxy-propylamidon et du pullulane.

5. Composition administrable par la voie nasale selon la revendication 2, dans laquelle ledit autre polymère naturel est choisi dans le groupe constitué de l'agar, de l'alginate de sodium, de la chitine, du chitosane, de la lécithine de jaune d'oeuf, de la gomme arabique, de la gomme adragante, de la gélatine, du collagène, de la caséine, de l'albumine, du fibrinogène et de la fibrine.

6. Composition administrable par la voie nasale selon la revendication 2, dans laquelle ledit polymère synthétique est choisi dans le groupe constitué du polyacrylate de sodium et de la polyvinylpyrrolidone.

7. Composition administrable par la voie nasale selon la revendication 1, dans laquelle ledit promoteur d'absorption est choisi dans le groupe constitué du riz, du riz glutineux, de l'amidon, de la gélatine, de la dextrine, de l'hydroxypropylcellulose, de l'hydroxypropylméthylcellulose, de la polyvinylpyrrolidone, de la lécithine de jaune d'oeuf, de la gomme arabique, de la gomme adragante et d'un de leurs mélanges.

8. Composition administrable par la voie nasale selon la revendication 1, dans laquelle ledit promoteur d'absorption est le riz glutineux.

9. Composition administrable par la voie nasale selon l'une quelconque des revendications 1 à 8, dans laquelle ledit véhicule pulvérulent ou cristallin physiologiquement acceptable est un véhicule organique.

10. Composition administrable par la voie nasale selon la revendication 9, dans laquelle ledit véhicule organique est une poudre à grains fins.

11. Composition administrable par la voie nasale selon la revendication 10, dans laquelle ladite poudre à grains fins est choisie dans le groupe constitué du riz, du blé, du sarrasin, de l'orge, de la graine de soya, du maïs, du millet et du sétaire d'Italie pulvérisés.

12. Composition administrable par la voie nasale selon l'une quelconque des revendications 1 à 8, dans laquelle ledit véhicule pulvérulent ou cristallin physiologiquement acceptable est un véhicule de métal polyvalent.

13. Composition administrable par la voie nasale selon la revendication 12, dans laquelle ledit véhicule de métal polyvalent est un composé de métal divalent choisi dans le groupe constitué des composés d'aluminium, de calcium, de magnésium, de silicium, de fer et de zinc.

14. Composition administrable par la voie nasale selon la revendication 13, dans laquelle ledit composé d'aluminium est choisi dans le groupe constitué du gel d'hydroxyde d'aluminium sec, de l'hydroxychlorure d'aluminium, du silicate d'aluminium synthétique, de l'oxyde d'aluminium léger, du silicate d'aluminium colloïdal hydraté, de l'hydroxyde de magnésium/aluminium, de l'hydroxyde d'aluminium, du gel d'hydroxyde d'aluminium, du sulfate d'aluminium, de l'aminoacétate de dihydroxyaluminium, du stéarate d'aluminium, du silicate d'aluminium naturel, du monostéarate d'aluminium et du sulfate d'aluminium de potassium.

15. Composition administrable par la voie nasale selon la revendication 13, dans laquelle ledit composé d'aluminium est un hydroxyde d'aluminium.

16. Composition administrable par la voie nasale selon la revendication 13, dans laquelle ledit composé de calcium est choisi dans le groupe constitué de composés d'apatite, d'hydroxyapatite, de carbonate de calcium, d'EDTA de calcium disodique, de chlorure de calcium, de citrate de calcium, de glycérophosphate de calcium, de gluconate de calcium, de silicate de calcium, d'oxyde de calcium, d'hydroxyde de calcium, de stéarate de calcium, de phosphate de calcium tribasique, de lactate de calcium, de pantothénate de calcium, d'oléate de calcium, de palmitate de calcium, de D-pantothénate de calcium, d'alginate de calcium, d'anhydride phosphate de calcium, d'hydrogénophosphate de calcium, de phosphate primaire de calcium, d'acétate de calcium, de saccharate de calcium, de sulfate de calcium, de phosphate secondaire de calcium, de para-aminosalicylate de calcium et de bio-calcilutite.

17. Composition administrable par la voie nasale selon la revendication 13, dans laquelle ledit composé de calcium est l'hydroxyapatite, le carbonate de calcium ou le lactate de calcium.

18. Composition administrable par la voie nasale selon la revendication 13, dans laquelle ledit composé de magnésium est choisi dans le groupe constitué du L-aspartate de magnésium, du chlorure de magnésium, du gluconate de magnésium, du silicate de magnésium/aluminium, du silicate de magnésium, de l'oxyde de magnésium, de l'hydroxyde de magnésium, du stéarate de magnésium, du carbonate de magnésium, du métasilicate aluminate de magnésium, du sulfate de magnésium, du sulfate de magnésium/sodium et du silicate de magnésium/ sodium synthétique.

19. Composition administrable par la voie nasale selon la revendication 13, dans laquelle ledit composé de magnésium est le stéarate de magnésium.

20. Composition administrable par la voie nasale selon la revendication 13, dans laquelle ledit composé de silicium est choisi parmi l'oxyde de silicium hydraté, l'anhydride silicique léger, l'hydrotalcite synthétique, la terre à diatomées ou le dioxyde de silicium.

21. Composition administrable par la voie nasale selon la revendication 13, dans laquelle ledit composé de fer est un sulfate ferreux.

22. Composition administrable par la voie nasale selon la revendication 13, dans laquelle ledit composé de zinc est choisi parmi le chlorure de zinc, le stéarate de zinc, l'oxyde de zinc ou le sulfate de zinc.

23. Composition administrable par la voie nasale selon l'une quelconque des revendications 1 à 8 et 12 à 22, dans laquelle ledit véhicule pulvérulent ou cristallin physiologiquement acceptable contenant un métal polyvalent a une taille particulière moyenne de 20 à 250 µm, de préférence de 20 à 100 µm.

24. Composition administrable par la voie nasale selon la revendication 23, dans laquelle la taille particulaire moyenne dudit véhicule pulvérulent ou cristallin physiologiquement acceptable contenant un métal polyvalent se situe dans la plage de 20 µm à 60 µm.

25. Composition administrable par la voie nasale selon l'une quelconque des revendications 9 à 11, dans laquelle la taille particulaire moyenne dudit véhicule pulvérulent ou cristallin physiologiquement acceptable contenant un véhicule organique se situe dans la plage de 20 µm à 300 µm.

26. Composition administrable par la voie nasale selon l'une quelconque des revendications 1 à 25, dans laquelle le peptide cyclique antifongique est l'une quelconque des aérothricines représentées par la formule (I) : 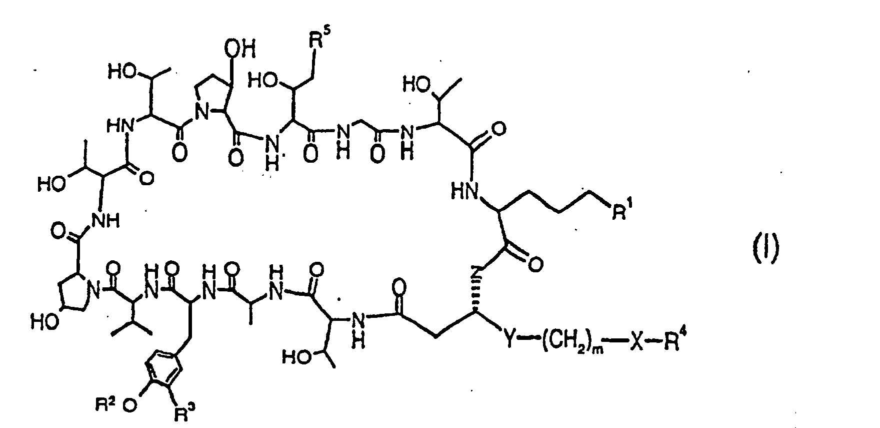 dans laquelle :
R¹ est un guanidino, un tri-alkyl(C₁₋₆)ammonio, -N(R¹⁰)-R¹¹, -N(R¹⁵)-CO-R¹⁴, -N(R¹⁵)-CO-CH[N(R¹⁰)R¹¹]-R¹³, -NHCOCH(R¹³)-NHCOCH(NH₂)-R¹³, 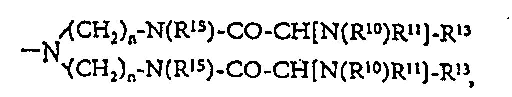 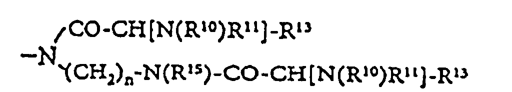 ou 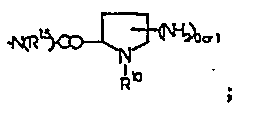
R¹⁰ et R¹¹ sont choisis, chacun indépendamment, parmi un atome d'hydrogène; un hétéroaryle substitué par un ou deux aminos; un alkyle en C₁₋₆ éventuellement substitué par un ou plusieurs, de préférence un ou deux groupements amino, amino-alkyle(C₁₋₆), cyano, guanidino, un ou des hétérocycles contenant de l'azote ou un ou des groupements phényle contenant un groupement amino, amidino ou guanidino;
R¹³ est un résidu dérivé d'acides aminés naturels ou non;
R¹⁴ est un alkyle en C₁₋₆ substitué par ou plusieurs, de préférence un ou deux groupements amino, guanidino, un ou des hétérocycles contenant de l'azote ou un ou des groupements phényle contenant un groupement amino, amidino ou guanidino;
R¹⁵ est un atome d'hydrogène, un alkyle en C₁₋₆ éventuellement substitué par un ou plusieurs, de préférence un ou deux groupements amino, guanidino, un ou des hétérocycles contenant de l'azote ou un ou des groupements phényle contenant un groupement amino, amidino ou guanidino;
R² est un atome d'hydrogène, un hydroxysulfonyle, un alkyle en C₁₋₆ ou un alcényle en C₂₋₆, où l'alkyle en C₁₋₆ et l'alcényle en C₂₋₆ peuvent éventuellement être substitués par un acyle, un carbamoyle, un amino, un monoalkylamino en C₁₋₆ ou un dialkylamino en C₁₋₆;
R³ est un atome d'hydrogène, un hydroxy, un nitro, un amino, un acylamino, un (alkylcarbamoyl en C₂₋₇) amino, un carboxyle, un alcoxy en C₁₋₆, un alcoxycarbonyle en C₂₋₇, un alkyle en C₁₋₆, un alcényle en C₂₋₆ ou un alcynyle en C₂₋₆, où l'alkyle en C₁₋₆, l'alcényle en C₂₋₆ et l'alcynyle en C₂₋₆ peuvent être éventuellement substitués par un hydroxy, un amino, un monoalkylamino en C₁₋₆, un dialkylamino en C₁₋₆, un alcoxycarbonyle en C₂₋₇ ou un carbamoyle;
R⁴ est un alkyle, un alcényle, un alcoxy ou un alcényloxy qui peut être éventuellement substitué par un alkyle en C₁₋₆, un aryle, un cycloalkyle ou un ou des atomes de fluor;
R⁵ est -CONH₂, -CN ou -CH₂NH₂;
X est une liaison unique ou un groupement aryle, biphényle ou terphényle contenant éventuellement un ou plusieurs hétéroatomes et/ou substitué par un ou des atomes d'halogène ou un alkyle en C₁₋₆;
Y est une liaison unique, -CH₂-, -CH(alkyle inférieur)-, -CONH- ou -CON(alkyle en C₁₋₆)-;
Z est -O-, -NH- ou -N(alkyle en C₁₋₆)-;
m est un entier de 0 à 4; et
n est un entier de 2 à 5;
ou leurs sels pharmaceutiquement acceptables.

27. Composition administrable par la voie nasale selon la revendication 26, dans laquelle le peptide cyclique antifongique est l'une quelconque des aérothricines représentées par la formule (I) :
dans laquelle :
R¹ est -N(R¹⁰)-R¹¹, -N(R¹⁵)-CO-R¹⁴, -N(R¹⁵)-CO-CH[N(R¹⁰)R¹¹]-R¹³, -NHCOCH(R¹³)-NHCOCH(NH₂)-R¹³, 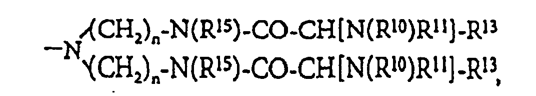 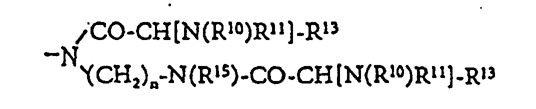 ou 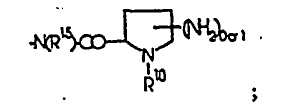
R¹⁰ et R¹¹ sont choisis, chacun indépendamment, parmi un atome d'hydrogène; un alkyle en C₁₋₆ éventuellement substitué par un ou plusieurs, de préférence un ou deux groupements amino, aminoalkyle en C₁₋₆, cyano, guanidino, ou un ou des hétérocycles contenant de l'azote;
R¹³ est un résidu dérivé d'acides aminés naturels ou non;
R¹⁴ est un alkyle en C₁₋₆ substitué par un ou plusieurs, de préférence un ou deux groupements amino, guanidino, ou un ou des hétérocycles contenant de l'azote;
R¹⁵ est un atome d'hydrogène, un alkyle en C₁₋₆ éventuellement substitué par un ou plusieurs, de préférence un ou deux amino, guanidino ou un ou des hétérocycles contenant de l'azote;
R² est un atome d'hydrogène ou un alkyle en C₁₋₆;
R³ est un atome d'hydrogène, un hydroxy ou un amino;
R⁴ est un alkyle;
R⁵ est -CONH₂, -CN ou -CH₂NH₂;
X est une liaison unique;
Y est une liaison unique, -CH₂-, -CH(alkyle en C₁₋₆)-;
Z est -O-;
m est un entier de 0 à 4; et
n est un entier de 2 à 5;
ou leurs sels pharmaceutiquement acceptables.

28. Composition administrable par la voie nasale selon la revendication 26, dans laquelle le peptide cyclique antifongique est choisi dans le groupe constitué des aérothricines 1 à 5, 14, 15, 17, 31, 32, 63, 96, 101 à 122, 124, 126 à 137.

29. Composition administrable par la voie nasale selon la revendication 26, dans laquelle le peptide cyclique antifongique est choisi dans le groupe constitué des aérothricines 132 à 137.

30. Composition administrable par la voie nasale selon l'une quelconque des revendications 1 à 25, dans laquelle le peptide cyclique antifongique est l'un quelconque des analogues de l'échinocandine.

31. Composition administrable par la voie nasale selon la revendication 30, dans laquelle ledit analogue de l'échinocandine est le LY303366 ou le FK463.

32. Composition administrable par la voie nasale selon la revendication 30, dans laquelle ledit analogue de l'échinocandine est l'un quelconque des analogues de la pneumocandine.

33. Composition administrable par la voie nasale selon la revendication 32, dans laquelle ledit analogue de la pneumocandine est le MK0991.

34. Utilisation d'une composition administrable par la voie nasale selon l'une quelconque des revendications 1 à 33 pour la production d'un médicament pour le traitement ou la prophylaxie de mycoses.

35. Procédé pour la préparation d'une composition administrable par la voie nasale selon l'une quelconque des revendications 1 à 33, ledit procédé comprenant la dispersion homogène d'une quantité physiologiquement efficace d'un peptide cyclique antifongique dans un véhicule pulvérulent ou cristallin physiologiquement acceptable contenant un métal polyvalent ou un véhicule organique en présence d'un promoteur d'absorption et l'adsorption de ladite substance active sur ledit peptide.
